# EUROPEAN PATENT SPECIFICATION

(11) **EP 2 142 640 B1**
(45) Date of publication and mention of the grant of the patent: **04.01.2012**
(21) Application number: 08747441.7
(22) Date of filing: 02.05.2008
(51) Int. Cl.: G01N 33/50, C12Q 1/68

(54) **IDENTIFYING GERMLINE COMPETENT EMBRYONIC STEM CELLS**
IDENTIFIZIERUNG VON KEIMBAHNFÄHIGEN EMBRYONISCHEN STAMMZELLEN
IDENTIFICATION DE CELLULES SOUCHES EMBRYONNAIRES À COMPÉTENCE GERMINALE

(30) Priority: 03.05.2007 US 927568 P
(43) Date of publication of application: 13.01.2010
(73) Proprietor: REGENERON PHARMACEUTICALS, INC., Tarrytown, NY 10591 (US)
(72) Inventor: WEI, Yi, Ossining, NY 10562 (US); MACDONALD, Lynn, White Plains, NY 10605 (US); LIN, Hsin, Chieh, Yorktown Heights, NY 10598 (US)
(74) Representative: Bentham, Andrew
(86) International application number: PCT/US2008/062340
(87) International publication number: WO 2008/137629

(56) References cited:
- WO-A-2008/080154
- CARMEN ALVAREZ M ET AL: "Fish ES Cells and Applications to Biotechnology" MARINE BIOTECHNOLOGY, SPRINGER-VERLAG, NE, vol. 9, no. 2, 6 November 2006 (2006-11-06), pages 117-127, XP019495024 ISSN: 1436-2236
- YEOM Y I ET AL: "GERMLINE REGULATORY ELEMENT OF OCT-4 SPECIFIC FOR THE TOTIPOTENT CYCLE OF EMBRYONAL CELLS" DEVELOPMENT, COMPANY OF BIOLOGISTS, CAMBRIDGE, GB, vol. 122, 1 January 1996 (1996-01-01), pages 881-894, XP000199302 ISSN: 0950-1991
- BREHM A ET AL: "OCT-4: MORE THAN JUST A POUERFUL MARKER OF THE MAMMALIAN GERMLINE?" APMIS, COPENHAGEN, DK, vol. 106, no. 1, 1 January 1998 (1998-01-01), pages 114-126, XP000700187 ISSN: 0903-4641
- RAMALHO-SANTOS M ET AL: "Stemness: transcriptional profiling of embryonic and adult stem cells" SCIENCE, WASHINGTON, DC, vol. 298, no. 5593, 18 October 2002 (2002-10-18), pages 597-600, XP002327804 ISSN: 0036-8075
- SHAROVA ET AL: "Global gene expression profiling reveals similarities and differences among mouse pluripotent stem cells of different origins and strains" DEVELOPMENTAL BIOLOGY, ACADEMIC PRESS, NEW YORK, NY, US, vol. 307, no. 2, 2 July 2007 (2007-07-02), pages 446-459, XP022137857 ISSN: 0012-1606

## Description

### FIELD OF INVENTION

The field of invention is related to methods for identifying germline competent embryonic stem cells, including mouse embryonic stem cells, wherein the stem cells can include a genetic alteration that is a deletion, a mutation, and/or a transgene.

### BACKGROUND

An important step in making transgenic animals is assessing the likelihood that a particular ES cell transplanted into an embryo will contribute to the germline of the animal that will result from the embryo. Unless the ES cell contributes to the animal's germline, it is generally not possible to breed animals containing a desired genetic modification reflected in the ES cell's genome. The most common method for assessing the likelihood that an ES cell will transmit to the germline is karyotyping, or another cytological method. An abnormal karyotype generally is accepted as an indication that the ES cell would not be germline competent. But karyotyping, as well as other commonly employed cytological methods, is not very predictive of germline competence. For example, some ES cell clones that display normal karyotypes do not give rise to germline transmitting chimeras, or any chimeras at all. Further, karyotyping is a relatively laborious and expensive process that is not amenable to high throughput processing, but instead represents a significant bottleneck in producing transgenic animals. Accordingly, there is a need for better methods for identifying ES cells that are likely to be germline competent, including methods that are amenable to high throughput processing.

### SUMMARY

The invention is based at least in part on the ability to predict germline competency of ES cells by analyzing RNA expression in the ES cells. ES cells that are totipotent, and thus germline-competent, display an expression profile (i.e., identity of genes expressed and/or level of expression) that can be differentiated from non-totipotent ES cells.

Described herein is a method for selecting an ES cell that is germline competent, comprising providing an expression profile of a subset of ES cell genes from an ES cell, and comparing the expression profile of the subset of ES cell genes from the ES cell with an expression profile of a subset of ES cell genes from a germline competent ES cell.
The invention provides a method for selecting an ES cell that is germline competent, comprising providing an expression profile of a subset of ES cell genes from an ES cell and comparing the expression profile of the subset of ES cell genes from the ES cell with an expression profile of a subset of ES cell genes from a germline competent ES cell, wherein the subset of ES cell genes is one or more genes listed in Table 8 in conjunction with one or more different genes selected from the genes listed in Table 5.

In one embodiment, the profile is a profile of RNA. In a specific embodiment, the profile is a profile of mRNA. In one embodiment, the profile is a profile of expressed proteins. In a specific embodiment, the expressed proteins are proteins that are secreted. In a specific embodiment, the proteins are cell surface proteins.

In one embodiment, the expression profile is generated on an expression array.

In another embodiment, the expression profile is generated by an ELISA method or a protein expression assay.

Additionally described herein are methods where the expression profile is for a single gene. In such methods, an ES cell is germline competent if at least one preselected RNA is expressed at a level comparable to the level at which the at least one preselected RNA is expressed in a germline competent ES cell.

In another embodiment, the expression profile is a profile of expressed and secreted genes. In one embodiment, the expression profile is generated using an ELISA assay. In another embodiment, the expression profile is generated using an affinity assay using microspheres such as, for example, a Luminex assay.

In another embodiment, the subset of genes is about 292 genes. In another embodiment, the subset of genes is about 200 genes; in another embodiment, about 100 genes; in another embodiment, about 50 genes; in another embodiment, about 25 genes; in another embodiment, about 10 genes; in another embodiment, about five genes; in another embodiment, two genes. Also described herein is use of one gene.

According to the invention, the subset of genes is one or more genes listed in Table 8 in conjunction with one or more different genes selected from the genes listed in Table 5. In other methods described herein, the subset of genes is: one or more genes listed in Table 8; or one or more genes listed in Table 7; or one or more genes listed in Table 6; or one or more genes listed in Table 5; or one or more genes listed in Table 4; or one or more genes listed in Table 3; or one or more genes listed in Table 2; or one or more genes listed in Table 1.

In additional methods described herein, the subset of genes is one or more genes listed in Table 8, in conjunction with one or more genes selected from the lists in Table 1 through Table 7. In another described method, the subset of genes consists of a single gene. In a specific described method, the single gene is gtl2.

Further described herein is a method for predicting germline competency for an ES cell comprising providing a subset of genes that are differentially expressed between germline competent ES cells and non-germline competent ES cells, ascertaining the level of expression of the subset of genes in an ES cell, and comparing the expression of the subset of genes in the ES cell to the expression of the subset of genes in a germline competent ES cell, wherein the ES cell is predicted to be germline competent if the expression profile is substantially similar to the expression profile of the ES cell that is germline competent.

In one aspect of the present disclosure, the expression profiles are substantially similar if at least 80% of the genes correlate with respect to up-regulation and down-regulation. In another aspect, the expression profiles are substantially similar if at least 90% of the genes correlate with respect to up-regulation and down-regulation. In another aspect, the expression profiles are substantially similar if at least 95% or more of the genes correlate with respect to up-regulation and down-regulation. In another aspect, genes correlate where they do not differ by more than 100% when comparing expression; in another aspect, they correlate where they do not differ by more than 50% when comparing expression level; in another aspect, they correlate where they do not differ by more than 20% or less when comparing expression level.

In one aspect, the prediction is at least 50% accurate. In another aspect, the prediction is at least 60%, at least 70%, at least 80%, at least about 84%, at least 90%, or at least 95% accurate. In a specific aspect, the prediction is at least 99% accurate. In one aspect, accuracy of prediction is determined by whether the ES cell contributes to the germline in a chimeric animal into whose embryo the ES cell has been injected.

Additionally described herein is, an expression array comprising a subset of genes of an ES cell that is differentially expressed in ES cells that are germline competent as compared with ES cells that are not germline competent.

In one embodiment, the subset of genes are selected from an ES cell genome based on the criterion that the subset of genes exhibit an up-regulation or down-regulation of at least two-fold between germline competent and non-germline competent ES cells. In one embodiment, the subset of genes exhibit a two- to three-fold difference between germline competent and non-germline competent ES cells. In another embodiment, the subset of genes exhibit a three- to four-fold difference between germline competent and non-germline competent ES cells. In another embodiment, the subset of genes exhibit at least a five-fold difference between germline competent and non-germline competent ES cells. In one embodiment, all genes of the subset exhibit about the same, cited fold difference in expression. In another embodiment, a majority of the genes of the subset exhibit the same, cited fold difference in expression.

Use of an expression array to predict germline competency of a test ES cell is also described herein, comprising comparing an expression profile of a subset of genes whose expression differs between totipotent ES cells and non-totipotent ES cells, wherein an expression profile of a test ES cell that is substantially similar to an expression profile of a totipotent ES cell indicates that the test ES cell is germline competent.

In another aspect, a method is described herein for selecting a cell from an ES cell population, wherein the selected cell has an increased likelihood of transmitting to the germline when injected into an embryo, comprising selecting an ES cell from the ES cell population whose expression profile for a preselected subset of genes is substantially similar to an expression profile of an ES cell that is germline competent.

Unless otherwise specified or apparent from the context, any feature of the invention can be used in conjunction with any other feature of the invention. Other objects and advantages will become apparent from a review of the ensuing detailed description.

### BRIEF DESCRIPTION OF THE FIGURES

Fig. 1 shows microarray analysis for two training sets of ES cells, which all had normal karyotypes; the first test set (two ES cell clones) were germline competent, whereas the second test set (two ES cell clones) were not germline competent as determined, in this example, by two microinjection attempts without chimeras.

Fig. 2 shows expression profile of test sets of ES cell clones (six clones) that had been targeted, and that had not been karyotyped.

Fig. 3 shows expression profile of a clone that was germline competent ("Germline Competent"), and the same clone differentiated ("Germline Competent differentiated"); "Non Germline Competent" clones are the "Non Germine Competent" clones of Fig. 2, presented for comparison.

Fig. 4 shows the percent of correct predictions of germline competency as a function of the number of genes analyzed in an expression array.

Fig. 5 shows the expression profile of a single gene (gtl2) in two germline competent ES cells ("Germline Competent 1") and ("Germline Competent 2"); and its profile in two non-germline competent ES cell lines ("Germline Competent 1" and "Germline Competent 2").

### DETAILED DESCRIPTION

The present invention is not limited to particular methods, and experimental conditions described, as such methods and conditions may vary. It is also to be understood that the terminology used herein is for the purpose of describing particular embodiments only, and is not intended to be limiting unless indicated, since the scope of the present invention will be limited only by the appended claims.

As used in this specification and the appended claims, the singular forms "a", "an", and "the" include plural references unless the context clearly dictates otherwise. Thus for example, references to "a method" include one or more methods, and/or steps of the type described herein and/or which will become apparent to those persons skilled in the art upon reading this disclosure.

Unless stated otherwise, all technical and scientific terms and phrases used herein have the same meaning as commonly understood by one of ordinary skill in the art to which the invention belongs. Although any methods and materials similar or equivalent to those described herein can be used in the practice or testing of the present invention, the preferred methods and materials are now described.

The invention is based at least in part on the realization that it is possible to assess the suitability of an ES cell for implantation in an embryo, such that the embryo develops into an animal wherein the ES cell contributes to the animal's germline.

The ability of an implanted ES cell to contribute to the development of an embryo and give rise to a chimeric animal that comprises the genetic material of the implanted ES cell, commonly referred to as germline competence, depends to a great extent on the totipotency of the ES cell.

As ES cells differentiate, they lose totipotency. Although ES cells that have differentiated and lost totipotency may retain some level of pluripotency, ES cells that are not totipotent are generally not useful in producing transgenic animals. ES cells lacking totipotency do not contribute to the germline of the animal and thus do not generally give rise to chimeras capable of transmitting the genetic material of the ES cell in the animal's germline-i.e., they are not generally germline competent. The differentiation state of an ES cell, or lack of differentiation, is reflected in the gene expression profile of the ES cell. Thus, based on an ES cell's expression profile, it may be possible to predict the potential of the ES cell to contribute to the germline of an embryo into which the ES cell is implanted.

The ability to predict with relative accuracy which ES cells in a population of ES cells, or between populations of ES cells, have the highest likelihood for germline competence is important. As germline competence drops, the number of ES cell clones that must be injected rises dramatically, as does the number of resulting chimeras that must be screened for germline competence. Injection and screening steps are both costly and time consuming. Further, proceeding in the face of a relatively unpredictable frequency of germline requires making more targeted clones (since more injections are necessary). Thus, increasing the likelihood that the ES clones injected are germline competent can result in large savings of resources and time.

However, beyond economic costs and adaptability to a high throughput set up, an assay that can reliably predict the GLT potential of an ES cell clone or line would also be of paramount utility in the following situations:

The ability to retain germline competence is a property that could be subject to or affected by the genetic background of the ES cell. Generally, inbred ES cells lines frequently display low germline transmission rates, with some B/6 lines displaying less than 40% germline transmission. Since the use of ES cells derived from inbred cell lines is becoming increasingly desirable, the ability to predict which targeted clones have a relatively high germline transmission rate would be of very high utility. For example, the methods and compositions provided herein would be of very high utility to large scale, high throughput knockout projects such as, for example, NIH's Knockout Mouse Project (KOMP).

In another aspect, improved methods for identifying germline competent ES cells would facilitate selecting ES cell clones that should be deposited into targeted ES cell repositories. Only those clones that have a relatively higher probability of germline competency will be stored, thus saving storage space and reducing the probability of spreading non germline competent clones throughout the scientific community.

In another aspect, improved methods for identifying germline competent ES cells would serve as a quality control step following thawing and expansion of clones for distribution purposes. Germline competency could be assessed prior to distribution, and clonal lines that have lost germline competency due to storage, handling, thawing, or expansion conditions can be eliminated prior to distribution.

In another aspect, improved methods for identifying germline competent ES cells would minimize the risk of using non-germline competent ES cell clones in projects that require multiple targeting steps, such as, for example, projects wherein large loci are sequentially modified, e.g., projects in which distal regions of the same chromosome must be modified, or projects that require repeated targeting in order to modify gene clusters or families of closely related genes.

In order to prepare a training set for comparing expression profiles and developing a method for predicting germline competency, two sets of ES cell clones were selected as training sets. The first set of ES cell clones (two clones in a set) had been sequentially targeted seven times using homologous recombination using standard homologous recombination methods. The second set (two clones in a set) had been sequentially targeted eight times. Both sets were karyotyped. All sets had normal karyotypes.

Each set of ES cells were grown cells were thawed, passed on feeder cells, and then grown on gelatin under standard culture conditions to prevent cell differentiation. The ES cells were F1 H4 ES cells. Cells were grown in triplicate and expanded to six-well format. RNA was extracted from each well using Qiagen's RNAEasy kit. Microarray analysis was done on the RNA prepared from each clone.

For each data set, targeted ES cells were injected into blastocysts or 8-cell stage mouse embryos, and a total of 50 or more blastocysts were transformed into pseudo-pregnant female mice. If chimeras are born, they are tested for germline competency by determining whether they produced F1 heterozygotes. Germline transmission is defined as chimeras that are born that will produce an F1 heterozygote. Non-germline transmission is defined as the lack of production of chimera, or the production of a chimera that does not contribute to the germline.

Briefly, ES cell clones were thawed and plated the presence of Lif and grown on feeders (standard culture media for maintaining the undifferentiated phenotype). RNA was extracted using Qiagen's RNAEasy kit, purified RNA was labeled using Agilent's low-input RNA amplification kit, and hybridized to an Agilent mouse whole genome array (Cat. No. 4122A). Arrays were scanned using Agilent's scanner and data was extracted using Agilent's feature extraction software.

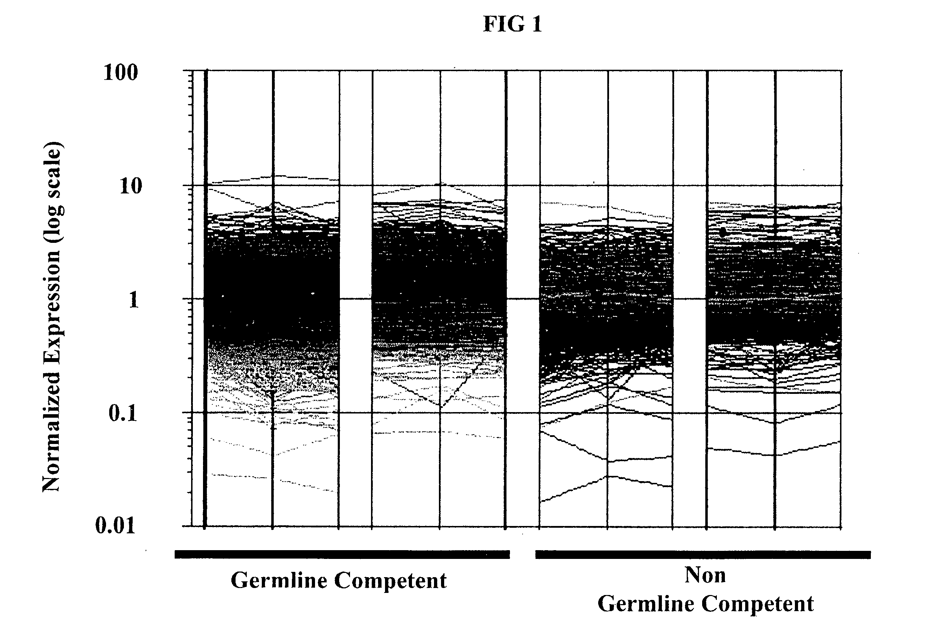

As shown in Fig. 1, microarray analysis each of the ES cell training sets revealed that the ES cells that went germline had similar expression profile s (see the two training sets, shown in triplicate, under "Germline Competent"). Genes above "1" represent up-regulated genes; genes below "1" represent down-regulated: As can be seen from the training set data of Fig. 1, germline competent ES cells have characteristic expression profile s of up-regulated and down-regulated genes. The second half of Fig. 1 ("Non Germline Competent") shows two sets of non-germine transmitting ES cells. As can be seen by comparison with the expression profile for germline transmitting ES cells, many genes that are up-regulated in germline competent ES cells are down-regulated in non-germline competent ES cells, and vice versa. In short, Fig. 1 illustrates that a loss of totipotency correlates with an observable expression profile in ES cells. This expression profile can be predictive of loss of totipotency and, thus, of germline competency in ES cells.

In Fig. 1, "Germline Competent" is defined as germline transmission; "non germline competent" is defined in this experiment as failure of germline transmission after two microinjection attempts. The microarray used was an Agilent array (Cat. No. G4122A). The Agilent array comprises about 40,000 genes. The microarray profile shows expression of a subset of about 362 genes that represents a set of genes that are differentially expressed between germline competent and non-germline competent ES cells. The 362 genes (Table 1) were selected by using a t-test analysis on germline and non-germline competent array profiles, selecting genes that exhibit at least a 2-fold up-regulation or down-regulation between germline competent and non-germline competent ES cells. In each of the figures, each vertical line represents a single sample (results shown in triplicate; three samples per experiment).

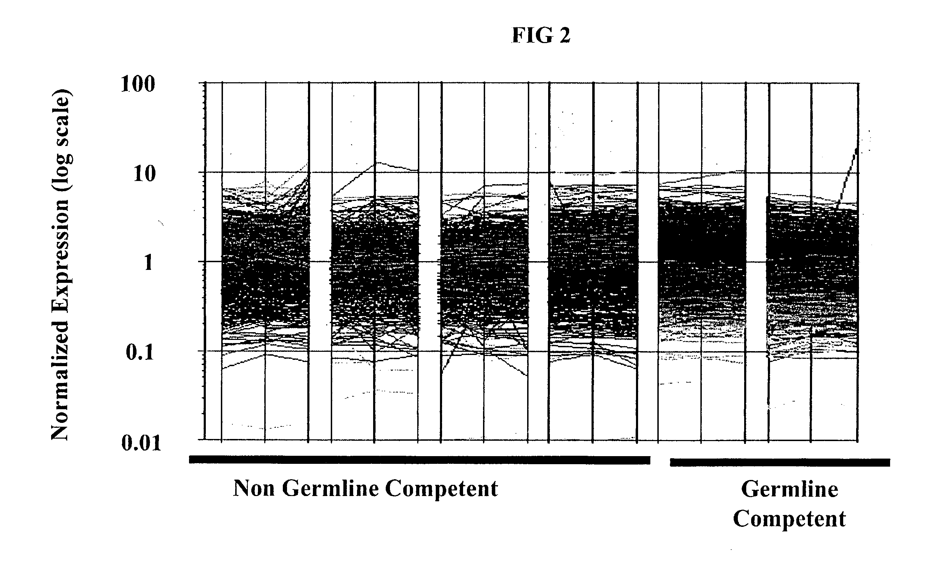

Fig. 2 shows expression profile of a test set of ES cell clones (six clones) that had been targeted, and that had not been karyotyped. Based on the expression profiling of the test set of Fig. 1, it is apparent from Fig. 2 that the final two clones (at right) would be expected to be germline competent based solely on the expression data shown, whereas the first four clones (at right) would be expected not to be germline competent.

Each clone of Fig. 2 was microinjected into a mouse blastocyst or a mouse embryo at the 8-cell stage. One of the "Germline Competent" clones at the right of Fig. 2 was determined to be germline competent (expression array profiling predicts that the other clone would be germline competent). The "Non Germline Competent" clones at the left of Fig. 2 were not germline competent.

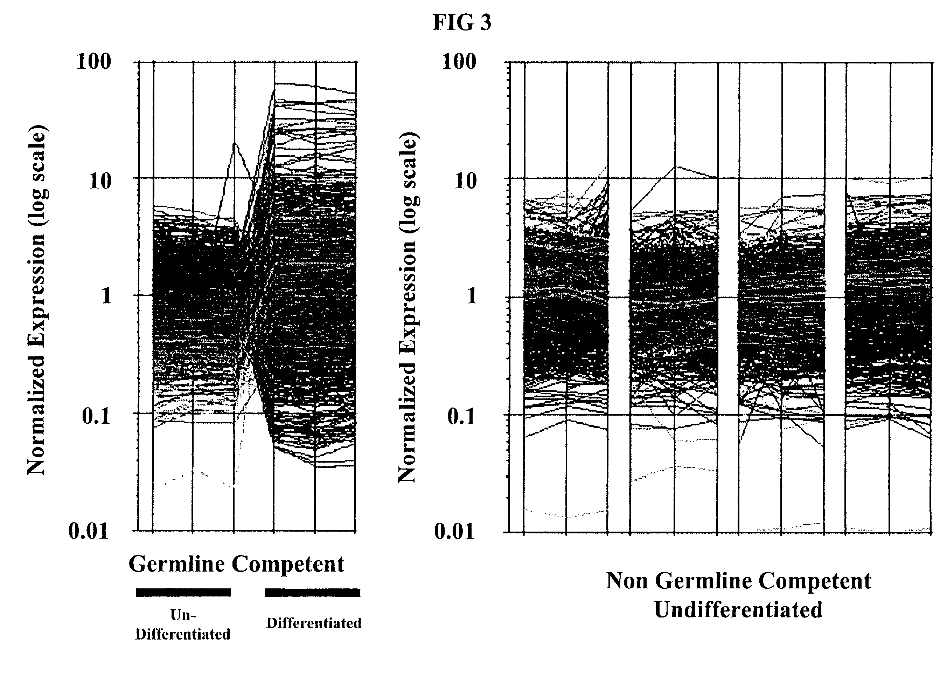

Fig. 3 shows expression profile of a clone that produced germline ("Germline Competent"), and the same clone differentiated ("Germline Competent differentiated"); "Non Germline Competent" clones are the "Non Germline Competent" clones of Fig. 2, presented for comparison. Fig. 3 illustrates that when a germline competent ES cell clone is differentiated (i.e., when it loses totipotency), its expression profile resembles that of a non-germline competent ES cell. For differentiation, ES cells were cultured in the absence of Lif, feeders, and gelatin, and grown in suspension for 14 days to develop EB bodies (embryonic bodies). EB bodies were trypsinized and plated out for a further seven days in the absence of Lif and in the absence of feeders and in the absence of gelatin, and RNA for expression profiling was prepared as described previously. No specific differentiation factors were administered.

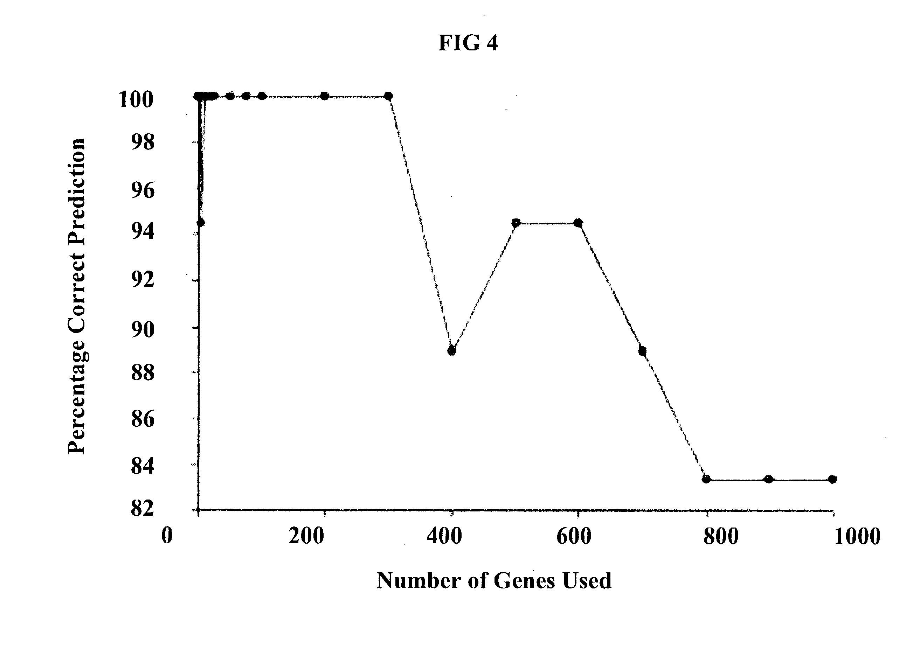

Fig. 4 shows the percent of correct predictions of germline competency of ES cells as a function of the number of genes analyzed in an expression array. The support vector machine class prediction method as implemented in GeneSpring (Agilent) was used. One hundred percent accuracy in predictive ability is achievable from about ten to about 292 genes. At least 93% accuracy is achievable from about five genes to about 25 genes. At ten genes or more, predictability is up to about 100%. The identities of the 362 genes used in the training set are shown in 6. The identities of the subsets of genes used in Fig. 4 are shown in Tables 1 through 8.

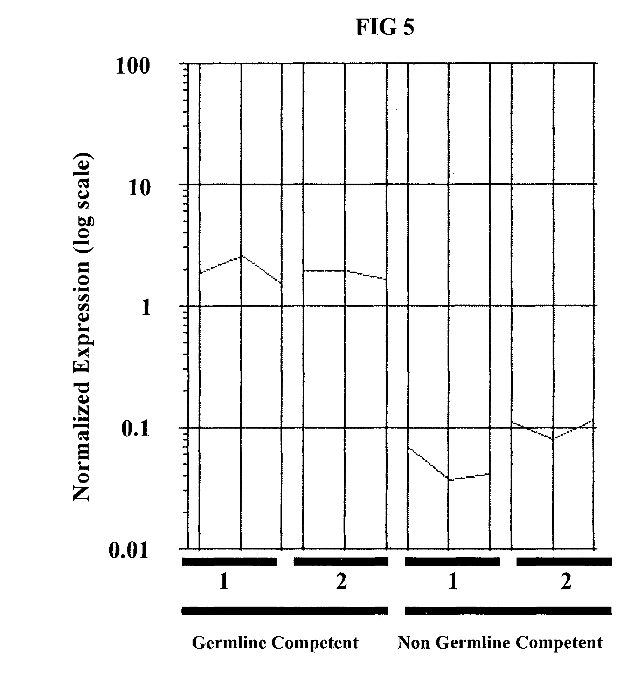

Fig. 5 shows the expression profile of a single gene (gtl2) in two germline competent ES cells ("Germline Competent 1 ") and ("Germline Competent 2"); and its profile in two non-germline competent ES cell lines ("Non Germline Competent 1" and "Non Germline Competent 2"). As is apparent from the results shown in Fig. 5, it is possible to predict germline transmission competency using an expression profile for a single gene.

Table 1 shows a subset of 362 genes that were used as a predictor of germline competence. This subset of 362 genes was further reduced to 292 genes (Table 2), 200 genes (Table 3), 100 genes (Table 4), 50 genes (Table 5), 25 genes (Table 6), 10 genes (Table 7), and 5 genes (Table 8), and each subset displays an expression profile difference analogous to the diffence shown in Fig. 1 for the set of 362 genes. Accordingly, relatively accurate predictions of germline competence can be made on subsets of genes as small as, for example, 5 genes. Further, accurate predictions can be made for subsets as small as 3 genes (Fig. 4), or one gene (Fig. 5).

**Table 1A - Training Set Genes (Gemline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P101975 | NM_010205 | 14179 | Fgf8 | 0.412 | 0.39 | 0.413 | 0.415 | 0.454 | 0.385 |
| A_51_P102081 | NM_007847 | 13222 | Defcr-rs2 | 0.061 | 0.0426 | 0.0644 | 0.0791 | 0.158 | 0.0864 |
| A_51_P102257 | AK053112;XM_619640 | 320477 | E030037J05Rik | 1.895 | 1.871 | 1.913 | 2.03 | 2.46 | 2.096 |
| A_51_P103397 | AK078521; | 54561 | Nap113 | 2.701 | 2.645 | 2.885 | 3.303 | 3.612 | 3.031 |
| A_51_P103406 | NM_011708 | 22371 | Vwf | 3.089 | 2.827 | 2.64 | 4.179 | 4.502 | 3.84 |
| A_51_P104162 | AK053379; | 328232 | | 2.537 | 3.096 | 3.391 | 3.126 | 2.998 | 3.127 |
| A_51_P105068 | NM_027990 | 71897 | 2310010M24Rik | 0.459 | 0.371 | 0.462 | 0.48 | 0.484 | 0.498 |
| A_51_P106918 | NM_009051 | 19715 | Rex2 | 2.006 | 2.032 | 2.425 | 6.886 | 2.95 | 2.717 |
| A_51_P109649 | AK018142; | 17263 | Gtl2 | 1.183 | 1.072 | 1.216 | 1.06 | 1.024 | 0.976 |
| A_51_P113162 | AK086843; | | | 1.844 | 2.453 | 2.29 | 2.743 | 2.858 | 1.748 |
| A_51_P114606 | NM_016965 | 50884 | Nckap1 | 0.446 | 0.473 | 0.337 | 0.707 | 0.582 | 0.773 |
| A_51_P115005 | NM_010104 | 13614 | Edn1 | 0.183 | 0.137 | 0.0678 | 0.336 | 0.288 | 0.302 |
| A_51_P121635 | NM_030700 | 80884 | Maged2 | 0.378 | 0.371 | 0.409 | 0.492 | 0.475 | 0.481 |
| A_51_P124315 | NM_177649 | 227712 | BC034076 | 1.796 | 1.742 | 1.797 | 2.396 | 2.68 | 2.14 |
| A_51_P125679 | AK010725;XM_134948 | 73673 | 2410076121Rik | 2.867 | 2.332 | 2.464 | 3.518 | 3.661 | 3.727 |
| A_51_P127560 | AK051724; | 76577 | | 1.567 | 2.006 | 1.603 | 1.713 | 1.753 | 1.298 |
| A_51_P130095 | NM_010187 | 14130 | Fcgr2b | 1.115 | 0.945 | 1.055 | 1.587 | 1.618 | 1.515 |
| A_51_P140751 | NM_011397 | 20522 | SIc23a1 | 1.819 | 1.664 | 1.85 | 2.855 | 2.607 | 2.546 |
| A_51_P141660 | AK053665;XM_358382 | 67776; 235380 | Loh11cr2a | 2.996 | 3.339 | 3.08 | 2.433 | 2.601 | 2.365 |
| A_51_P144417 | NM_181750 | 226412 | R3hdm | 2.79 | 2.726 | 2.677 | 2.283 | 2.582 | 2.164 |
| A_51_P144712 | NM_178930 | 107338 | Gbf1 | 1.805 | 1.74 | 1.841 | 1.291 | 1.456 | 1.202 |
| A_51_P147651 | AK041797;XM_146705 | 245902 | | 2.797 | 2.965 | 2.694 | 2.18 | 2.325 | 1.652 |
| A_51_P149469 | NM_009608 | 11464 | Actc1 | 0.324 | 0.387 | 0.384 | 0.496 | 0.551 | 0.471 |
| A_51_P153079 | NM_133923 | 101100 | 4833441J24Rik | 1.312 | 1.174 | 1.271 | 1.222 | 1.343 | 1.299 |
| A_51_P157986 | NM_029930 | 77574 | 3321401 G04Rik | 0.426 | 0.339 | 0.345 | 0.368 | 0.318 | 0.37 |
| A_51_P158037 | NM_013505 | 13506 | Dsc2 | 0.271 | 0.249 | 0.258 | 0.437 | 0.415 | 0.453 |
| A_51_P160160 | AK017624; | 70548 | 5730437C12Rik | 2.323 | 2.175 | 2.466 | 2.08 | 1.666 | 3.278 |
| A_51_P161115 | AK050756; | | | 2.088 | 3.879 | 2.31 | 2.955 | 2.678 | 1.887 |
| A_51_P162474 | AF285577; | 107815 | Scml2 | 0.539 | 0.413 | 0.405 | 0.643 | 0.645 | 0.71 |
| A_51_P162481 | NM_133194 | 107815 | Scml2 | 0.432 | 0.357 | 0.385 | 0.594 | 0.55 | 0.618 |
| A_51_P164219 | NM_011909 | 24110 | Usp18 | 0.435 | 0.307 | 0.303 | 0.435 | 0.448 | 0.457 |
| A_51_P164504 | NM_007469 | 11812 | Apoc1 | 3.245 | 2.908 | 3.052 | 2.863 | 3.059 | 2.591 |
| A_51_P165082 | AB041601;XM_489523 | 224019 | D16Bwg1494e | 3.114 | 3.318 | 3.453 | 3.352 | 3.568 | 3.01 |
| A_51_P165244 | NM_018734 | 55932 | Gbp4 | 0.585 | 0.512 | 0.474 | 1.008 | 0.981 | 1.006 |
| A_51_P168646 | AK039580; | | | 0.442 | 0.434 | 0.509 | 0.537 | 0.548 | 0.598 |
| A_51_P171728 | NM_011926 | 26365 | Ceacam1 | 2.703 | 1.992 | 2.013 | 3.11 | 3.457 | 2.977 |
| A_51_P174943 | NM_008485 | 16782 | Lamc2 | 0.234 | 0.255 | 0.286 | 0.433 | 0.411 | 0.451 |
| A_51_P176474 | AK010466; | 76484 | 2410012C07Rik | 2.797 | 2.691 | 2.733 | 3.87 | 4.072 | 3.726 |
| A_51_P180413 | NM_176930 | 319504 | C130076O07Rik | 0.359 | 0.393 | 0.415 | 0.471 | 0.514 | 0.494 |
| A_51_P187645 | BC004786;XM_354700 | 16061 | Igh-VJ558 | 0.714 | 0.737 | 0.706 | 0.927 | 0.803 | 0.637 |
| A_51_P189631 | NM_172427 | 76455 | 2310067E19Rik | 1.139 | 0.941 | 0.985 | 1.62 | 1.653 | 1.604 |
| A_51_P191990 | AK087548; | 26563 | Ror1 | 1.628 | 1.623 | 1.626 | 1.509 | 1.588 | 1.479 |
| A_51_P193019 | NM_011157 | 19073 | Prg1 | 1.283 | 1.107 | 1.495 | 0.759 | 3.853 | 1.325 |
| A_51_P199888 | NM_026082 | 67299 | Dock7 | 0.407 | 0.403 | 0.245 | 0.558 | 0.428 | 0.546 |
| A_51_P200223 | XM_619497 | | | 2.332 | 2.259 | 2.383 | 1.575 | 1.797 | 1.553 |
| A_51_P202911 | NM_144882 | 67198 | 2810022L02Rik | 0.297 | 0.283 | 0.328 | 0.346 | 0.333 | 0.365 |
| A_51_P203955 | NM_010260 | 14469 | Gbp2 | 0.252 | 0.267 | 0.292 | 0.582 | 0.578 | 0.664 |
| A_51_P207934 | NM_199016 | 224794 | Enpp4 | 2.477 | 2.57 | 2.229 | 2.606 | 2.707 | 2.427 |
| A_51_P219166 | NM_027756 | 71306 | Mfap3I | 0.459 | 0.46 | 0.497 | 0.549 | 0.502 | 0.507 |
| A_51_P221291 | NM_009229 | 20650 | Sntb2 | 1.876 | 1.793 | 1.783 | 1.622 | 1.693 | 1.524 |
| A_51_P222773 | NM_010446 | 15376 | Foxa2 | 0.423 | 0.314 | 0.4 | 0.447 | 0.809 | 0.489 |
| A_51_P223132 | NM_031183;XM_486227 | 83395; 70549 | Sp6 | 2.329 | 2.088 | 2.196 | 1.457 | 1.639 | 1.36 |
| A_51_P223656 | NM_008484 | 16780 | Lamb3 | 0.447 | 0.289 | 0.361 | 0.538 | 0.544 | 0.402 |
| A_51_P228472 | NM_008343 | 16009 | Igfbp3 | 0.288 | 0.22 | 0.276 | 0.283 | 0.322 | 0.364 |
| A_51_P229613 | NM_008792 | 18549 | Pcsk2 | 0.512 | 0.483 | 0.515 | 0.58 | 0.58 | 0.504 |
| A_51_P231341 | AK087764; | 108160 | DOHXS9928E | 2.885 | 3.795 | 2.39 | 3.305 | 3.578 | 2.451 |
| A_51_P236267 | NM_009183 | 20452 | St8sia4 | 0.265 | 0.182 | 0.148 | 0.233 | 0.291 | 0.32 |
| A_51_P237040 | NM_008711 | 18121 | Nog | 0.266 | 0.299 | 0.281 | 0.396 | 0.368 | 0.407 |
| A_51_P240253 | NM_019662 | 56437 | Rrad | 0.316 | 0.322 | 0.314 | 0.392 | 0.337 | 0.342 |
| A_51_P240811 | NM_009290 | 20890 | Wnt8a | 0.709 | 0.589 | 0.527 | 1.532 | 0.979 | 1.578 |
| A_51_P242399 | NM_031170 | 16691 | Krt2-8 | 0.193 | 0.233 | 0.208 | 0.403 | 0.4 | 0.39 |
| qA_51_P252565 | NM_009660 | 11687 | Aloxl5 | 0.236 | 0.24 | 0.156 | 0.217 | 0.231 | 0.212 |
| A_51_P253053 | XM_618867 | | | 2.544 | 3.669 | 3.053 | 2.284 | 2.658 | 3.007 |
| A_51_P262171 | NM_008326 | 15944 | Ifi1 | 0.29 | 0.264 | 0.261 | 0.474 | 0.475 | 0.507 |
| A_51_P266403 | AK009163; | 76730 | 2310005C01Rik | 3.079 | 3.053 | 3.092 | 2.733 | 2.967 | 2.734 |
| A_51_P266644 | BC055753;XM_125538 | 140742 | | 0.424 | 0.369 | 0.474 | 0.821 | 0.739 | 0.949 |
| A_51_P268167 | BC037232;XM_621365 | 229363; 218850 | Gmps | 2.665 | 2.898 | 2.35 | 2.187 | 2.366 | 2.198 |
| A_51_P270725 | AK089493; | 102093 | Phkb | 2.083 | 3.565 | 2.709 | 2.953 | 2.589 | 2.102 |
| A_51_P272363 | NM_134160 | 171166 | Mcoln3 | 0.559 | 0.82 | 0.792 | 0.686 | 0.762 | 0.915 |
| A_51_P275435 | NM_172411 | 71874 | 2310007B03Rik | 0.368 | 0.492 | 0.206 | 0.875 | 0.83 | 0.857 |
| A_51_P276305 | NM_009292 | 20899 | Stra8 | 3.137 | 3.069 | 3.174 | 2.824 | 2.972 | 2.777 |
| A_51_P282227 | NM_020577 | 57344 | As3mt | 2.651 | 2.607 | 2.876 | 3.224 | 3.327 | 3.147 |
| A_51_P283300 | NM_023116 | 12296 | Cacnb2 | 0.373 | 0.27 | 0.335 | 0.375 | 0.321 | 0.398 |
| A_51_P285725 | NM_009309 | 20997 | T | 0.141 | 0.102 | 0.113 | 0.134 | 0.13 | 0.141 |
| A_51_P289341 | AK030946;XM_141372 | 241639 | 5830467J12Rik | 0.436 | 0.286 | 0.249 | 0.623 | 0.601 | 0.689 |
| A_51_P291227 | NM_001005508 | 226652 | 6030405P05Rik | 1.876 | 1.826 | 1.86 | 2.054 | 2.185 | 2.004 |
| A_51_P294505 | NM_025658 | 66607 | Ms4a4d | 0.63 | 0.414 | 0.365 | 0.532 | 0.521 | 0.629 |
| A_51_P294566 | AK020687; | 78083 | 9930116N10Rik | 2.623 | 2.571 | 2.706 | 3.706 | 4.077 | 3.155 |
| A_51_P297131 | NM_009502 | 22330 | Vcl | 0.402 | 0.372 | 0.378 | 0.411 | 0.377 | 0.401 |
| A_51_P298266 | NM_025681 | 66643 | Lix1 | 0.507 | 0.414 | 0.416 | 0.554 | 0.584 | 0.54 |
| A_51_P302377 | NM_008009 | 14181 | Fgfbp1 | 0.258 | 0.246 | 0.273 | 0.413 | 0.381 | 0.451 |
| A_51_P305019 | AK020601; | 78423 | 9530049005Rik | 1.297 | 1.416 | 1.484 | 1.43 | 1.551 | 1.387 |
| A_51_P311038 | BC009666;XM_134720 | 57267 | Apba3 | 0.334 | 0.341 | 0.339 | 0.446 | 0.472 | 0.482 |
| A_51_P316733 | AK173008; | 330914 | Grit | 1.501 | 1.439 | 1.367 | 1.586 | 1.703 | 1.516 |
| A_51_P318577 | AK010729; | 73671 | 2410078J06Rik | 3.283 | 4.227 | 3.815 | 6.599 | 6.454 | 5.366 |
| A_51_P321579 | XM_131865;XM_196334 | 433809 | LOC433809 | 2.839 | 2.589 | 2.323 | 1.805 | 1.942 | 1.521 |
| A_51 P327011 | NM_016744 | 18573 | Pde1a | 0.252 | 0.126 | 0.236 | 0.377 | 0.349 | 0.295 |
| A_51_P330380 | XM_130138;XM_145466 | 243881 | 4921517N04Rik | 0.448 | 0.326 | 0.174 | 0.467 | 0.44 | 0.467 |
| A_51_P333253 | NM_178440 | 246177 | Myo1g | 1.419 | 1.413 | 1.621 | 1.544 | 1.504 | 1.394 |
| A_51_P334093 | NM_026228 | 67547 | Slc39a8 | 0.208 | 0.0771 | 0.105 | 0.19 | 0.198 | 0.231 |
| A_51_P335460 | NM_009132 | 20259 | Scin | 0.718 | 0.631 | 0.719 | 0.678 | 0.674 | 0.666 |
| A_51_P337125 | NM_010566 | 16331 | Inpp5d | 3.162 | 2.936 | 3.033 | 4.399 | 4.495 | 4.161 |
| A_51_P341137 | AK035176; | 224829 | Trerf1 | 1.935 | 1.833 | 2.021 | 2.024 | 2.334 | 1.984 |
| A 51_P343129 | AK012079; | 70472 | Atad2 | 3.752 | 4.107 | 4.116 | 2.307 | 2.496 | 1.87 |
| A_51_P345422 | NM_010747 | 17096 | Lyn | 0.244 | 0.247 | 0.256 | 0.373 | 0.392 | 0.424 |
| A_51_P352572 | NM_019701 | 56365 | Clcnkb | 5.193 | 4.267 | 4.344 | 4.514 | 5.226 | 4.635 |
| A_51_P353221 | NM_011582 | 21828 | Thbs4 | 0.342 | 0.418 | 0.408 | 0.589 | 0.504 | 0.543 |
| A_51_P354265 | NM_009256 | 20723 | Serpinb9 | 0.132 | 0.108 | 0.0848 | 0.29 | 0.281 | 0.346 |
| A_51_P354307 | NM_172469 | 209195 | Clic6 | 0.43 | 0.319 | 0.329 | 0.398 | 0.398 | 0.387 |
| A_51 P356003 | NM_001007580 | 333564 | Gm784 | 0.494 | 0.445 | 0.513 | 0.509 | 0.483 | 0.534 |
| A_51_P356883 | NM_008728 | 18162 | Npr3 | 0.397 | 0.367 | 0.304 | 0.418 | 0.383 | 0.399 |
| A_51_P358344 | NM_007732 | 12821 | Col17a1 | 3.116 | 2.917 | 3.011 | 3.334 | 3.243 | 2.913 |
| A_51_P358714 | AK031433; | | | 2.043 | 2.448 | 1.68 | 2.647 | 2.627 | 1.899 |
| A_51_P378051 | NM_009675 | 11754 | Aoc3 | 2.988 | 3.162 | 3.341 | 3.251 | 3.583 | 3.381 |
| A_51_P378371 | | | | 0.454 | 0.542 | 0.39 | 0.468 | 0.484 | 0.468 |
| A_51_P381069 | XM_132045 | 231252 | Chrna9 | 1.424 | 2.361 | 3.023 | 3.047 | 2.764 | 2.469 |
| A_51_P387528 | AK019664; | 78801 | Ak7 | 2.693 | 2.814 | 2.57 | 3.786 | 3.834 | 3.71 |
| A_51_P393518 | BC016539; | 240793 | | 0.393 | 0.238 | 0.292 | 0.344 | 0.296 | 0.462 |
| A_51_P397003 | XM_133990 | 68904 | 1110065L07Rik | 0.45 | 0.438 | 0.438 | 0.542 | 0.479 | 0.613 |
| A_51_P398766 | NM_010259 | 14468 | Gbp1 | 0.374 | 0.372 | 0.361 | 0.894 | 0.876 | 0.949 |
| A_51_P404193 | NM_022435 | 64406 | Sp5 | 0.194 | 0.182 | 0.19 | 0.214 | 0.202 | 0.207 |
| A_51_P405375 | NM_175358 | 108672 | Zdhhc15 | 0.458 | 0.438 | 0.301 | 0.668 | 0.553 | 0.495 |
| A_51_P407323 | NM_007976 | 14067 | F5 | 0.17 | 0.072 | NO DATA | 0.352 | 0.305 | 0.242 |
| A_51_P410813 | AK079732; | | | 2.732 | 2.716 | 2.559 | 2.898 | 2.605 | 2.3 |
| A_51_P412817 | NM_177617 | 216851 | D330014H01Rik | 3.151 | 3.039 | 3.326 | 2.136 | 2.194 | 1.865 |
| A_51_P418029 | NM_175541 | 245631 | Mum1l1 | 0.319 | 0.265 | 0.278 | 0.419 | 0.397 | 0.534 |
| A_51_P418375 | NM_023844 | 67374 | Jam2 | 1.691 | 1.467 | 1.625 | 2.142 | 2.132 | 2.288 |
| A_51_P418884 | NM_001013789 | 432823 | | 1.678 | 2.207 | 2.11 | 2.759 | 2.736 | 2.272 |
| A_51_P423859 | NM_007601 | 12335 | Capn3 | 2.32 | 2.027 | 1.637 | 2.955 | 2.666 | 3.034 |
| A_51_P437050 | BC072664; | 233328 | Lrrk1 | 0.339 | 0.28 | 0.261 | 0.525 | 0.503 | 0.498 |
| A_51_P442894 | AK048310; | 621603 | C130048D07Rik | 2.632 | 2.364 | 2.281 | 3.657 | 3.714 | 3.102 |
| A_51_P447189 | AK041326;XM_358090 | 385154 | | 2.336 | 2.35 | 2.641 | 3.32 | 2.976 | 3.108 |
| A_51_P455647 | NM_009801 | 12349 | Car2 | 0.335 | 0.288 | 0.301 | 0.568 | 0.516 | 0.638 |
| A_51_P455997 | Y13832; | 17263 | Gtl2 | 1.505 | 1.455 | 1.44 | 0.998 | 1.082 | 0.998 |
| A_51_P457187 | NM_028075 | 72049 | Tnfrsf13c | 2.82 | 3.275 | 3.026 | 2.654 | 3.432 | 2.546 |
| A_51_P463789 | BC051947; | 58212 | | 3.253 | 2.961 | 3.321 | 2.093 | 2.306 | 1.934 |
| A_51_P463791 | BC051947; | 58212 | | 3.074 | 2.866 | 2.865 | 2.058 | 2.064 | 1.757 |
| A_51_P463846 | NM_145545 | 229900 | 9830147J24Rik | 0.694 | 0.519 | 0.59 | 0.86 | 0.816 | 0.817 |
| A_51_P467505 | NM_013689 | 21682 | Tec | 0.28 | 0.242 | 0.227 | 0.502 | 0.464 | 0.499 |
| A_51_P473953 | XM_358600;XM_355454 | 381498 | 4930592C13Rik | 0.42 | 0.306 | 0.298 | 0.365 | 0.365 | 0.38 |
| A_51_P478930 | AK080016; | | | 2.436 | 2.701 | 2.337 | 2.779 | 2.919 | 2.466 |
| A_51_P480311 | NM_010168 | 14061 | F2 | 1.991 | 1.809 | 1.969 | 2.465 | 2.4 | 1.874 |
| A_51_P481777 | NM_018756 | 54382 | Tcstv1 | 1.532 | 1.822 | 1.792 | 2.619 | 3.155 | 2.956 |
| A_51_P481958 | NM_016674 | 12737 | Cldn1 | 0.867 | 0.572 | 0.569 | 0.535 | 0.328 | 0.499 |
| A_51_P485985 | NM_134163 | 171170 | Mbnl3 | 0.223 | 0.181 | 0.161 | 0.625 | 0.543 | 0.697 |
| A_51_P487004 | NM_027732 | 71241 | Dmrtc2 | 10.3 | 11.69 | 10.98 | 5.024 | 6.192 | 5.685 |
| A_51_P493682 | XM_134870;XM_140758 | 72672 | Rasl12 | 2.563 | 2.641 | 2.403 | 3.095 | 3.051 | 2.581 |
| A_51_P497100 | NM_010706 | 16855 | Lgals4 | 2.115 | 2.049 | 2.368 | 1.716 | 1.861 | 1.527 |
| A_51_P499854 | NM_010284 | 14600 | Ghr | 0.407 | 0.375 | 0.412 | 0.413 | 0.429 | 0.489 |
| A_51_P507880 | NM_028980 | 74521 | 8430415E04Rik | 0.223 | 0.221 | 0.235 | 0.35 | 0.326 | 0.332 |
| A_51_P512085 | NM_130449 | 140792 | Colec12 | 0.335 | 0.364 | 0.296 | 0.382 | 0.368 | 0.385 |
| A_51_P512172 | AK010953;XM_125673 | 52463 | Cxxc6 | 2.864 | 2.384 | 2.216 | 2.184 | 2.11 | 2.068 |
| A_51_P514177 | NM_028838 | 74249 | Lrrc2 | 3.542 | 3.2 | 3.896 | 3.884 | 4.114 | 3.603 |
| A_51_P520175 | AK076272; | 12223 | Btc | 0.505 | 0.139 | 0.208 | 0.462 | 0.57 | 0.379 |
| A_51_P521084 | NM_025626 | 66540 | 3110001A13Rik | 0.408 | 0.406 | 0.391 | 0.474 | 0.456 | 0.494 |
| A_52_P100042 | AK045143; | 320938 | Tnpo3 | 1.761 | 1.695 | 1.74 | 1.697 | 1.665 | 1.526 |
| A_52_P1011737 | AK039096; | 106258 | Al790442 | 1.846 | 1.977 | 1.978 | 2.087 | 2.67 | 2.065 |
| A_52_P1013441 | | | | 3.248 | 3.164 | 3.191 | 4.558 | 5.61 | 4.683 |
| A_52_P103929 | NM_009306 | 20979 | Syt1 | 0.382 | 0.356 | 0.39 | 0.419 | 0.498 | 0.423 |
| A_52_P1044262 | AK051949; | | | 1.478 | 1.257 | 1.19 | 1.611 | 1.769 | 1.41 |
| A_52_P1044696 | AK087281; | | | 1.535 | 1.733 | 1.77 | 1.994 | 2.551 | 1.772 |
| A_52_P1075773 | AK036921; | 399602 | 9930024M15Rik | 1.958 | 2.689 | 1.619 | 2.065 | 2.285 | 1.59 |
| A_52_P1084149 | AK051413; | 553138 | 9930021J03Rik | 1.669 | 1.913 | 1.945 | 1.806 | 2.202 | 1.595 |
| A_52_P1108139 | AK053079; | | | 1.801 | 2.214 | 1.538 | 1.924 | 2.168 | 1.724 |
| A_52_P1124598 | AK081090; | | | 1.689 | 1.334 | 1.065 | 1.621 | 1.94 | 1.111 |
| A_52_P113190 | NM_053214 | 17916 | Myo1f | 1.428 | 1.513 | 1.396 | 1.988 | 1.959 | 2.083 |
| A_52_P1172548 | AK084146; | 13204 | Dhx15 | 2.924 | 3.082 | 2.339 | 3.164 | 3.492 | 2.732 |
| A_52_P1180315 | AK049088; | 74022 | 3930401K13Rik | 1.789 | 1.737 | 1.505 | 1.313 | 1.364 | 1.207 |
| A_52_P1188270 | AK042362; | | | 3.076 | 1.828 | 1.917 | 2.932 | 3.046 | 2.87 |
| A_52_P1196751 | AK086488; | | | 2.217 | 2.193 | 2.198 | 2.38 | 2.677 | 2.156 |
| A_52_P126158 | NM_008326 | 15944 | Ifi1 | 0.237 | 0.208 | 0.22 | 0.415 | 0.412 | 0.425 |
| A_52_P129428 | BC049774; | 57265 | Fzd2 | 0.482 | 0.364 | 0.221 | 0.539 | 0.518 | 0.547 |
| A_52_P132608 | NM_172685 | 229731 | Slc25a24 | 0.358 | 0.291 | 0.25 | 0.504 | 0.401 | 0.432 |
| A_52_P135873 | XM_132015;XM_488584 | 231128 | BC037112 | 1.243 | 1.332 | 1.392 | 1.507 | 1.53 | 1.464 |
| A_52_P13815 | NM_010686 | 16792 | Laptm5 | 0.991 | 1.234 | 0.785 | 1.685 | 1.566 | 1.469 |
| A_52_P147870 | NM_029963 | 77721 | Mrps5 | 2.14 | 2.028 | 2.027 | 1.789 | 1.827 | 1.856 |
| A_52_P148222 | BC055037; | 224139 | 4930428L02Rik | 0.273 | 0.151 | 0.238 | 0.426 | 0.424 | 0.55 |
| A_52_P152983 | AK011739; | 26886 | Cenph | 2.134 | 3.107 | 2.171 | 2.114 | 2.408 | 2.327 |
| A_52_P153685 | AK129323; | 70422 | 2810417D08Rik | 0.452 | 0.397 | 0.407 | 0.587 | 0.526 | 0.735 |
| A_52_P159095 | AK051960; | 17685 | | 2.867 | 2.413 | 1.869 | 3.388 | 3.17 | 2.951 |
| A_52_P163148 | AB010370; | 544945 | | 2.883 | 2.86 | 2.64 | 3.58 | 3.156 | 3.085 |
| A_52_P164524 | NM_013628 | 18548 | Pcsk1 | 0.728 | 0.727 | 0.407 | 0.826 | 0.801 | 0.71 |
| A_52_P165497 | NM_011385 | 20481 | Ski | 2.312 | 2.37 | 2.474 | 1.296 | 1.485 | 1.406 |
| A_52_P165610 | NM_153534 | 210044 | Adcy2 | 0.452 | 0.354 | 0.394 | 0.474 | 0.421 | 0.374 |
| A_52_P16973 | AK081934; | 320391 | D930017J03Rik | 2.586 | 2.277 | 2.281 | 2.137 | 2.272 | 1.872 |
| A_52_P175685 | NM_008390 | 16362 | Irf1 | 0.401 | 0.37 | 0.3 | 0.663 | 0.637 | 0.749 |
| A_52_P176333 | NM_001005787 | 12695 | Cipp | 0.372 | 0.357 | 0.365 | 0.452 | 0.392 | 0.42 |
| A_52_P179250 | AK129127; | 230249 | Al314180 | 5.166 | 6.292 | 4.565 | 8.052 | 10.48 | 5.993 |
| A_52_P183368 | NM_011535 | 21386 | Tbx3 | 1.964 | 1.81 | 1.931 | 2.023 | 2.272 | 2.026 |
| A_52_P18887 | NM_011468 | 20755 | Sprr2a | 0.21 | 0.171 | 0.214 | 0.282 | 0.277 | 0.25 |
| A_52_P196568 | NM_144513 | 17263 | Gtl2 | 1.892 | 2.538 | 1.516 | 1.938 | 1.959 | 1.652 |
| A_52_P197826 | NM_010397 | 15039 | H2-T22 | 0.541 | 0.505 | 0.41 | 0.653 | 0.634 | 0.622 |
| A_52_P199633 | NM_199146 | 209387 | LOC209387 | 0.492 | 0.426 | 0.236 | 0.608 | 0.439 | 0.597 |
| A_52_P199922 | | | | 3.785 | 1.908 | 2.417 | 2.334 | 2.502 | 2.545 |
| A_52_P200458 | NM_010242 | 14345 | Fut4 | 0.429 | 0.343 | 0.355 | 0.453 | 0.391 | 0.512 |
| A_52_P206573 | NM_027427 | 70439 | Taf15 | 2.14 | 3.003 | 2.282 | 1.904 | 2.123 | 1.956 |
| A_52_P211956 | NM_025658 | 66607 | Ms4a4d | 0.61 | 0.455 | 0.457 | 0.534 | 0.519 | 0.685 |
| A_52_P214042 | NM_028125 | 72147 | Btbd4 | 0.512 | 0.42 | 0.228 | 0.756 | 0.669 | 0.611 |
| A_52_P21595 | NM_028238 | 72433 | Rab38 | 0.234 | 0.188 | 0.168 | 0.439 | 0.467 | 0.472 |
| A_52_P21628 | NM_019978 | 13175 | Dcamkl1 | 0.543 | 0.459 | 0.338 | 0.471 | 0.5 | 0.397 |
| A_52_P223083 | BC038323; | 72096 | 2010208K18Rik | 4.486 | 6.15 | 3.745 | 3.784 | 3.918 | 3.378 |
| A_52_P224125 | XM_622260 | | | 1.795 | 1.653 | 1.727 | 1.625 | 1.654 | 1.663 |
| A_52_P233673 | NM_020594 | 57432 | Zc3hdc8 | 3.001 | 3.71 | 2.727 | 1.913 | 2.05 | 1.886 |
| A_52_P237357 | AF095690; | | | 1.813 | 1.675 | 1.846 | 1.603 | 1.706 | 1.432 |
| A_52_P238468 | XM_133238 | 381853 | Gm160 | 2.307 | 2.173 | 2.136 | 1.929 | 1.906 | 1.683 |
| A_52_P240706 | NM_007543 | 26367 | Ceacam2 | 2.285 | 1.866 | 1.892 | 2.654 | 2.556 | 2.628 |
| A_52_P253179 | AK077477; | 16009 | lgfbp3 | 0.397 | 0.511 | 0.257 | 0.517 | 0.468 | 0.482 |
| A_52_P255224 | AK032711;XM_486482 | 234371 | BC021367 | 2.31 | 3.108 | 2.526 | 2.817 | 2.716 | 2.5 |
| A_52_P255250 | NM_009543 | 22644 | Rnf103 | 0.532 | 0.371 | 0.413 | 0.471 | 0.455 | 0.491 |
| A_52_P258116 | NM_009521 | 22415 | Wnt3 | 0.156 | 0.164 | 0.16 | 0.312 | 0.309 | 0.296 |
| A_52_P26416 | AF106279; | 16782 | | 0.254 | 0.252 | 0.241 | 0.358 | 0.391 | 0.378 |
| A_52_P269158 | NM_001003948 | 98496 | AL024069 | 0.316 | 0.33 | 0.33 | 0.388 | 0.393 | 0.443 |
| A_52_P274960 | NM_001005508 | 226652 | 6030405P05Rik | 1.83 | 1.82 | 1.948 | 2.07 | 2.181 | 2.076 |
| A_52_P277415 | AK077313; | 76252 | 0610006014Rik | 1.282 | 1.291 | 1.224 | 0.99 | 1.044 | 0.981 |
| A_52_P282000 | AK020248; | 13800 | Enah | 2.7 | 2.435 | 2.683 | 2.632 | 2.812 | 2.59 |
| A_52_P284243 | XM_359390;XM_358896 | 384373 | LOC386546 | 0.671 | 0.371 | 0.191 | 0.435 | 0.369 | 0.372 |
| A_52_P284981 | NM_007462 | 11789 | Apc | 1.566 | 2.024 | 1.253 | 1.307 | 1.36 | 1.114 |
| A_52_P286166 | NM_001002927 | 18619 | Penk1 | 0.497 | 0.324 | 0.339 | 0.671 | 0.658 | 0.647 |
| A_52_P286528 | BC055771; | 102954 | Nudt11 | 0.411 | 0.273 | 0.263 | 0.433 | 0.371 | 0.517 |
| A_52_P286970 | XM_131162;XM_127565 | 76561 | Snx7 | 0.44 | 0.492 | 0.435 | 0.608 | 0.611 | 0.895 |
| A_52_P287576 | AK036308; | | | 1.703 | 2.271 | 1.347 | 2.425 | 2.794 | 1.7 |
| A_52_P289559 | NM_177784 | 277396 | C130068N17Rik | 0.448 | 0.222 | 0.378 | 0.393 | 0.428 | 0.506 |
| A_52_P293443 | AK087208; | 13819 | | 1.278 | 1.075 | 1.13 | 1.496 | 1.604 | 1.349 |
| A_52_P295933 | AK045060; | 14051 | B130023L16Rik | 0.336 | 0.293 | 0.594 | 0.466 | 0.5 | 0.466 |
| A_52_P296244 | BC048734; | | | 0.369 | 0.373 | 0.292 | 0.457 | 0.42 | 0.426 |
| A_52_P301821 | NM_053078 | 27528 | D0H4S114 | 0.442 | 0.427 | 0.469 | 0.472 | 0.525 | 0.486 |
| A_52_P30386 | AK084172; | | | 1.86 | 1.591 | 1.593 | 1.74 | 1.966 | 1.403 |
| A_52_P306387 | NM_178709 | 235315 | D130054N24Rik | 2.399 | 3.176 | 2.415 | 1.899 | 2.109 | 1.481 |
| A_52_P307801 | NM_007847 | 13222 | Defcr-rs2 | 0.119 | 0.091 | 0.0705 | 0.133 | 0.175 | 0.108 |
| A_52_P309718 | AK173099; | 218850 | D14Abb1e | 2.324 | 2.579 | 2.18 | 2.614 | 2.72 | 2.711 |
| A_52_P317273 | NM_172846 | 240960 | A230079K17Rik | 0.567 | 0.535 | 0.323 | 0.739 | 0.524 | 0.595 |
| A_52_P320170 | NM_144787 | 76804 | Jmjd2c | 1.626 | 2.821 | 1.963 | 2.515 | 2.362 | 2.293 |
| A_52_P322927 | XM_487182 | 435271 | LOC435271 | 2.109 | 2.45 | 2.167 | 3.906 | 4.277 | 3.453 |
| A_52_P324934 | AK045391; | 16403 | | 1.983 | 2.443 | 1.872 | 2.691 | 2.881 | 2.226 |
| A_52_P32502 | AK020822; | 77944 | A930007B11 Rik | 2.593 | 4.558 | 2.534 | 2.973 | 3.231 | 2.237 |
| A_52_P3304 | NM_080446 | 117599 | Helb | 2.823 | 2.774 | 3.121 | 2.206 | 2.73 | 2.462 |
| A_52_P335089 | BE198552; | | | 1.331 | 1.348 | 1.455 | 1.151 | 1.229 | 0.977 |
| A_52_P336259 | AK087367;XM_485576 | 319467 | A930002C04Rik | 1.736 | 2.368 | 2.023 | 1.677 | 1.679 | 1.401 |
| A_52_P344424 | NM_010720 | 16891 | Lipg | 0.568 | 0.423 | 0.304 | 0.628 | 0.636 | 0.828 |
| A_52_P345626 | AY149175; | 386626 | Mzf6d | 2.677 | 3.059 | 3.002 | 3.203 | 2.748 | 3.757 |
| A_52_P34823 | NM_010747 | 17096 | Lyn | 0.232 | 0.214 | 0.173 | 0.248 | 0.256 | 0.228 |
| A_52_P350477 | NM_139295 | 193813 | Mcfd2 | 0.43 | 0.349 | 0.245 | 0.661 | 0.554 | 0.759 |
| A_52_P354363 | NM_026574 | 68142 | | 1.74 | 1.737 | 1.937 | 1.525 | 1.61 | 1.378 |
| A_52_P354785 | AK037929; | 14852 | Gspt1 | 2.114 | 1.965 | 1.835 | 1.904 | 1.907 | 2.055 |
| A_52_P355276 | NM_001002764 | 103677 | AI317223 | 2.549 | 2.993 | 2.548 | 1.703 | 1.935 | 1.511 |
| A_52_P358951 | AK081999;XM_134736 | 271457; 22688 | Rab5a | 0.417 | 0.378 | 0.448 | 0.464 | 0.449 | 0.46 |
| A_52_P359010 | AK129478; | 72193 | Sfrs2ip | 2.152 | 2.149 | 2.122 | 2.349 | 2.38 | 2.318 |
| A_52_P361534 | NM_009521 | 22415 | Wnt3 | 0.0992 | 0.0782 | 0.0774 | 0.211 | 0.198 | 0.243 |
| A_52_P362805 | AK044730; | 269254 | Als4 | 2.262 | 2.248 | 2.26 | 1.818 | 1.967 | 1.718 |
| A_52_P364021 | NM_026713 | 68393 | Mogat1 | 0.585 | 0.322 | 0.317 | 0.491 | 0.352 | 0.331 |
| A_52_P367294 | NM_176966 | 319636 | Ccdc10 | 2.167 | 2.857 | 1.874 | 1.884 | 2.063 | 1.699 |
| A_52_P3698 | NM_001015099 | 217558 | | 1.885 | 1.84 | 1.762 | 2.392 | 2.296 | 2.486 |
| A 52 P370162 | NM_175271 | 78134 | Gpr23 | 0.303 | 0.145 | 0.219 | 0.384 | 0.365 | 0.464 |
| A_52_P376169 | NM_177139 | 320343 | E130115E03Rik | 0.314 | 0.285 | 0.295 | 0.539 | 0.508 | 0.528 |
| A_52_P376502 | BC025011; | 227541 | Camk1d | 1.575 | 1.641 | 1.563 | 1.989 | 2.543 | 1.959 |
| A_52_P377299 | AK048360; | 271377 | Zbtb11 | 1.927 | 1.892 | 1.923 | 1.678 | 1.79 | 1.546 |
| A_52_P380263 | NM_013723 | 27205 | Podxl | 0.395 | 0.422 | 0.286 | 0.532 | 0.511 | 0.504 |
| A_52_P382565 | XM_483943 | 432493 | LOC432493 | 0.976 | 1.222 | 1.221 | 1.492 | 1.439 | 1.198 |
| A_52_P384392 | AK080827; | 328087 | D930001 B02 | 2.365 | 2.375 | 2.393 | 1.135 | 1.269 | 1.098 |
| A_52_P384718 | BC052056;XM_283264 | 320873 | | 0.508 | 0.441 | 0.458 | 0.541 | 0.512 | 0.578 |
| A_52_P386544 | NM_026594 | 68172 | 4930517K11Rik | 2.843 | 2.618 | 2.802 | 2.673 | 2.913 | 2.894 |
| A_52_P386682 | AK033609; | 69587 | Rnf3 | 0.518 | 0.47 | 0.415 | 0.761 | 0.727 | 0.798 |
| A_52_P389874 | NM_011918 | 24131 | Ldb3 | 2.421 | 2.244 | 2.421 | 2.216 | 2.284 | 2.066 |
| A_52_P391505 | NM_144513 | 17263 | Gtl2 | 1.36 | 1.291 | 1.327 | 1.053 | 1.04 | 0.852 |
| A_52_P399095 | NM_177793 | 327747 | 9030224M15Rik | 0.366 | 0.325 | 0.163 | 0.433 | 0.401 | 0.466 |
| A_52_P400425 | NM_178656 | 193003 | A530088H08Rik | 2.498 | 2.036 | 2.006 | 2.674 | 2.834 | 2.576 |
| A_52_P402786 | NM_008935 | 19126 | Prom1 | 0.37 | 0.396 | 0.335 | 0.551 | 0.506 | 0.594 |
| A_52_P405340 | U76762; | 14158 | Fert2 | 2.538 | 2.399 | 2.414 | 2.011 | 2.124 | 1.824 |
| A_52_P410609 | NM_133685 | 106572 | 1700093E07Rik | 0.339 | 0.207 | 0.31 | 0.346 | 0.326 | 0.413 |
| A_52_P412167 | NM_080467 | 140494 | Atp6v0a4 | 2.324 | 2.449 | 2.406 | 4.276 | 4.719 | 3.334 |
| A_52_P413348 | XM_127674 | 75698 | 3110001 K24Rik | 2.072 | 2.063 | 2.27 | 1.908 | 2.161 | 1.733 |
| A_52_P413661 | BC079861;XM_484162 | 217716 | Mlh3 | 2.28 | 2.065 | 2.239 | 2.466 | 2.362 | 2.456 |
| A_52_P416123 | AK020480; | 77358 | 9430069107Rik | 2.511 | 2.833 | 2.298 | 1.785 | 1.993 | 1.656 |
| A_52_P420369 | NM_029444 | 75801 | 4930447C04Rik | 3.256 | 2.433 | 2.971 | 2.042 | 2.189 | 2.206 |
| A_52_P420608 | NM_201366 | 381371 | Gm1631 | 2.287 | 3.006 | 2.818 | 2.552 | 2.723 | 3.242 |
| A_52_P421234 | NM_133832 | 98711 | Rdh10 | 0.356 | 0.309 | 0.338 | 0.406 | 0.4 | 0.427 |
| A_52_P423384 | BC057551;XM_354546 | 104248 | Cabin1 | 1.58 | 1.963 | 1.524 | 1.442 | 1.596 | 1.426 |
| A_52_P425734 | NM_145146 | 280662 | Afm | 3.913 | 3.546 | 3.74 | 3.764 | 4.135 | 3.335 |
| A_52_P430927 | AK042385; | 11908 | Atf1 | 1.603 | 1.64 | 1.71 | 1.368 | 1.604 | 1.26 |
| A_52_P432464 | AK053863; | 192196 | Luc712 | 2.643 | 3.226 | 2.225 | 1.973 | 2.004 | 1.643 |
| A_52_P434549 | NM_023190 | 56215 | Acin1 | 1.868 | 1.876 | 1.953 | 1.241 | 1.454 | 1.123 |
| A_52_P438036 | NM_175271 | 78134 | Gpr23 | 0.376 | 0.334 | 0.36 | 0.455 | 0.441 | 0.489 |
| A_52_P442567 | NM_009099 | 20128 | Trim30 | 0.444 | 0.22 | 0.332 | 0.345 | 0.439 | 0.265 |
| A_52_P445799 | AK046777; | 52490 | D18Ertd289e | 0.676 | 0.602 | 0.389 | 0.613 | 0.532 | 0.592 |
| A_52_P452689 | NM_007498 | 11910 | Atf3 | 0.291 | 0.326 | 0.293 | 0.307 | 0.321 | 0.307 |
| A_52_P458916 | AK016751; | 218850 | D14Abb1e | 2.421 | 2.013 | 2.081 | 2.688 | 2.359 | 2.992 |
| A_52_P460882 | XM_130319;XM_130308 | 73668; 14725 | Ttc21b | 0.412 | 0.637 | 0.533 | 0.396 | 0.633 | 0.502 |
| A_52_P469789 | NM_007588 | 12311 | Calcr | 0.48 | 0.383 | 0.445 | 0.625 | 0.707 | 0.696 |
| A_52_P470663 | | | | 0.377 | 0.334 | 0.403 | 0.467 | 0.439 | 0.566 |
| A_52_P474242 | BE457768; | | | 0.499 | 0.53 | 0.46 | 0.69 | 0.658 | 0.726 |
| A_52_P483885 | AK078465; | 76916 | 4930455C21Rik | 2.455 | 2.834 | 1.947 | 2.313 | 2.262 | 1.788 |
| A_52_P485939 | NM_026228 | 67547 | Slc39a8 | 0.407 | 0.418 | 0.26 | 0.31 | 0.338 | 0.278 |
| A_52_P486234 | AK033497; | 22680 | | 3.81 | 2.878 | 2.669 | 3.19 | 3.59 | 3.068 |
| A_52_P487362 | NM_028980 | 74521 | 8430415E04Rik | 0.317 | 0.316 | 0.334 | 0.477 | 0.454 | 0.469 |
| A_52_P490263 | NM_013819 | 14991 | H2-M3 | 0.59 | 0.414 | 0.28 | 0.748 | 0.649 | 0.724 |
| A_52_P492189 | | | | 0.591 | 0.43 | 0.274 | 0.52 | 0.592 | 0.557 |
| A_52_P500979 | NM_013628 | 18548 | Pcsk1 | 0.75 | 0.686 | 0.598 | 1.08 | 1.031 | 0.991 |
| A_52_P501767 | AK012272; | 20637 | Snrp70 | 1.314 | 1.333 | 1.34 | 0.962 | 1 | 0.898 |
| A_52_P50496 | AY048587;XM_619453 | 114640 | Tifp39 | 0.454 | 0.501 | 0.422 | 0.661 | 0.638 | 0.694 |
| A_52_P505907 | NM_146112 | 227331 | Tnrc15 | 1.163 | 2.733 | 2.089 | 1.999 | 2.239 | 1.448 |
| A_52_P518352 | AK046930; | | | 1.712 | 1.676 | 1.27 | 1.313 | 1.441 | 1.085 |
| A_52_P519870 | AB010340; | | | 2.391 | 3.34 | 2.621 | 3.76 | 3.72 | 1.955 |
| A_52_P523399 | NM_029444 | 75801 | 4930447C04Rik | 3.312 | 7.019 | 4.341 | 3.597 | 4.009 | 2.819 |
| A_52_P532323 | NM_178410 | 218518 | Marveld2 | 0.399 | 0.326 | 0.264 | 0.434 | 0.363 | 0.431 |
| A_52_P532910 | NM_024427 | 22003 | Tpm1 | 0.358 | 0.392 | 0.35 | 0.386 | 0.39 | 0.433 |
| A_52_P537140 | AK047235; | | | 1.74 | 2.015 | 2.044 | 1.613 | 1.708 | 1.261 |
| A_52_P538515 | BC049181; | 226412 | R3hdm | 2.219 | 2.049 | 2.456 | 1.981 | 2.364 | 1.119 |
| A_52_P549815 | XM_621314 | 109620 | | 0.27 | 0.112 | 0.135 | 0.367 | 0.406 | 0.421 |
| A_52_P553100 | AK040413; | | | 2.967 | 3.025 | 3.491 | 3.19 | 3.168 | 2.79 |
| A_52_P553386 | AK041018; | | | 2.234 | 2.327 | 1.991 | 2.369 | 2.909 | 2.525 |
| A_52_P555603 | AK032770; | 11787 | Apbb2 | 1.409 | 1.222 | 1.29 | 1.326 | 1.427 | 1.18 |
| A_52_P556014 | AK087367;XM_620168 | 545738 | A930002C04Rik. | 1.947 | 2.241 | 1.96 | 1.785 | 1.768 | 1.418 |
| A_52_P557293 | NM_015775 | 50528 | Tmprss2 | 0.277 | 0.205 | 0.179 | 0.45 | 0.457 | 0.355 |
| A_52_P557940 | AK004302; | 66704 | 4921506122Rik | 2.114 | 1.863 | 2.221 | 2.17 | 2.013 | 2.051 |
| A_52_P560006 | NM_008426 | 16519 | Kcnj3 | 0.763 | 0.536 | 0.497 | 0.479 | 0.449 | 0.493 |
| A_52_P561046 | AK044509; | | | 1.501 | 1.345 | 1.04 | 1.708 | 2.08 | 1.486 |
| A_52_P565940 | AF419220; | 18193 | Nsd1 | 1.84 | 1.873 | 1.944 | 1.445 | 1.546 | 1.33 |
| A_52_P566681 | NM_153581 | 234267 | Gpm6a | 0.396 | 0.243 | 0.333 | 0.326 | 0.468 | 0.481 |
| A_52_P569375 | NM_010203 | 14176 | Fgf5 | 0.776 | 0.678 | 0.554 | 0.45 | 0.467 | 0.574 |
| A_52_P57078 | AK052455; | | | 2.172 | 1.912 | 1.757 | 1.727 | 1.952 | 1.877 |
| A_52_P571746 | XM_355533;XM_357399 | 230863 | LOC381571 | 5.455 | 5.043 | 4.955 | 4.478 | 4.627 | 3.701 |
| A_52_P571987 | AK053601; | 15525 | | 1.669 | 2.992 | 2.149 | 2.356 | 2.814 | 1.19 |
| A_52_P574668 | NM_011851 | 23959 | Nt5e | 0.15 | 0.159 | 0.148 | 0.316 | 0.285 | 0.335 |
| A_52_P575505 | NM_008994 | 19302 | Pxmp3 | 0.305 | 0.306 | 0.317 | 0.463 | 0.445 | 0.525 |
| A_52_P577072 | NM_011901 | 24074 | Taf7 | 2.198 | 2.692 | 2.144 | 1.999 | 2.035 | 1.851 |
| A_52_P581100 | NM_027285 | 70005 | 1700029I01Rik | 2.295 | 3.266 | 2.708 | 3.168 | 3.146 | 2.879 |
| A_52_P581138 | NM_212450 | 329506 | D2Ertd485e | 1.847 | 1.821 | 1.932 | 1.418 | 1.573 | 1.284 |
| A_52_P584874 | NM_152895 | 75605 | Jarid1b | 2.228 | 2.064 | 2.215 | 1.844 | 1.912 | 1.769 |
| A_52_P586157 | NM_022309 | 12400 | Cbfb | 0.481 | 0.354 | 0.384 | 0.606 | 0.523 | 0.75 |
| A_52_P59318 | NM_133832 | 98711 | Rdh10 | 0.306 | 0.273 | 0.275 | 0.373 | 0.395 | 0.445 |
| A_52_P601333 | AK078961; | 67786 | 6230424C14Rik | 0.466 | 0.125 | 0.157 | 0.36 | 0.335 | 0.333 |
| A_52_P620345 | NM_133979 | 102566 | Al604832 | 0.368 | 0.402 | 0.382 | 0.441 | 0.438 | 0.493 |
| A_52_P626069 | AK040994;XM_284439 | 109151 | Chd9 | 1.61 | 3.967 | 1.955 | 2.209 | 2.26 | 1.715 |
| A_52_P628212 | AK044339; | 26380 | | 9.353 | 5.604 | 7.112 | 6.273 | 6.769 | 7.237 |
| A_52_P630964 | NM_027533 | 70747 | Tspan2 | 0.361 | 0.283 | 0.324 | 0.499 | 0.473 | 0.525 |
| A_52_P634829 | NM_023835 | 76681 | Trim12 | 0.59 | 0.561 | 0.672 | 1.012 | 0.996 | 0.866 |
| A_52_P636199 | NM_008017 | 14211 | Smc2I1 | 1.61 | 2.123 | 1.631 | 1.531 | 1.607 | 1.38 |
| A_52_P636559 | AK053008; | 268469 | 9530033F24Rik | 2.63 | 2.537 | 2.752 | 2.132 | 2.334 | 1.998 |
| A_52_P650215 | NM_029278 | 75416 | 2610033H07Rik | 1.439 | 4.127 | 1.948 | 2.359 | 2.484 | 1.577 |
| A_52_P651469 | NM_001012726 | 503692 | | 5.11 | 4.303 | 5.085 | 5.606 | 6.363 | 4.012 |
| A_52_P654624 | AK005333; | 236604 | 4933439C20Rik | 2.049 | 1.763 | 2.06 | 1.639 | 1.865 | 1.518 |
| A_52_P655008 | AK044382; | 320678 | A930037G23Rik | 1.653 | 1.779 | 1.572 | 1.52 | 1.586 | 1.325 |
| A_52_P656336 | NM_023733 | 74114 | Crot | 0.407 | 0.351 | 0.381 | 0.755 | 0.647 | 0.8 |
| A_52_P661144 | XM_485365 | | | 2.066 | 2.37 | 2.399 | 2.3 | 2.365 | 2.13 |
| A_52_P6621 | | | | 2.585 | 4.954 | 2.769 | 4.277 | 4.565 | 2.653 |
| A_52_P672761 | NM_013689 | 21682 | Tec | 0.215 | 0.109 | NO DATA | 0.476 | 0.33 | 0.379 |
| A_52_P676403 | NM_019494 | 56066 | Cxcl11 | 0.525 | 0.596 | 0.597 | 0.761 | 0.809 | 0.704 |
| A_52_P676510 | NM_011579 | 21822 | Tgtp | 0.225 | 0.162 | 0.155 | 0.382 | 0.378 | 0.362 |
| A_52_P680436 | NM_025793 | 66840 | Wdr45I | 0.344 | 0.369 | 0.331 | 0.651 | 0.557 | 0.675 |
| A_52_P680941 | NM_010284 | 14600 | Ghr | 0.389 | 0.396 | 0.371 | 0.448 | 0.445 | 0.488 |
| A_52_P68702 | NM_145148 | 232288 | Frmd4b | 0.545 | 0.476 | 0.538 | 0.501 | 0.43 | 0.424 |
| A_52_P68942 | NM_026952 | 69131 | Crk7 | 1.741 | 2.133 | 1.422 | 1.895 | 2.138 | 1.574 |
| A_52_P731549 | AK085965; | 67939 | 2010316F05Rik | 1.602 | 1.397 | 1.252 | 1.252 | 1.393 | 1.078 |
| A_52 P74441 | NM_011570 | 21753 | Tes | 0.423 | 0.418 | 0.328 | 0.54 | 0.558 | 0.659 |
| A_52_P75971 | NM_026599 | 68178 | Cgnl1 | 1.56 | 1.535 | 1.702 | 1.265 | 1.357 | 1.222 |
| A_52_P78168 | AK031518; | | | 2.108 | 1.677 | 1.617 | 1.783 | 1.791 | 1.711 |
| A_52_P795628 | AK086352; | | | 4.861 | 5.344 | 4.508 | 6.792 | 7.236 | 5.913 |
| A_52_P804224 | | | | 1.522 | 1.688 | 1.828 | 2.31 | 2.487 | 2.241 |
| A_52_P812362 | BC016205; | 408064 | BC064078 | 0.25 | 0.167 | 0.217 | 0.302 | 0.27 | 0.27 |
| A_52_P81252 | NM_172599 | 218978 | D14Ertd436e | 2.478 | 2.667 | 2.607 | 1.82 | 1.981 | 1.764 |
| A_52_P819356 | AK041684; | | | 3.003 | 2.848 | 2.7 | 3.026 | 2.915 | 3.006 |
| A_52_P82488 | NM_021476 | 58861 | Cysltr1 | 0.319 | 0.379 | 0.306 | 0.5 | 0.438 | 0.534 |
| A_52_P826666 | AK007019; | 51799 | Rap2ip | 4.063 | 3.142 | 3.547 | 3.365 | 3.764 | 3.267 |
| A_52_P82722 | NM_009988 | 13052 | Cxadr | 0.311 | 0.136 | 0.21 | 0.361 | 0.254 | 0.355 |
| A_52_P827911 | AK081941; | | | 2.387 | 2.657 | 2.47 | 2.075 | 2.44 | 2.116 |
| A_52_P84015 | AK037762; | 11863 | | 3.675 | 3.344 | 3.705 | 4.008 | 3.981 | 3.736 |
| A_52_P85805 | NM_009525 | 22419 | Wnt5b | 0.548 | 0.377 | 0.209 | 0.495 | 0.38 | 0.469 |
| A_52_P87839 | NM_013657 | 20348 | Sema3c | 0.32 | 0.145 | 0.267 | 0.494 | 0.379 | 0.47 |
| A_52_P93837 | NM_008604 | 17380 | Mme | 0.559 | 0.439 | 0.513 | 0.653 | 0.573 | 0.747 |
| A_52_P947879 | AK048751; | | | 1.687 | 1.729 | 1.647 | 2.149 | 2.232 | 2.009 |
| A_52_P957500 | XM_622354 | | | 2.438 | 1.963 | 2.267 | 2.142 | 2.231 | 1.855 |
| A_52_P995717 | AK045222; | | | 3.292 | 3.495 | 3.303 | 2.04 | 2.108 | 1.729 |

**Table 1B - Training Set Genes (Non Gemline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P101975 | NM_010205 | 14179 | Fgf8 | 2.683 | 2.96 | 2.139 | 3.25 | 3.463 | 3.854 |
| A_51_P102081 | NM_007847 | 13222 | Defcr-rs2 | 2.408 | 2.238 | 1.498 | 3.38 | 3.255 | 2.763 |
| A_51_P102257 | AK053112;XM_619640 | 320477 | E030037J05Rik | 0.455 | 0.507 | 0.624 | 1.76 | 1.58 | 1.703 |
| A_51_P103397 | AK078521; | 54561 | Nap1I3 | 0.942 | 0.996 | 1.004 | 1.047 | 0.978 | 1.076 |
| A_51_P103406 | NM_011708 | 22371 | Vwf | 0.798 | 1.058 | 1.093 | 1.007 | 0.932 | 1.019 |
| A_51_P104162 | AK053379; | 328232 | | 0.351 | 0.468 | 0.481 | 1.289 | 1.159 | 1.082 |
| A_51_P105068 | NM_027990 | 71897 | 2310010M24Rik | 1.902 | 1.982 | 1.72 | 2.012 | 2.165 | 2.002 |
| A_51_P106918 | NM_009051 | 19715 | Rex2 | 1.032 | 1.019 | 0.654 | 1.31 | 1.285 | 1.541 |
| A_51_P109649 | AK018142; | 17263 | Gtl2 | 0.11 | 0.17 | 0.136 | 0.167 | 0.167 | 0.195 |
| A_51_P113162 | AK086843; | | | 0.616 | 0.947 | 0.722 | 1.731 | 1.215 | 1.78 |
| A_51_P114606 | NM_016965 | 50884 | Nckap1 | 1.512 | 1.408 | 1.677 | 0.736 | 0.694 | 0.805 |
| A_51_P115005 | NM_010104 | 13614 | Edn1 | 0.521 | 0.469 | 0.61 | 0.968 | 0.997 | 1.054 |
| A_51_P121635 | NM_030700 | 80884 | Maged2 | 1.225 | 1.426 | 1.335 | 0.918 | 0.99 | 0.88 |
| A_51_P124315 | NM_177649 | 227712 | BC034076 | 0.654 | 0.611 | 0.726 | 1.267 | 1.174 | 1.278 |
| A_51_P125679 | AK010725;XM_134948 | 73673 | 2410076121Rik | 0.588 | 0.959 | 0.916 | 0.84 | 0.843 | 0.849 |
| A_51_P127560 | AK051724; | 76577 | | 0.24 | 0.497 | 0.587 | 1.321 | 1.349 | 1.505 |
| A_51_P130095 | NM_010187 | 14130 | Fcgr2b | 0.4 | 0.349 | 0.423 | 0.624 | 0.547 | 0.453 |
| A_51_P140751 | NM_011397 | 20522 | Slc23a1 | 0.522 | 0.618 | 0.556 | 1.152 | 1.255 | 1.186 |
| A_51_P141660 | AK053665;XM_358382 | 67776; 235380 | Loh11cr2a | 0.43 | 0.62 | 0.908 | 1.844 | 1.422 | 1.976 |
| A_51_P144417 | NM_181750 | 226412 | R3hdm | 0.745 | 0.757 | 0.789 | 2.029 | 1.993 | 2.191 |
| A_51_P144712 | NM_178930 | 107338 | Gbf1 | 0.424 | 0.476 | 0.425 | 1.192 | 1.19 | 1.292 |
| A_51_P147651 | AK041797;XM_146705 | 245902 | | 0.511 | 0.729 | 0.963 | 1.47 | 1.214 | 1.502 |
| A_51_P149469 | NM_009608 | 11464 | Actc1 | 1.012 | 1.006 | 0.935 | 0.971 | 0.997 | 1.12 |
| A_51_P153079 | NM_133923 | 101100 | 4833441J24Rik | 0.42 | 0.232 | 0.278 | 1.153 | 1.158 | 1.047 |
| A_51_P157986 | NM_029930 | 77574 | 3321401G04Rik | 0.909 | 1.196 | 1.088 | 0.563 | 0.621 | 0.598 |
| A_51_P158037 | NM_013505 | 13506 | Dsc2 | 1.238 | 1.562 | 1.304 | 0.743 | 0.819 | 0.875 |
| A_51_P160160 | AK017624; | 70548 | 5730437C12Rik | 1.025 | 1.558 | 1.042 | 0.701 | 0.738 | 0.923 |
| A_51_P161115 | AK050756; | | | NO DATA | 0.189 | 0.363 | 1.733 | 0.972 | 1.474 |
| A_51_P162474 | AF285577; | 107815 | Scml2 | 2.885 | 2.549 | 2.371 | 3.021 | 3.116 | 3.1 |
| A_51_P162481 | NM_133194 | 107815 | Scml2 | 2.434 | 2.616 | 2.232 | 2.312 | 2.394 | 2.596 |
| A_51_P164219 | NM_011909 | 24110 | Usp18 | 1.876 | 1.698 | 1.64 | 1.445 | 1.463 | 1.349 |
| A_51_P164504 | NM_007469 | 11812 | Apoc1 | 1.307 | 1.245 | 1.314 | 1.412 | 1.375 | 1.457 |
| A_51_P165082 | AB041601;XM_489523 | 224019 | D16Bwg1494e | 1.656 | 1.924 | 1.93 | 1.376 | 1.394 | 1.295 |
| A_51_P165244 | NM_018734 | 55932 | Gbp4 | 2.388 | 1.319 | 2.543 | 2.404 | 2.051 | 2.534 |
| A_51_P168646 | AK039580; | | | 1.655 | 1.373 | 1.436 | 2.357 | 2.478 | 2.267 |
| A_51_P171728 | NM_011926 | 26365 | Ceacam1 | 0.474 | 0.703 | 0.686 | 0.748 | 0.952 | 0.943 |
| A_51_P174943 | NM_008485 | 16782 | Lamc2 | 1.282 | 1.255 | 1.249 | 1.068 | 1.04 | 1.004 |
| A_51_P176474 | AK010466; | 76484 | 2410012C07Rik | 0.842 | 0.903 | 1.004 | 0.996 | 1.036 | 1.127 |
| A_51_P180413 | NM_176930 | 319504 | C130076007Rik | 1.603 | 1.54 | 1.395 | 1.297 | 1.25 | 1.155 |
| A_51_P187645 | BC004786;XM_354700 | 16061 | Igh-VJ558 | 2.489 | 2.63 | 2.11 | 2.267 | 1.973 | 2.108 |
| A_51_P189631 | NM_172427 | 76455 | 2310067E19Rik | 0.318 | 0.341 | 0.373 | 1.015 | 0.905 | 0.892 |
| A_51_P191990 | AK087548; | 26563 | Ror1 | 0.378 | 0.368 | 0.389 | 1.197 | 1.149 | 1.176 |
| A_51_P193019 | NM_011157 | 19073 | Prg1 | 0.263 | 0.419 | 0.589 | 1.602 | 0.893 | 0.295 |
| A_51_P199888 | NM_026082 | 67299 | Dock7 | 1.182 | 1.246 | 1.295 | 0.669 | 0.678 | 1 |
| A_51_P200223 | XM_619497 | | | 0.65 | 0.646 | 0.76 | 1.392 | 1.367 | 1.332 |
| A_51_P202911 | NM_144882 | 67198 | 2810022L02Rik | 1.005 | 0.995 | 0.908 | 0.913 | 0.931 | 0.902 |
| A_51_P203955 | NM_010260 | 14469 | Gbp2 | 2.194 | 2.061 | 2.214 | 2.45 | 2.681 | 2.345 |
| A_51_P207934 | NM_199016 | 224794 | Enpp4 | 0.71 | 0.576 | 0.811 | 1.068 | 0.833 | 1.143 |
| A_51_P219166 | NM_027756 | 71306 | Mfap3l | 1.187 | 1.279 | 1.37 | 1.013 | 0.915 | 1.095 |
| A_51_P221291 | NM_009229 | 20650 | Sntb2 | 0.614 | 0.612 | 0.631 | 1.259 | 1.1 | 1.157 |
| A_51_P222773 | NM_010446 | 15376 | Foxa2 | 2.333 | 1.693 | 1.64 | 6.867 | 6.289 | 6.137 |
| A_51_P223132 | NM_031183;XM_486227 | 83395; 70549 | Sp6 | 0.649 | 0.542 | 0.552 | 1.095 | 0.998 | 1.125 |
| A_51_P223656 | NM_008484 | 16780 | Lamb3 | 0.994 | 1.434 | 1.253 | 1.02 | 1.017 | 1.132 |
| A_51_P228472 | NM_008343 | 16009 | lgfbp3 | 1.472 | 2.26 | 1.637 | 1.02 | 1.11 | 1.239 |
| A_51_P229613 | NM_008792 | 18549 | Pcsk2 | 1.463 | 1.53 | 1.37 | 1.333 | 1.422 | 1.554 |
| A_51_P231341 | AK087764; | 108160 | D0HXS9928E | 0.131 | 0.284 | 0.334 | 1.584 | 1 | 1.654 |
| A_51_P236267 | NM_009183 | 20452 | St8sia4 | 1.305 | 1.382 | 1.289 | 1.168 | 1.234 | 1.322 |
| A_51_P237040 | NM_008711 | 18121 | Nog | 1.152 | 1.407 | 1.101 | 0.859 | 0.96 | 0.924 |
| A_51_P240253 | NM_019662 | 56437 | Rrad | 1.392 | 1.648 | 1.29 | 0.851 | 0.9 | 0.913 |
| A_51_P240811 | NM_009290 | 20890 | Wnt8a | 4.437 | 4.432 | 3.479 | 4.373 | 5.655 | 4.468 |
| A_51_P242399 | NM_031170 | 16691 | Krt2-8 | 1.013 | 1.199 | 0.939 | 0.962 | 0.977 | 0.946 |
| A_51_P252565 | NM_009660 | 11687 | Alox15 | 1.618 | 1.425 | 1.194 | 1.366 | 1.436 | 1.457 |
| A_51_P253053 | XM_618867 | | | 0.601 | 1.748 | 0.724 | 1.594 | 1.644 | 2.037 |
| A_51_P262171 | NM_008326 | 15944 | Ifi1 | 1.271 | 1.011 | 1.135 | 1.457 | 1.571 | 1.485 |
| A_51_P266403 | AK009163; | 76730 | 2310005C01Rik | 0.984 | 0.901 | 1.009 | 1.273 | 1.313 | 1.391 |
| A_51_P266644 | BC055753;XM_125538 | 140742 | | 1.51 | 1.548 | 1.621 | 1.064 | 0.96 | 1.069 |
| A_51_P268167 | BC037232;XM_621365 | 229363; 218850 | Gmps | 0.465 | 0.448 | 0.523 | 1.267 | 1.053 | 1.205 |
| A_51_P270725 | AK089493; | 102093 | Phkb | 0.756 | 0.923 | 0.755 | 2.239 | 1.725 | 2.287 |
| A_51_P272363 | NM_134160 | 171166 | Mcoln3 | 2.423 | 3.074 | 2.25 | 1.242 | 1.432 | 0.843 |
| A_51_P275435 | NM_172411 | 71874 | 2310007B03Rik | 3.355 | 3.105 | 3.53 | 5.636 | 6.155 | 6.288 |
| A_51_P276305 | NM_009292 | 20899 | Stra8 | 0.873 | 0.934 | 0.982 | 0.431 | 0.426 | 0.471 |
| A_51_P282227 | NM_020577 | 57344 | As3mt | 0.79 | 0.761 | 0.777 | 1.02 | 0.91 | 0.98 |
| A_51_P283300 | NM_023116 | 12296 | Cacnb2 | 1.159 | 1.029 | 1.146 | 0.648 | 0.72 | 0.636 |
| A_51_P285725 | NM_009309 | 20997 | T | 2.672 | 1.6 | 1.512 | 3.129 | 3.214 | 3.267 |
| A_51_P289341 | AK030946;XM_141372 | 241639 | 5830467J12Rik | 0.978 | 1.482 | 1.063 | 0.881 | 0.972 | 1.046 |
| A_51_P291227 | NM_001005508 | 226652 | 6030405P05Rik | 0.705 | 0.681 | 0.731 | 0.814 | 0.735 | 0.798 |
| A_51_P294505 | NM_025658 | 66607 | Ms4a4d | 1.546 | 1.551 | 1.883 | 2.127 | 2.066 | 2.183 |
| A_51_P294566 | AK020687; | 78083 | 9930116N10Rik | 0.689 | 1.237 | 0.848 | 1.59 | 1.475 | 1.545 |
| A_51_P297131 | NM_009502 | 22330 | Vcl | 1.015 | 1.436 | 0.955 | 0.556 | 0.672 | 0.587 |
| A_51_P298266 | NM_025681 | 66643 | Lix1 | 1.123 | 1.145 | 1.109 | 1.143 | 1.15 | 1.141 |
| A_51_P302377 | NM_008009 | 14181 | Fgfbp1 | 1.542 | 1.782 | 1.567 | 1.002 | 1.052 | 1.132 |
| A_51_P305019 | AK020601; | 78423 | 9530049005Rik | 0.201 | 0.4 | 0.429 | 1.187 | 1.059 | 1.249 |
| A_51_P311038 | BC009666;XM_134720 | 57267 | Apba3 | 1.847 | 1.642 | 1.538 | 2.55 | 2.405 | 2.808 |
| A_51_P316733 | AK173008; | 330914 | Grit | 0.195 | 0.471 | 0.456 | 1.1 | 1.238 | 1.129 |
| A_51_P318577 | AK010729; | 73671 | 2410078J06Rik | 0.605 | 0.863 | 0.641 | 0.897 | 1.028 | 1.15 |
| A_51_P321579 | XM_131865;XM_196334 | 433809 | LOC433809 | 0.495 | 0.534 | 0.598 | 1.155 | 1.087 | 1.237 |
| A_51_P327011 | NM_016744 | 18573 | Pde1a | 1.629 | 1.061 | 1.167 | 1.387 | 1.253 | 1.051 |
| A_51_P330380 | XM_130138;XM_145466 | 243881 | 4921517N04Rik | 0.714 | 1.211 | 0.583 | 1.659 | 2.149 | 0.895 |
| A_51_P333253 | NM_178440 | 246177 | Myo1g | 0.535 | 0.491 | 0.538 | 0.474 | 0.481 | 0.37 |
| A_51_P334093 | NM_026228 | 67547 | Slc39a8 | 2.173 | 2.638 | 2.466 | 1.07 | 1.006 | 1.061 |
| A_51_P335460 | NM_009132 | 20259 | Scin | 1.658 | 1.913 | 1.519 | 1.101 | 1.225 | 1.107 |
| A_51_P337125 | NM_010566 | 16331 | Inpp5d | 0.938 | 0.873 | 1.036 | 1.261 | 1.272 | 1.297 |
| A_51_P341137 | AK035176; | 224829 | Trerf1 | 0.563 | 0.731 | 0.649 | 1.51 | 1.436 | 1.382 |
| A_51_P343129 | AK012079; | 70472 | Atad2 | 0.633 | 0.896 | 0.848 | 1.965 | 1.637 | 1.677 |
| A_51_P345422 | NM_010747 | 17096 | Lyn | 0.888 | 0.803 | 0.881 | 1.006 | 0.995 | 0.89 |
| A_51_P352572 | NM_019701 | 56365 | Clcnkb | 1.441 | 1.565 | 1.422 | 1.707 | 1.362 | 1.314 |
| A_51_P353221 | NM_011582 | 21828 | Thbs4 | 1.037 | 0.982 | 1.145 | 0.826 | 0.851 | 0.782 |
| A_51_P354265 | NM_009256 | 20723 | Serpinb9 | 1.028 | 1.006 | 1.064 | 0.948 | 0.995 | 0.916 |
| A_51_P354307 | NM_172469 | 209195 | Clic6 | 1.31 | 1.578 | 1.57 | 0.719 | 0.748 | 0.659 |
| A_51_P356003 | NM_001007580 | 333564 | Gm784 | 1.223 | 1.221 | 1.186 | 1.543 | 1.618 | 1.409 |
| A_51_P356883 | NM_008728 | 18162 | Npr3 | 0.813 | 0.973 | 1.027 | 0.692 | 0.599 | 0.672 |
| A_51_P358344 | NM_007732 | 12821 | Col17a1 | 0.283 | 0.661 | 0.876 | 1.118 | 0.984 | 1.246 |
| A_51_P358714 | AK031433; | | | 0.23 | 0.294 | 0.418 | 1.005 | 0.775 | 1.214 |
| A_51_P378051 | NM_009675 | 11754 | Aoc3 | 1.41 | 1.106 | 1.668 | 0.9 | 0.88 | 1.25 |
| A_51_P378371 | | | | 0.986 | 1.006 | 1.208 | 1.217 | 1.156 | 1.234 |
| A_51_P381069 | XM_132045 | 231252 | Chrna9 | 0.803 | 0.789 | 0.615 | 1.017 | 1.052 | 0.983 |
| A_51_P387528 | AK019664; | 78801 | Ak7 | 0.65 | 0.537 | 0.813 | 0.767 | 0.778 | 1.11 |
| A_51_P393518 | BC016539; | 240793 | | 1.037 | 1.122 | 1.147 | 0.727 | 0.85 | 0.8 |
| A_51_P397003 | XM_133990 | 68904 | 1110065L07Rik | 1.235 | 1.343 | 1.238 | 0.778 | 0.763 | 0.855 |
| A_51_P398766 | NM_010259 | 14468 | Gbp1 | 3.558 | 2.476 | 3.155 | 4.099 | 3.953 | 3.971 |
| A_51_P404193 | NM_022435 | 64406 | Sp5 | 1.798 | 1.335 | 1.388 | 3.365 | 3.261 | 3.306 |
| A_51_P405375 | NM_175358 | 108672 | Zdhhc15 | 1.724 | 1.355 | 1.63 | 0.826 | 1.108 | 1.317 |
| A_51_P407323 | NM_007976 | 14067 | F5 | 0.837 | 0.684 | 0.686 | 2.154 | 2.023 | 1.925 |
| A_51_P410813 | AK079732; | | | 0.717 | 0.877 | 0.904 | 1.593 | 1.473 | 1.607 |
| A_51_P412817 | NM_177617 | 216851 | D330014H01 Rik | 0.725 | 0.834 | 1.04 | 1.083 | 0.986 | 0.997 |
| A_51_P418029 | NM_175541 | 245631 | Mum1I1 | 2.026 | 1.803 | 2.159 | 1.492 | 1.654 | 1.714 |
| A_51_P418375 | NM_023844 | 67374 | Jam2 | 1.044 | 0.943 | 0.99 | 0.826 | 0.786 | 0.84 |
| A_51_P418884 | NM_001013789 | 432823 | | 0.496 | 0.536 | 0.695 | 1.716 | 1.402 | 1.593 |
| A_51_P423859 | NM_007601 | 12335 | Capn3 | 0.976 | 0.768 | 0.966 | 0.825 | 0.789 | 1.053 |
| A_51_P437050 | BC072664; | 233328 | Lrrk1 | 0.941 | 1.002 | 0.944 | 1.031 | 1.066 | 0.969 |
| A_51_P442894 | AK048310; | 621603 | C130048D07Rik | 1.023 | 0.841 | 1.218 | 0.788 | 0.867 | 0.977 |
| A_51_P447189 | AK041326;XM_358090 | 385154 | | 0.755 | 0.808 | 0.675 | 2.409 | 2.277 | 2.233 |
| A_51_P455647 | NM_009801 | 12349 | Car2 | 1.592 | 1.463 | 1.337 | 2.139 | 2.347 | 2.17 |
| A_51_P455997 | Y13832; | 17263 | Gtl2 | 0.123 | 0.184 | 0.114 | 0.213 | 0.197 | 0.258 |
| A_51_P457187 | NM_028075 | 72049 | Tnfrsf13c | 1.083 | 1.58 | 1.373 | 0.916 | 1.099 | 1.091 |
| A_51_P463789 | BC051947; | 58212 | | 0.924 | 0.962 | 0.995 | 1.518 | 1.581 | 1.46 |
| A_51_P463791 | BC051947; | 58212 | | 0.648 | 0.879 | 1.013 | 1.447 | 1.472 | 1.498 |
| A_51_P463846 | NM_145545 | 229900 | 9830147J24Rik | 2.28 | 2.199 | 2.49 | 2.068 | 1.958 | 1.72 |
| A_51_P467505 | NM_013689 | 21682 | Tec | 1.077 | 1.062 | 1.153 | 0.842 | 0.861 | 0.881 |
| A_51_P473953 | XM_358600;XM_355454 | 381498 | 4930592C13Rik | 2.138 | 1.593 | 1.947 | 2.323 | 2.226 | 2.26 |
| A_51_P478930 | AK080016; | | | 0.64 | 0.623 | 0.816 | 1.097 | 1.039 | 1.17 |
| A_51_P48031 | NM_010168 | 14061 | F2 | 0.348 | 0.476 | 0.622 | 0.996 | 1.004 | 1.186 |
| A_51_P481777 | NM_018756 | 54382 | Tcstv1 | 0.208 | 0.696 | 0.654 | 0.592 | 0.503 | 0.72 |
| A_51_P481958 | NM_016674 | 12737 | Cldn1 | 2.017 | 2.213 | 2.426 | 0.904 | 0.646 | 0.974 |
| A_51_P485985 | NM_134163 | 171170 | Mbnl3 | 1.781 | 1.802 | 1.648 | 0.929 | 1.067 | 1.035 |
| A_51_P487004 | NM_027732 | 71241 | Dmrtc2 | 1.918 | 2.991 | 1.94 | 1.77 | 1.537 | 1.901 |
| A_51_P493682 | XM_134870;XM_140758 | 72672 | Rasl12 | 0.352 | 0.801 | 0.945 | 1.611 | 1.596 | 1.849 |
| A_51_P497100 | NM_010706 | 16855 | Lgals4 | 0.692 | 0.771 | 0.849 | 1.002 | 0.908 | 0.828 |
| A_51_P499854 | NM_010284 | 14600 | Ghr | 1.55 | 1.19 | 1.486 | 2.502 | 2.537 | 2.196 |
| A_51_P507880 | NM_028980 | 74521 | 8430415E04Rik | 2.104 | 2.169 | 2.081 | 2.44 | 2.499 | 2.391 |
| A_51_P512085 | NM_130449 | 140792 | Colec12 | 1.574 | 1.25 | 1.471 | 1.463 | 1.651 | 1.693 |
| A_51_P512172 | AK010953;XM_125673 | 52463 | Cxxc6 | 0.8 | 0.986 | 0.748 | 1.033 | 1.014 | 1.046 |
| A_51_P514177 | NM_028838 | 74249 | Lrrc2 | 0.911 | 0.956 | 0.975 | 1.199 | 1.209 | 1.304 |
| A_51_P520175 | AK076272; | 12223 | Btc | 0.978 | 0.996 | 1.252 | 1.035 | 0.896 | 0.744 |
| A_51_P521084 | NM_025626 | 66540 | 3110001A13Rik | 1.423 | 1.425 | 1.474 | 0.832 | 0.865 | 0.776 |
| A_52_P100042 | AK045143; | 320938 | Tnpo3 | 0.301 | 0.507 | 0.406 | 1.444 | 1.417 | 1.439 |
| A_52_P1011737 | AK039096; | 106258 | Al790442 | 0.224 | 0.44 | 0.438 | 1.367 | 1.308 | 1.503 |
| A_52_P1013441 | | | | 1.255 | 1.032 | 2.041 | 1.173 | 1.203 | 1.341 |
| A_52_P103929 | NM_009306 | 20979 | Syt1 | 0.898 | 0.938 | 0.967 | 1.626 | 1.464 | 1.422 |
| A_52_P1044262 | AK051949; | | | 0.374 | 0.535 | 0.443 | 1.042 | 1.044 | 1.091 |
| A_52_P1044696 | AK087281; | | | 0.323 | 0.722 | 0.629 | 1.236 | 1.397 | 1.547 |
| A_52_P1075773 | AK036921; | 399602 | 9930024M15Rik | 0.274 | 0.379 | 0.59 | 1.459 | 1.225 | 1.486 |
| A_52_P1084149 | AK051413; | 553138 | 9930021J03Rik | 0.447 | 0.508 | 0.452 | 1.499 | 1.58 | 1.485 |
| A_52_P1108139 | AK053079; | | | 0.169 | 0.348 | 0.199 | 1.068 | 1.372 | 1.161 |
| A_52_P1124598 | AK081090; | | | 0.304 | 0.598 | 0.609 | 1.045 | 0.474 | 1.511 |
| A_52_P113190 | NM_053214 | 17916 | Myo1f | 0.386 | 0.513 | 0.397 | 0.243 | 0.232 | 0.264 |
| A_52_P1172548 | AK084146; | 13204 | Dhx15 | 0.655 | 0.839 | 0.889 | 2.469 | 2.249 | 2.786 |
| A_52_P1180315 | AK049088; | 74022 | 3930401K13Rik | 0.255 | 0.505 | 0.516 | 1.337 | 1.179 | 0.966 |
| A_52_P1188270 | AK042362; | | | 0.341 | 0.594 | 0.564 | 1.758 | 1.462 | 1.658 |
| A_52_P1196751 | AK086488; | | | 0.314 | 0.63 | 0.521 | 1.285 | 1.098 | 1.263 |
| A_52_P126158 | NM_008326 | 15944 | Ifi1 | 1.157 | 1.034 | 1.006 | 1.254 | 1.3 | 1.232 |
| A_52_P129428 | BC049774; | 57265 | Fzd2 | 1.029 | 0.862 | 1.274 | 1.343 | 1.356 | 1.851 |
| A_52_P132608 | NM_172685 | 229731 | Slc25a24 | 0.809 | 0.906 | 0.807 | 0.708 | 0.722 | 0.693 |
| A_52_P135873 | XM_132015;XM_488584 | 231128 | BC037112 | 0.389 | 0.408 | 0.392 | 1.158 | 1.118 | 1.166 |
| A_52_P13815 | NM_010686 | 16792 | Laptm5 | 0.187 | 0.227 | 0.332 | 0.483 | 0.494 | 0.599 |
| A_52_P147870 | NM_029963 | 77721 | Mrps5 | 0.574 | 0.622 | 0.64 | 1.321 | 1.377 | 1.297 |
| A_52_P148222 | BC055037; | 224139 | 4930428L02Rik | 1.242 | 1.264 | 1.274 | 1.061 | 0.874 | 0.976 |
| A_52_P152983 | AK011739; | 26886 | Cenph | 0.423 | 0.805 | 0.28 | 1.393 | 1.582 | 1.833 |
| A_52_P153685 | AK129323; | 70422 | 2810417D08Rik | 1.763 | 1.676 | 1.845 | 2.204 | 2.099 | 2.085 |
| A_52_P159095 | AK051960; | 17685 | | 0.49 | 0.948 | 0.865 | 2.32 | 2.123 | 2.226 |
| A_52_P163148 | AB010370; | 544945 | | 0.704 | 0.687 | 0.76 | 1.098 | 1.144 | 1.325 |
| A_52_P164524 | NM_013628 | 18548 | Pcsk1 | 2.458 | 2.533 | 2.589 | 2.384 | 2.201 | 2.401 |
| A_52_P165497 | NM_011385 | 20481 | Ski | 0.541 | 0.665 | 0.649 | 1.004 | 0.817 | 0.972 |
| A_52_P165610 | NM_153534 | 210044 | Adcy2 | 1.106 | 1.129 | 1.34 | 0.778 | 0.709 | 0.763 |
| A_52_P16973 | AK081934; | 320391 | D930017J03Rik | 0.56 | 0.604 | 0.612 | 1.894 | 1.918 | 1.856 |
| A_52_P175685 | NM_008390 | 16362 | Irf1 | 1.072 | 1.33 | 1.162 | 0.971 | 1.106 | 1.017 |
| A_52_P176333 | NM_001005787 | 12695 | Cipp | 1.168 | 1.146 | 1.249 | 0.974 | 1.014 | 1.036 |
| A_52_P179250 | AK129127; | 230249 | Al314180 | 0.641 | 0.606 | 0.56 | 3.467 | 4.289 | 6.052 |
| A_52_P183368 | NM_011535 | 21386 | Tbx3 | 0.707 | 0.655 | 0.649 | 0.948 | 0.84 | 0.841 |
| A_52_P18887 | NM_011468 | 20755 | Sprr2a | 7.008 | 6.235 | 5.038 | 6.207 | 6.689 | 6.331 |
| A_52_P196568 | NM_144513 | 17263 | Gtl2 | 0.0697 | 0.0369 | 0.0417 | 0.112 | 0.0801 | 0.115 |
| A_52_P197826 | NM_010397 | 15039 | H2-T22 | 1.502 | 1.687 | 1.444 | 1.028 | 1.049 | 1.039 |
| A_52_P199633 | NM_199146 | 209387 | LOC209387 | 0.911 | 1.276 | 1.628 | 1.284 | 1.253 | 2.216 |
| A_52_P199922 | | | | 0.316 | 0.331 | 0.255 | 1.479 | 1.195 | 1.071 |
| A_52_P200458 | NM_010242 | 14345 | Fut4 | 1.003 | 0.997 | 1.017 | 1.108 | 1.203 | 1.09 |
| A_52_P206573 | NM_027427 | 70439 | Taf15 | 0.525 | 0.7 | 0.806 | 1.685 | 1.521 | 1.76 |
| A_52_P211956 | NM_025658 | 66607 | Ms4a4d | 1.71 | 1.799 | 1.851 | 2.105 | 2.274 | 1.961 |
| A_52_P214042 | NM_028125 | 72147 | Btbd4 | 1.267 | 1.407 | 1.626 | 0.852 | 0.647 | 0.866 |
| A_52_P21595 | NM_028238 | 72433 | Rab38 | 1.043 | 0.791 | 0.992 | 0.807 | 0.819 | 0.929 |
| A_52_P21628 | NM_019978 | 13175 | Dcamkl1 | 1.407 | 1.646 | 1.591 | 1.067 | 1.203 | 1.097 |
| A_52_P223083 | BC038323; | 72096 | 2010208K18Rik | 0.89 | 0.64 | 1.018 | 2.024 | 1.641 | 2.309 |
| A_52_P224125 | XM_622260 | | | 0.593 | 0.637 | 0.657 | 1.232 | 0.927 | 0.967 |
| A_52_P233673 | NM_020594 | 57432 | Zc3hdc8 | 0.731 | 0.498 | 0.877 | 1.204 | 1.041 | 1.395 |
| A_52_P237357 | AF095690; | | | 0.454 | 0.422 | 0.442 | 1.34 | 1.372 | 1.159 |
| A_52_P238468 | XM_133238 | 381853 | Gm160 | 0.673 | 0.817 | 0.882 | 1.027 | 1.116 | 1.106 |
| A_52_P240706 | NM_007543 | 26367 | Ceacam2 | 0.87 | 0.778 | 0.892 | 0.689 | 0.756 | 0.859 |
| A_52_P253179 | AK077477; | 16009 | Igfbp3 | 2.157 | 1.536 | 2.035 | 1.993 | 1.965 | 2.545 |
| A_52_P255224 | AK032711;XM_486482 | 234371 | BC021367 | 0.812 | 0.712 | 0.548 | 2.288 | 1.957 | 2.287 |
| A_52_P255250 | NM_009543 | 22644 | Rnf103 | 1.205 | 1.217 | 1.524 | 0.663 | 0.701 | 0.687 |
| A_52_P258116 | NM_009521 | 22415 | Wnt3 | 1.304 | 1.249 | 1.083 | 1.811 | 1.943 | 2.006 |
| A_52_P26416 | AF106279; | 16782 | | 1.436 | 1.802 | 1.779 | 0.723 | 0.806 | 0.697 |
| A_52_P269158 | NM_001003948 | 98496 | AL024069 | 1.775 | 1.419 | 1.506 | 1.494 | 1.391 | 1.254 |
| A_52_P274960 | NM_001005508 | 226652 | 6030405P05Rik | 0.671 | 0.634 | 0.712 | 0.763 | 0.781 | 0.808 |
| A_52_P277415 | AK077313; | 76252 | 0610006014Rik | 0.0165 | 0.0277 | 0.0223 | 0.0497 | 0.0428 | 0.0552 |
| A_52_P282000 | AK020248; | 13800 | Enah | 0.368 | 0.482 | 0.376 | 1.791 | 1.63 | 1.536 |
| A_52_P284243 | XM_359390;XM_358896 | 384373 | LOC386546 | 1.151 | 1.169 | 1.447 | 0.803 | 0.82 | 0.911 |
| A_52_P284981 | NM_007462 | 11789 | Apc | 0.319 | 0.415 | 0.649 | 1.173 | 0.969 | 1.155 |
| A_52_P286166 | NM_001002927 | 18619 | Penk1 | 1.649 | 2.509 | 2.439 | 1.542 | 1.453 | 1.374 |
| A_52_P286528 | BC055771; | 102954 | Nudt11 | 1.419 | 1.344 | 1.431 | 1.048 | 1.063 | 1.053 |
| A_52_P286970 | XM_131162;XM_127565 | 76561 | Snx7 | 1.591 | 2.536 | 1.327 | 1.128 | 1.271 | 0.96 |
| A_52_P287576 | AK036308; | | | 0.284 | 0.537 | 0.429 | 1.515 | 1.516 | 1.834 |
| A_52_P289559 | NM_177784 | 277396 | C130068N17Rik | 0.89 | 1.215 | 1.34 | 0.528 | 0.7 | 0.835 |
| A_52_P293443 | AK087208; | 13819 | | 0.179 | 0.222 | 0.219 | 0.543 | 0.565 | 0.552 |
| A_52_P295933 | AK045060; | 14051 | B130023L16Rik | 1.637 | 1.973 | 2.08 | 1.007 | 0.993 | 1.019 |
| A_52_P296244 | BC048734; | | | 2.333 | 2.799 | 1.851 | 2.371 | 2.371 | 2.372 |
| A_52_P301821 | NM_053078 | 27528 | D0H4S114 | 1.076 | 1.275 | 1.138 | 0.88 | 0.828 | 0.741 |
| A_52_P30386 | AK084172; | | | 0.381 | 0.53 | 0.593 | 1.296 | 1.275 | 1.34 |
| A_52_P306387 | NM_178709 | 235315 | D130054N24Rik | 0.574 | 0.504 | 0.652 | 1.816 | 1.515 | 1.965 |
| A_52_P307801 | NM_007847 | 13222 | Defcr-rs2 | 2.149 | 1.602 | 1.28 | 2.942 | 3.1 | 2.825 |
| A_52_P309718 | AK173099; | 218850 | D14Abb1e | 1.006 | 0.813 | 1.127 | 1.186 | 1.29 | 1.289 |
| A_52_P317273 | NM_172846 | 240960 | A230079K17Rik | 1.135 | 1.343 | 0.878 | 1.131 | 1.103 | 1.019 |
| A_52_P320170 | NM_144787 | 76804 | Jmjd2c | 0.502 | 0.764 | 0.8 | 0.999 | 0.89 | 1.597 |
| A_52_P322927 | XM_487182 | 435271 | LOC435271 | 0.444 | 0.68 | 0.767 | 0.515 | 0.426 | 0.637 |
| A_52_P324934 | AK045391; | 16403 | | 0.758 | 0.573 | 0.774 | 1.808 | 1.513 | 1.534 |
| A_52_P32502 | AK020822; | 77944 | A930007B11Rik | 1.212 | 1.019 | 1.21 | 2.052 | 1.291 | 1.824 |
| A_52_P3304 | NM_080446 | 117599 | Helb | 1.004 | 1.239 | 1.059 | 1.614 | 1.432 | 1.418 |
| A_52_P335089 | BE198552; | | | 0.338 | 0.471 | 0.43 | 0.921 | 0.981 | 0.901 |
| A_52_P336259 | AK087367;XM_485576 | 319467 | A930002C04Rik | 0.552 | 0.507 | 0.623 | 1.053 | 0.913 | 0.8 |
| A_52_P344424 | NM_010720 | 16891 | Lipg | 1.688 | 1.328 | 2.214 | 1.439 | 1.09 | 1.069 |
| A_52_P345626 | AY149175; | 386626 | Mzf6d | 0.699 | 0.636 | 0.76 | 1.173 | 1.052 | 1.267 |
| A_52_P34823 | NM_010747 | 17096 | Lyn | 0.523 | 0.589 | 0.625 | 0.371 | 0.427 | 0.434 |
| A_52_P350477 | NM_139295 | 193813 | Mcfd2 | 1.118 | 1.377 | 1.329 | 0.667 | 0.758 | 0.786 |
| A_52_P354363 | NM_026574 | 68142 | | 0.545 | 0.546 | 0.579 | 1.246 | 1.113 | 1.072 |
| A_52_P354785 | AK037929; | 14852 | Gspt1 | 0.487 | 0.598 | 0.585 | 1.587 | 1.447 | 1.483 |
| A_52_P355276 | NM_001002764 | 103677 | Al317223 | 0.704 | 0.605 | 0.784 | 1.531 | 1.432 | 1.606 |
| A_52_P358951 | AK081999;XM_134736 | 271457; 22688 | Rab5a | 1.346 | 1.445 | 1.326 | 0.843 | 0.883 | 0.787 |
| A_52_P359010 | AK129478; | 72193 | Sfrs2ip | 0.54 | 0.706 | 0.574 | 1.484 | 1.398 | 1.403 |
| A_52_P361534 | NM_009521 | 22415 | Wnt3 | 1.193 | 0.891 | 0.969 | 2.247 | 2.459 | 2.231 |
| A_52_P362805 | AK044730; | 269254 | Als4 | 0.692 | 0.79 | 0.89 | 1.216 | 1.11 | 1.17 |
| A_52_P364021 | NM_026713 | 68393 | Mogat1 | 1.295 | 1.109 | 1.272 | 1.124 | 1.301 | 1.292 |
| A_52_P367294 | NM_176966 | 319636 | Ccdc10 | 0.64 | 0.752 | 1.224 | 1.255 | 1.037 | 1.385 |
| A_52_P3698 | NM_001015099 | 217558 | | 0.658 | 0.653 | 0.676 | 1.487 | 1.564 | 1.537 |
| A_52_P370162 | NM_175271 | 78134 | Gpr23 | 1.104 | 1.003 | 1.165 | 1.307 | 1.348 | 1.868 |
| A_52_P376169 | NM_177139 | 320343 | E130115E03Rik | 1.184 | 1.025 | 1.076 | 1.949 | 1.81 | 1.828 |
| A_52_P376502 | BC025011; | 227541 | Camk1d | 0.664 | 0.71 | 0.737 | 0.973 | 0.893 | 1.008 |
| A_52_P377299 | AK048360; | 271377 | Zbtb11 | 0.577 | 0.582 | 0.645 | 1.152 | 1.092 | 1.023 |
| A_52_P380263 | NM_013723 | 27205 | Podxl | 1.179 | 1.159 | 1.66 | 0.899 | 0.909 | 0.942 |
| A_52_P382565 | XM_483943 | 432493 | LOC432493 | 0.228 | 0.273 | 0.254 | 0.607 | 0.622 | 0.462 |
| A_52_P384392 | AK080827; | 328087 | D930001B02 | 0.533 | 0.516 | 0.471 | 1.258 | 1.062 | 1.099 |
| A_52_P384718 | BC052056;XM_283264 | 320873 | | 1.488 | 1.451 | 1.435 | 1.051 | 1.032 | 1.026 |
| A_52_P386544 | NM_026594 | 68172 | 4930517K11Rik | 0.74 | 0.606 | 0.729 | 1.188 | 1.159 | 1.351 |
| A_52_P386682 | AK033609; | 69587 | Rnf3 | 1.349 | 2.019 | 1.908 | 0.901 | 0.891 | 1.063 |
| A_52_P389874 | NM_011918 | 24131 | Ldb3 | 0.702 | 0.892 | 0.937 | 1.166 | 1.063 | 1.078 |
| A_52_P391505 | NM_144513 | 17263 | Gtl2 | 0.0793 | 0.114 | 0.0867 | 0.158 | 0.15 | 0.148 |
| A_52_P399095 | NM_177793 | 327747 | 9030224M15Rik | 1.11 | 0.976 | 1.056 | 1.583 | 1.692 | 1.596 |
| A_52_P400425 | NM_178656 | 193003 | A530088H08Rik | 0.241 | 0.469 | 0.547 | 0.79 | 0.835 | 1.024 |
| A_52_P402786 | NM_008935 | 19126 | Prom1 | 1.498 | 1.71 | 1.749 | 1.028 | 1.023 | 0.92 |
| A_52_P405340 | U76762; | 14158 | Fert2 | 0.434 | 0.372 | 0.598 | 1.654 | 1.494 | 1.493 |
| A_52_P410609 | NM_133685 | 106572 | 1700093E07Rik | 0.956 | 1.237 | 1.105 | 0.415 | 0.492 | 0.422 |
| A_52_P412167 | NM_080467 | 140494 | Atp6v0a4 | 0.553 | 1.045 | 0.878 | 1.777 | 2.017 | 2.418 |
| A_52_P413348 | XM_127674 | 75698 | 3110001 K24Rik | 0.593 | 0.709 | 0.685 | 1.434 | 1.352 | 1.356 |
| A_52_P413661 | BC079861;XM_484162 | 217716 | Mlh3 | 0.513 | 0.589 | 0.64 | 1.73 | 1.608 | 1.611 |
| A_52_P416123 | AK020480; | 77358 | 9430069l07Rik | 0.584 | 0.644 | 1.001 | 1.681 | 1.367 | 1.728 |
| A_52_P420369 | NM_029444 | 75801 | 4930447C04Rik | 1.087 | 1.084 | 1.129 | 1.176 | 0.989 | 1.041 |
| A_52_P420608 | NM_201366 | 381371 | Gm1631 | 1.252 | 0.926 | 0.759 | 1.014 | 1.175 | 1.159 |
| A_52_P421234 | NM_133832 | 98711 | Rdh10 | 1.706 | 1.519 | 1.676 | 0.845 | 0.856 | 0.776 |
| A_52_P423384 | BC057551;XM_354546 | 104248 | Cabin1 | 0.481 | 0.424 | 0.586 | 1.189 | 1.05 | 1.408 |
| A_52_P425734 | NM_145146 | 280662 | Afm | 1.466 | 1.63 | 2.105 | 1.902 | 1.789 | 2.093 |
| A_52_P430927 | AK042385; | 11908 | Atf1 | 0.409 | 0.436 | 0.428 | 1.263 | 1.186 | 1.26 |
| A_52_P432464 | AK053863; | 192196 | Luc7l2 | 0.7 | 0.863 | 1.032 | 1.457 | 1.269 | 1.582 |
| A_52_P434549 | NM_023190 | 56215 | Acin1 | 0.489 | 0.399 | 0.44 | 1.376 | 1.207 | 1.143 |
| A_52_P438036 | NM_175271 | 78134 | Gpr23 | 1.082 | 0.96 | 0.968 | 1.634 | 1.593 | 1.618 |
| A_52_P442567 | NM_009099 | 20128 | Trim30 | 1.331 | 1.1 | 1.212 | 1.814 | 1.462 | 1.507 |
| A_52_P445799 | AK046777; | 52490 | D18Ertd289e | 2.088 | 2.22 | 2.899 | 2.952 | 2.963 | 3.531 |
| A_52_P452689 | NM_007498 | 11910 | Atf3 | 0.672 | 0.61 | 0.659 | 0.89 | 0.955 | 0.943 |
| A_52_P458916 | AK016751; | 218850 | D14Abb1e | 0.488 | 0.694 | 0.807 | 1.15 | 1.185 | 1.257 |
| A_52_P460882 | XM_130319;XM_130308 | 73668; 14725 | Ttc21 b | 1.498 | 1.698 | 2.077 | 1.06 | 1.276 | 1.219 |
| A_52_P469789 | NM_007588 | 12311 | Calcr | 2.087 | 2.206 | 2.278 | 1.646 | 1.638 | 1.533 |
| A_52_P470663 | | | | 2.339 | 2.72 | 2.405 | 1.069 | 1.105 | 1.018 |
| A_52_P474242 | BE457768; | | | 1.31 | 1.785 | 1.55 | 1.137 | 1.132 | 0.997 |
| A_52_P483885 | AK078465; | 76916 | 4930455C21 Rik | 0.763 | 0.712 | 1.027 | 1.428 | 1.429 | 1.483 |
| A_52_P485939 | NM_026228 | 67547 | Sic39a8 | 3.203 | 2.606 | 3.687 | 2.136 | 1.903 | 2.146 |
| A_52_P486234 | AK033497; | 22680 | | 0.658 | 0.943 | 0.756 | 2.133 | 2.185 | 1.908 |
| A_52_P487362 | NM_028980 | 74521 | 8430415E04Rik | 3.136 | 2.67 | 3.124 | 4.295 | 4.001 | 3.546 |
| A_52_P490263 | NM_013819 | 14991 | H2-M3 | 1.272 | 1.518 | 1.745 | 0.961 | 1.184 | 1.166 |
| A_52_P492189 | | | | 2.909 | 3.045 | 3.596 | 2.009 | 2.077 | 2.009 |
| A_52_P500979 | NM_013628 | 18548 | Pcsk1 | 4.338 | 3.184 | 4.164 | 4.966 | 5.086 | 4.923 |
| A_52_P501767 | AK012272; | 20637 | Snrp70 | 0.263 | 0.243 | 0.263 | 1.047 | 1 | 0.926 |
| A_52_P50496 | AY048587;XM_619453 | 114640 | Tifp39 | 1.196 | 1.551 | 1.504 | 1.114 | 1.171 | 1.214 |
| A_52_P505907 | NM_146112 | 227331 | Tnrc15 | 0.219 | 0.415 | 0.633 | 1.334 | 1.307 | 1.752 |
| A_52_P518352 | AK046930; | | | 0.266 | 0.346 | 0.376 | 1.223 | 1.034 | 1.313 |
| A_52_P519870 | AB010340; | | | 0.758 | 0.675 | 1.128 | 0.989 | 0.863 | 1.393 |
| A_52_P523399 | NM_029444 | 75801 | 4930447C04Rik | 1.167 | 0.873 | 1.364 | 1.373 | 1.087 | 1.771 |
| A_52_P532323 | NM_178410 | 218518 | Marveld2 | 1.824 | 2.102 | 1.904 | 0.871 | 0.884 | 1.027 |
| A_52_P532910 | NM_024427 | 22003 | Tpm1 | 1.081 | 1.106 | 1.238 | 0.891 | 0.87 | 0.851 |
| A_52_P537140 | AK047235; | | | 0.566 | 0.565 | 0.531 | 1.029 | 1.127 | 1.156 |
| A_52_P538515 | BC049181; | 226412 | R3hdm | 0.209 | 0.659 | 0.565 | 1.913 | 1.725 | 1 |
| A_52_P549815 | XM_621314 | 109620 | | 0.982 | 1.23 | 0.97 | 0.813 | 0.899 | 0.906 |
| A_52_P553100 | AK040413; | | | 0.696 | 0.886 | 0.727 | 2.086 | 2.022 | 1.86 |
| A_52_P553386 | AK041018; | | | 0.235 | 0.571 | 0.685 | 1.632 | 1.95 | 1.925 |
| A_52_P555603 | AK032770; | 11787 | Apbb2 | 0.273 | 0.355 | 0.304 | 1.021 | 1.021 | 1.054 |
| A_52_P556014 | AK087367;XM_620168 | 545738 | A930002C04Rik | NO DATA | 0.365 | 0.504 | 0.806 | 0.777 | 0.943 |
| A_52_P557293 | NM_015775 | 50528 | Tmprss2 | 1.142 | 1.091 | 1.111 | 1.208 | 1.127 | 1.225 |
| A_52_P557940 | AK004302; | 66704 | 4921506l22Rik | 0.544 | 0.595 | 0.569 | 1.371 | 1.379 | 1.241 |
| A_52_P560006 | NM_008426 | 16519 | Kcnj3 | 2.321 | 2.818 | 2.608 | 2.191 | 1.875 | 2.19 |
| A_52_P561046 | AK044509; | | | 0.146 | 0.211 | 0.311 | 0.792 | 0.777 | 1.011 |
| A_52_P565940 | AF419220; | 18193 | Nsd1 | 0.487 | 0.593 | 0.616 | 1.236 | 1.181 | 1.08 |
| A_52_P566681 | NM_153581 | 234267 | Gpm6a | 1.117 | 1.337 | 1.114 | 1.045 | 1.073 | 0.961 |
| A_52_P569375 | NM_010203 | 14176 | Fgf5 | 4.338 | 3.206 | 3.958 | 5.889 | 5.927 | 6.75 |
| A_52_P57078 | AK052455; | | | 0.552 | 0.439 | 0.686 | 1.676 | 1.452 | 1.294 |
| A_52_P571746 | XM_355533;XM_357399 | 230863 | LOC381571 | 1.076 | 1.21 | 1.362 | 2.199 | 2.23 | 2.231 |
| A_52_P571987 | AK053601; | 15525 | | 0.285 | 0.758 | 0.643 | 1.776 | 0.839 | 1.405 |
| A_52_P574668 | NM_011851 | 23959 | Nt5e | 1.611 | 1.127 | 1.284 | 1.764 | 1.707 | 1.826 |
| A_52_P575505 | NM_008994 | 19302 | Pxmp3 | 1.123 | 1.07 | 1.092 | 0.797 | 0.815 | 0.802 |
| A_52_P577072 | NM_011901 | 24074 | Taf7 | 0.654 | 0.591 | 0.759 | 1.655 | 1.407 | 1.87 |
| A_52_P581100 | NM_027285 | 70005 | 1700029I01Rik | 1.164 | 1.1 | 1.141 | 1.525 | 1.383 | 1.305 |
| A_52_P581138 | NM_212450 | 329506 | D2Ertd485e | 0.457 | 0.451 | 0.472 | 1.47 | 1.244 | 1.239 |
| A_52_P584874 | NM_152895 | 75605 | Jarid1b | 0.737 | 0.83 | 0.752 | 1.242 | 1.251 | 1.242 |
| A_52_P586157 | NM_022309 | 12400 | Cbfb | 1.417 | 1.551 | 1.338 | 0.773 | 0.863 | 0.903 |
| A_52_P59318 | NM_133832 | 98711 | Rdh10 | 1.689 | 1.611 | 1.584 | 0.876 | 1.002 | 0.955 |
| A_52_P601333 | AK078961; | 67786 | 6230424C14Rik | 0.336 | 0.445 | 0.476 | 1.075 | 1.112 | 0.946 |
| A_52_P620345 | NM_133979 | 102566 | AI604832 | 1.26 | 1.488 | 1.375 | 0.778 | 0.752 | 0.729 |
| A_52_P626069 | AK040994;XM_284439 | 109151 | Chd9 | 0.449 | 0.512 | 0.687 | 1.302 | 1.102 | 1.65 |
| A_52_P628212 | AK044339; | 26380 | | 1.058 | 1.337 | 0.967 | 4.002 | 3.276 | 3.435 |
| A_52_P630964 | NM_027533 | 70747 | Tspan2 | 1.217 | 1.314 | 1.26 | 0.815 | 0.863 | 0.897 |
| A_52_P634829 | NM_023835 | 76681 | Trim12 | 1.52 | 1.676 | 1.775 | 1.472 | 1.69 | 1.577 |
| A_52_P636199 | NM_008017 | 14211 | Smc2I1 | 0.482 | 0.452 | 0.534 | 1.172 | 1.125 | 1.415 |
| A_52_P636559 | AK053008; | 268469 | 9530033F24Rik | 0.506 | 0.53 | 0.567 | 1.485 | 1.451 | 1.363 |
| A_52_P650215 | NM_029278 | 75416 | 2610033H07Rik | 0.869 | 0.561 | 1.153 | 1.294 | 1.106 | 1.919 |
| A_52_P651469 | NM_001012726 | 503692 | | 1.087 | 2.041 | 1.585 | 2.114 | 1.954 | 2.198 |
| A_52_P654624 | AK005333; | 236604 | 4933439C20Rik | 0.543 | 0.696 | 0.523 | 1.316 | 1.231 | 1.139 |
| A_52_P655008 | AK044382; | 320678 | A930037G23Rik | 0.364 | 0.363 | 0.425 | 1.339 | 1.302 | 1.272 |
| A_52_P656336 | NM_023733 | 74114 | Crot | 1.233 | 1.547 | 1.442 | 0.983 | 1.067 | 0.998 |
| A_52_P661144 | XM_485365 | | | 0.511 | 0.546 | 0.638 | 1.627 | 1.676 | 2.009 |
| A_52_P6621 | | | | 0.661 | 0.506 | 1.607 | 1.455 | 1.079 | 2.626 |
| A_52_P672761 | NM_013689 | 21682 | Tec | 0.969 | 1.307 | 1.208 | 0.717 | 0.737 | 0.713 |
| A_52_P676403 | NM_019494 | 56066 | Cxcl11 | 3.86 | 3.177 | 3.663 | 2.834 | 2.723 | 2.826 |
| A_52 P676510 | NM_011579 | 21822 | Tgtp | 1.166 | 1.03 | 1.064 | 1.15 | 1.119 | 1.082 |
| A_52_P680436 | NM_025793 | 66840 | Wdr45l | 1.338 | 1.213 | 1.346 | 0.755 | 0.702 | 0.805 |
| A_52_P680941 | NM_010284 | 14600 | Ghr | 1.72 | 1.563 | 1.682 | 2.058 | 1.978 | 2.065 |
| A_52_P68702 | NM_145148 | 232288 | Frmd4b | 0.928 | 1.071 | 0.877 | 1.242 | 1.234 | 1.139 |
| A_52_P68942 | NM_026952 | 69131 | Crk7 | 0.535 | 0.706 | 0.746 | 1.388 | 1 | 1.146 |
| A_52_P731549 | AK085965; | 67939 | 2010316F05Rik | 0.236 | 0.333 | 0.323 | 1.081 | 1 | 1.122 |
| A_52_P74441 | NM_011570 | 21753 | Tes | 1.498 | 1.167 | 1.425 | 0.853 | 0.882 | 0.885 |
| A_52_P75971 | NM_026599 | 68178 | Cgnl1 | 0.432 | 0.439 | 0.529 | 0.801 | 0.761 | 0.771 |
| A_52_P78168 | AK031518; | | | 0.277 | 0.385 | 0.299 | 0.704 | 0.652 | 0.544 |
| A_52_P795628 | AK086352; | | | 0.551 | 1.049 | 0.818 | 3.259 | 4.511 | 4.2 |
| A_52_P804224 | | | | 0.299 | 0.314 | 0.291 | 1.263 | 1.2 | 1.068 |
| A_52_P812362 | BC016205; | 408064 | BC064078 | 1.424 | 1.508 | 1.444 | 1.323 | 1.468 | 1.262 |
| A_52_P81252 | NM_172599 | 218978 | D14Ertd436e | 0.265 | 0.408 | 0.488 | 1.153 | 1.046 | 0.927 |
| A_52_P819356 | AK041684; | | | 0.651 | 0.704 | 0.763 | 1.654 | 1.657 | 1.821 |
| A_52_P82488 | NM_021476 | 58861 | Cysltr1 | 1.919 | 1.474 | 1.811 | 2.106 | 2.164 | 2.621 |
| A_52_P826666 | AK007019; | 51799 | Rap2ip | 0.894 | 1.131 | 1.14 | 2.913 | 2.479 | 3.106 |
| A_52_P82722 | NM_009988 | 13052 | Cxadr | 0.584 | 0.807 | 0.998 | 0.725 | 0.811 | 0.798 |
| A_52_P827911 | AK081941; | | | 0.209 | 0.476 | 0.487 | 1.73 | 1.566 | 1.778 |
| A_52_P84015 | AK037762; | 11863 | | 0.695 | 0.924 | 0.842 | 2.965 | 3.344 | 3.023 |
| A_52_P85805 | NM_009525 | 22419 | Wnt5b | 1.366 | 1.006 | 1.074 | 0.994 | 0.884 | 1.009 |
| A_52_P87839 | NM_013657 | 20348 | Sema3c | 1.257 | 0.996 | 1.513 | 0.958 | 1.004 | 0.817 |
| A_52_P93837 | NM_008604 | 17380 | Mme | 1.516 | 1.805 | 1.761 | 1.442 | 1.369 | 1.329 |
| A_52_P947879 | AK048751; | | | 0.354 | 0.496 | 0.449 | 1.501 | 1.634 | 1.919 |
| A_52_P957500 | XM_622354 | | | 0.371 | 0.532 | 0.397 | 1.528 | 1.372 | 1.305 |
| A_52_P995717 | AK045222; | | | 0.672 | 0.74 | 0.709 | 2.392 | 1.854 | 2.412 |

**Table 2A - 292 Gene Set (Germline Comeptent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 0.266 | 0.299 | 0.281 | 0.396 | 0.368 | 0.407 |
| A_51_P335460 | NM_009132 | 20259 | Scin | 0.718 | 0.631 | 0.719 | 0.678 | 0.674 | 0.666 |
| A_52_P391505 | NM_144513 | 17263 | Gtl2 | 1.36 | 1.291 | 1.327 | 1.053 | 1.04 | 0.852 |
| A_51_P497100 | NM_010706 | 16855 | Lgals4 | 2.115 | 2.049 | 2.368 | 1.716 | 1.861 | 1.527 |
| A_51_P378371 | | | | 0.454 | 0.542 | 0.39 | 0.468 | 0.484 | 0.468 |
| A_51_P298266 | NM_025681 | 66643 | Lix1 | 0.507 | 0.414 | 0.416 | 0.554 | 0.584 | 0.54 |
| A_52_P269158 | NM_001003948 | 98496 | AL024069 | 0.316 | 0.33 | 0.33 | 0.388 | 0.393 | 0.443 |
| A_51_P311038 | BC009666;XM_134720 | 57267 | Apba3 | 0.334 | 0.341 | 0.339 | 0.446 | 0.472 | 0.482 |
| A_51_P457187 | NM_028075 | 72049 | Tnfrsf13c | 2.82 | 3.275 | 3.026 | 2.654 | 3.432 | 2.546 |
| A_52_P68702 | NM_145148 | 232288 | Frmd4b | 0.545 | 0.476 | 0.538 | 0.501 | 0.43 | 0.424 |
| A_52_P293443 | AK087208; | 13819 | | 1.278 | 1.075 | 1.13 | 1.496 | 1.604 | 1.349 |
| A_51_P157986 | NM_029930 | 77574 | 3321401G04Rik | 0.426 | 0.339 | 0.345 | 0.368 | 0.318 | 0.37 |
| A_52_P1013441 | | | | 3.248 | 3.164 | 3.191 | 4.558 | 5.61 | 4.683 |
| A_51_P418375 | NM_023844 | 67374 | Jam2 | 1.691 | 1.467 | 1.625 | 2.142 | 2.132 | 2.288 |
| A_52_P557940 | AK004302; | 66704 | 4921506I22Rik | 2.114 | 1.863 | 2.221 | 2.17 | 2.013 | 2.051 |
| A_51_P480311 | NM_010168 | 14061 | F2 | 1.991 | 1.809 | 1.969 | 2.465 | 2.4 | 1.874 |
| A_52_P200458 | NM_010242 | 14345 | Fut4 | 0.429 | 0.343 | 0.355 | 0.453 | 0.391 | 0.512 |
| A_51_P463846 | NM_145545 | 229900 | 9830147J24Rik | 0.694 | 0.519 | 0.59 | 0.86 | 0.816 | 0.817 |
| A_51_P162474 | AF285577; | 107815 | Scml2 | 0.539 | 0.413 | 0.405 | 0.643 | 0.645 | 0.71 |
| A_51_P165244 | NM_018734 | 55932 | Gbp4 | 0.585 | 0.512 | 0.474 | 1.008 | 0.981 | 1.006 |
| A_52_P59318 | NM_133832 | 98711 | Rdh10 | 0.306 | 0.273 | 0.275 | 0.373 | 0.395 | 0.445 |
| A_52_P438036 | NM_175271 | 78134 | Gpr23 | 0.376 | 0.334 | 0.36 | 0.455 | 0.441 | 0.489 |
| A_52_P474242 | BE457768; | | | 0.499 | 0.53 | 0.46 | 0.69 | 0.658 | 0.726 |
| A_51_P499854 | NM_010284 | 14600 | Ghr | 0.407 | 0.375 | 0.412 | 0.413 | 0.429 | 0.489 |
| A_51_P455647 | NM_009801 | 12349 | Car2 | 0.335 | 0.288 | 0.301 | 0.568 | 0.516 | 0.638 |
| A_52_P103929 | NM_009306 | 20979 | Syt1 | 0.382 | 0.356 | 0.39 | 0.419 | 0.498 | 0.423 |
| A_51_P149469 | NM_009608 | 11464 | Actc1 | 0.324 | 0.387 | 0.384 | 0.496 | 0.551 | 0.471 |
| A_52_P557293 | NM_015775 | 50528 | Tmprss2 | 0.277 | 0.205 | 0.179 | 0.45 | 0.457 | 0.355 |
| A_52_P50496 | AY048587;XM_619453 | 114640 | Tifp39 | 0.454 | 0.501 | 0.422 | 0.661 | 0.638 | 0.694 |
| A_51_P180413 | NM_176930 | 319504 | C130076O07Rik | 0.359 | 0.393 | 0.415 | 0.471 | 0.514 | 0.494 |
| A_51_P229613 | NM_008792 | 18549 | Pcsk2 | 0.512 | 0.483 | 0.515 | 0.58 | 0.58 | 0.504 |
| A_51_P121635 | NM_030700 | 80884 | Maged2 | 0.378 | 0.371 | 0.409 | 0.492 | 0.475 | 0.481 |
| A_51_P481777 | NM_018756 | 54382 | Tcstv1 | 1.532 | 1.822 | 1.792 | 2.619 | 3.155 | 2.956 |
| A_51_P412817 | NM_177617 | 216851 | D330014H01Rik | 3.151 | 3.039 | 3.326 | 2.136 | 2.194 | 1.865 |
| A_51_P387528 | AK019664; | 78801 | Ak7 | 2.693 | 2.814 | 2.57 | 3.786 | 3.834 | 3.71 |
| A_52_P78168 | AK031518; | | | 2.108 | 1.677 | 1.617 | 1.783 | 1.791 | 1.711 |
| A_52_P274960 | NM_001005508 | 226652 | 6030405P05Rik | 1.83 | 1.82 | 1.948 | 2.07 | 2.181 | 2.076 |
| A_52_P1188270 | AK042362; | | | 3.076 | 1.828 | 1.917 | 2.932 | 3.046 | 2.87 |
| A_51_P291227 | NM_001005508 | 226652 | 6030405P05Rik | 1.876 | 1.826 | 1.86 | 2.054 | 2.185 | 2.004 |
| A_52_P377299 | AK048360; | 271377 | Zbtb11 | 1.927 | 1.892 | 1.923 | 1.678 | 1.79 | 1.546 |
| A_51_P297131 | NM_009502 | 22330 | Vcl | 0.402 | 0.372 | 0.378 | 0.411 | 0.377 | 0.401 |
| A_52_P804224 | | | | 1.522 | 1.688 | 1.828 | 2.31 | 2.487 | 2.241 |
| A_51_P442894 | AK048310; | 621603 | C130048D07Rik | 2.632 | 2.364 | 2.281 | 3.657 | 3.714 | 3.102 |
| A_52_P309718 | AK173099; | 218850 | D14Abb1e | 2.324 | 2.579 | 2.18 | 2.614 | 2.72 | 2.711 |
| A_52_P359010 | AK129478; | 72193 | Sfrs2ip | 2.152 | 2.149 | 2.122 | 2.349 | 2.38 | 2.318 |
| A_51_P410813 | AK079732; | | | 2.732 | 2.716 | 2.559 | 2.898 | 2.605 | 2.3 |
| A_52_P382565 | XM_483943 | 432493 | LOC432493 | 0.976 | 1.222 | 1.221 | 1.492 | 1.439 | 1.198 |
| A_51_P333253 | NM_178440 | 246177 | Myo1g | 1.419 | 1.413 | 1.621 | 1.544 | 1.504 | 1.394 |
| A_52_P113190 | NM_053214 | 17916 | Myo1f | 1.428 | 1.513 | 1.396 | 1.988 | 1.959 | 2.083 |
| A_51_P473953 | XM_358600;XM_355454 | 381498 | 4930592C13Rik | 0.42 | 0.306 | 0.298 | 0.365 | 0.365 | 0.38 |
| A_52_P519870 | AB010340; | | | 2.391 | 3.34 | 2.621 | 3.76 | 3.72 | 1.955 |
| A_51_P266403 | AK009163; | 76730 | 2310005C01Rik | 3.079 | 3.053 | 3.092 | 2.733 | 2.967 | 2.734 |
| A_52_P3698 | NM_001015099 | 217558 | | 1.885 | 1.84 | 1.762 | 2.392 | 2.296 | 2.486 |
| A_52_P581100 | NM_027285 | 70005 | 1700029I01Rik | 2.295 | 3.266 | 2.708 | 3.168 | 3.146 | 2.879 |
| A_51_P276305 | NM_009292 | 20899 | Stra8 | 3.137 | 3.069 | 3.174 | 2.824 | 2.972 | 2.777 |
| A_51_P455997 | Y13832; | 17263 | Gtl2 | 1.505 | 1.455 | 1.44 | 0.998 | 1.082 | 0.998 |
| A_52_P656336 | NM_023733 | 74114 | Crot | 0.407 | 0.351 | 0.381 | 0.755 | 0.647 | 0.8 |
| A_51_P512172 | AK010953;XM_125673 | 52463 | Cxxc6 | 2.864 | 2.384 | 2.216 | 2.184 | 2.11 | 2.068 |
| A_51_P282227 | NM_020577 | 57344 | As3mt | 2.651 | 2.607 | 2.876 | 3.224 | 3.327 | 3.147 |
| A_52_P386544 | NM_026594 | 68172 | 4930517K11Rik | 2.843 | 2.618 | 2.802 | 2.673 | 2.913 | 2.894 |
| A_51_P125679 | AK010725;XM_134948 | 73673 | 2410076I1Rik | 2.867 | 2.332 | 2.464 | 3.518 | 3.661 | 3.727 |
| A_51_P164504 | NM_007469 | 11812 | Apoc1 | 3.245 | 2.908 | 3.052 | 2.863 | 3.059 | 2.591 |
| A_52_P240706 | NM_007543 | 26367 | Ceacam2 | 2.285 | 1.866 | 1.892 | 2.654 | 2.556 | 2.628 |
| A_52_P420369 | NM_029444 | 75801 | 4930447C04Rik | 3.256 | 2.433 | 2.971 | 2.042 | 2.189 | 2.206 |
| A_51_P242399 | NM_031170 | 16691 | Krt2-8 | 0.193 | 0.233 | 0.208 | 0.403 | 0.4 | 0.39 |
| A_51_P158037 | NM_013505 | 13506 | Dsc2 | 0.271 | 0.249 | 0.258 | 0.437 | 0.415 | 0.453 |
| A_52_P258116 | NM_009521 | 22415 | Wnt3 | 0.156 | 0.164 | 0.16 | 0.312 | 0.309 | 0.296 |
| A_52_P286166 | NM_001002927 | 18619 | Penk1 | 0.497 | 0.324 | 0.339 | 0.671 | 0.658 | 0.647 |
| A_52_P399095 | NM_177793 | 327747 | 9030224M15Rik | 0.366 | 0.325 | 0.163 | 0.433 | 0.401 | 0.466 |
| A_52_P470663 | | | | 0.377 | 0.334 | 0.403 | 0.467 | 0.439 | 0.566 |
| A_52_P376169 | NM_177139 | 320343 | E130115E03Rik | 0.314 | 0.285 | 0.295 | 0.539 | 0.508 | 0.528 |
| A_51_P512085 | NM_130449 | 140792 | Colec12 | 0.335 | 0.364 | 0.296 | 0.382 | 0.368 | 0.385 |
| A_52_P676403 | NM_019494 | 56066 | Cxcl11 | 0.525 | 0.596 | 0.597 | 0.761 | 0.809 | 0.704 |
| A_51_P354307 | NM_172469 | 209195 | Clic6 | 0.43 | 0.319 | 0.329 | 0.398 | 0.398 | 0.387 |
| A_52_P569375 | NM_010203 | 14176 | Fgf5 | 0.776 | 0.678 | 0.554 | 0.45 | 0.467 | 0.574 |
| A_51_P162481 | NM_133194 | 107815 | Scml2 | 0.432 | 0.357 | 0.385 | 0.594 | 0.55 | 0.618 |
| A_51_P202911 | NM_144882 | 67198 | 2810022L02Rik | 0.297 | 0.283 | 0.328 | 0.346 | 0.333 | 0.365 |
| A_51_P418029 | NM_175541 | 245631 | Mum1l1 | 0.319 | 0.265 | 0.278 | 0.419 | 0.397 | 0.534 |
| A_52_P93837 | NM_008604 | 17380 | Mme | 0.559 | 0.439 | 0.513 | 0.653 | 0.573 | 0.747 |
| A_51_P275435 | NM_172411 | 71874 | 2310007B03Rik | 0.368 | 0.492 | 0.206 | 0.875 | 0.83 | 0.857 |
| A_51_P199888 | NM_026082 | 67299 | Dock7 | 0.407 | 0.403 | 0.245 | 0.558 | 0.428 | 0.546 |
| A_52_P211956 | NM_025658 | 66607 | Ms4a4d | 0.61 | 0.455 | 0.457 | 0.534 | 0.519 | 0.685 |
| A_51_P485985 | NM_134163 | 171170 | Mbnl3 | 0.223 | 0.181 | 0.161 | 0.625 | 0.543 | 0.697 |
| A_52_P630964 | NM_027533 | 70747 | Tspan2 | 0.361 | 0.283 | 0.324 | 0.499 | 0.473 | 0.525 |
| A_51_P294505 | NM_025658 | 66607 | Ms4a4d | 0.63 | 0.414 | 0.365 | 0.532 | 0.521 | 0.629 |
| A_52_P82488 | NM_021476 | 58861 | Cysltr1 | 0.319 | 0.379 | 0.306 | 0.5 | 0.438 | 0.534 |
| A_52_P634829 | NM_023835 | 76681 | Trim12 | 0.59 | 0.561 | 0.672 | 1.012 | 0.996 | 0.866 |
| A_52_P460882 | XM_130319;XM_130308 | 73668; 14725 | Ttc21b | 0.412 | 0.637 | 0.533 | 0.396 | 0.633 | 0.502 |
| A_52_P197826 | NM_010397 | 15039 | H2-T22 | 0.541 | 0.505 | 0.41 | 0.653 | 0.634 | 0.622 |
| A_51_P240253 | NM_019662 | 56437 | Rrad | 0.316 | 0.322 | 0.314 | 0.392 | 0.337 | 0.342 |
| A_51_P101975 | NM_010205 | 14179 | Fgf8 | 0.412 | 0.39 | 0.413 | 0.415 | 0.454 | 0.385 |
| A_52_P21595 | NM_028238 | 72433 | Rab38 | 0.234 | 0.188 | 0.168 | 0.439 | 0.467 | 0.472 |
| A_52_P402786 | NM_008935 | 19126 | Prom1 | 0.37 | 0.396 | 0.335 | 0.551 | 0.506 | 0.594 |
| A_52_P126158 | NM_008326 | 15944 | Ifi1 | 0.237 | 0.208 | 0.22 | 0.415 | 0.412 | 0.425 |
| A_52_P676510 | NM_011579 | 21822 | Tgtp | 0.225 | 0.162 | 0.155 | 0.382 | 0.378 | 0.362 |
| A_52_P175685 | NM_008390 | 16362 | Irf1 | 0.401 | 0.37 | 0.3 | 0.663 | 0.637 | 0.749 |
| A_52_P384718 | BC052056;XM_283264 | 320873 | | 0.508 | 0.441 | 0.458 | 0.541 | 0.512 | 0.578 |
| A_52_P176333 | NM_001005787 | 12695 | Cipp | 0.372 | 0.357 | 0.365 | 0.452 | 0.392 | 0.42 |
| A_52_P358951 | AK081999;XM_134736 | 271457; 22688 | Rab5a | 0.417 | 0.378 | 0.448 | 0.464 | 0.449 | 0.46 |
| A_51_P168646 | AK039580; | | | 0.442 | 0.434 | 0.509 | 0.537 | 0.548 | 0.598 |
| A_52_P620345 | NM_133979 | 102566 | Al604832 | 0.368 | 0.402 | 0.382 | 0.441 | 0.438 | 0.493 |
| A_52_P253179 | AK077477; | 16009 | lgfbp3 | 0.397 | 0.511 | 0.257 | 0.517 | 0.468 | 0.482 |
| A_51_P397003 | XM_133990 | 68904 | 1110065L07Rik | 0.45 | 0.438 | 0.438 | 0.542 | 0.479 | 0.613 |
| A_52_P74441 | NM_011570 | 21753 | Tes | 0.423 | 0.418 | 0.328 | 0.54 | 0.558 | 0.659 |
| A_52_P255250 | NM_009543 | 22644 | Rnf103 | 0.532 | 0.371 | 0.413 | 0.471 | 0.455 | 0.491 |
| A_52_P575505 | NM_008994 | 19302 | Pxmp3 | 0.305 | 0.306 | 0.317 | 0.463 | 0.445 | 0.525 |
| A_52_P469789 | NM_007588 | 12311 | Calcr | 0.48 | 0.383 | 0.445 | 0.625 | 0.707 | 0.696 |
| A_51_P345422 | NM_010747 | 17096 | Lyn | 0.244 | 0.247 | 0.256 | 0.373 | 0.392 | 0.424 |
| A_51_P356003 | NM_001007580 | 333564 | Gm784 | 0.494 | 0.445 | 0.513 | 0.509 | 0.483 | 0.534 |
| A_52_P680941 | NM_010284 | 14600 | Ghr | 0.389 | 0.396 | 0.371 | 0.448 | 0.445 | 0.488 |
| A_52_P380263 | NM_013723 | 27205 | Podxl | 0.395 | 0.422 | 0.286 | 0.532 | 0.511 | 0.504 |
| A_52_P286528 | BC055771; | 102954 | Nudt11 | 0.411 | 0.273 | 0.263 | 0.433 | 0.371 | 0.517 |
| A_51_P105068 | NM_027990 | 71897 | 2310010M24Rik | 0.459 | 0.371 | 0.462 | 0.48 | 0.484 | 0.498 |
| A_52_P132608 | NM_172685 | 229731 | Slc25a24 | 0.358 | 0.291 | 0.25 | 0.504 | 0.401 | 0.432 |
| A_51_P467505 | NM_013689 | 21682 | Tec | 0.28 | 0.242 | 0.227 | 0.502 | 0.464 | 0.499 |
| A_52_P153685 | AK129323; | 70422 | 2810417D08Rik | 0.452 | 0.397 | 0.407 | 0.587 | 0.526 | 0.735 |
| A_51_P174943 | NM_008485 | 16782 | Lamc2 | 0.234 | 0.255 | 0.286 | 0.433 | 0.411 | 0.451 |
| A_52_P301821 | NM_053078 | 27528 | D0H4S114 | 0.442 | 0.427 | 0.469 | 0.472 | 0.525 | 0.486 |
| A_52_P532910 | NM_024427 | 22003 | Tpm1 | 0.358 | 0.392 | 0.35 | 0.386 | 0.39 | 0.433 |
| A_51_P521084 | NM_025626 | 66540 | 3110001A13Rik | 0.408 | 0.406 | 0.391 | 0.474 | 0.456 | 0.494 |
| A_52_P680436 | NM_025793 | 66840 | Wdr45l | 0.344 | 0.369 | 0.331 | 0.651 | 0.557 | 0.675 |
| A_51_P164219 | NM_011909 | 24110 | Usp18 | 0.435 | 0.307 | 0.303 | 0.435 | 0.448 | 0.457 |
| A_52_P421234 | NM_133832 | 98711 | Rdh10 | 0.356 | 0.309 | 0.338 | 0.406 | 0.4 | 0.427 |
| A_52_P586157 | NM_022309 | 12400 | Cbfb | 0.481 | 0.354 | 0.384 | 0.606 | 0.523 | 0.75 |
| A_51_P262171 | NM_008326 | 15944 | Ifi1 | 0.29 | 0.264 | 0.261 | 0.474 | 0.475 | 0.507 |
| A_52_P452689 | NM_007498 | 11910 | Atf3 | 0.291 | 0.326 | 0.293 | 0.307 | 0.321 | 0.307 |
| A_52_P295933 | AK045060; | 14051 | B130023L16Rik | 0.336 | 0.293 | 0.594 | 0.466 | 0.5 | 0.466 |
| A_51_P222773 | NM_010446 | 15376 | Foxa2 | 0.423 | 0.314 | 0.4 | 0.447 | 0.809 | 0.489 |
| A_52_P361534 | NM_009521 | 22415 | Wnt3 | 0.0992 | 0.0782 | 0.0774 | 0.211 | 0.198 | 0.243 |
| A_51_P507880 | NM_028980 | 74521 | 8430415E04Rik | 0.223 | 0.221 | 0.235 | 0.35 | 0.326 | 0.332 |
| A_52_P574668 | NM_011851 | 23959 | Nt5e | 0.15 | 0.159 | 0.148 | 0.316 | 0.285 | 0.335 |
| A_52_P957500 | XM_622354 | | | 2.438 | 1.963 | 2.267 | 2.142 | 2.231 | 1.855 |
| A_52_P345626 | AY149175; | 386626 | Mzf6d | 2.677 | 3.059 | 3.002 | 3.203 | 2.748 | 3.757 |
| A_52_P84015 | AK037762; | 11863 | | 3.675 | 3.344 | 3.705 | 4.008 | 3.981 | 3.736 |
| A_52_P432464 | AK053863; | 192196 | Luc7l2 | 2.643 | 3.226 | 2.225 | 1.973 | 2.004 | 1.643 |
| A_52_P376502 | BC025011; | 227541 | Camk1d | 1.575 | 1.641 | 1.563 | 1.989 | 2.543 | 1.959 |
| A_52_P354785 | AK037929; | 14852 | Gspt1 | 2.114 | 1.965 | 1.835 | 1.904 | 1.907 | 2.055 |
| A_51_P398766 | NM_010259 | 14468 | Gbp1 | 0.374 | 0.372 | 0.361 | 0.894 | 0.876 | 0.949 |
| A_52_P486234 | AK033497; | 22680 | | 3.81 | 2.878 | 2.669 | 3.19 | 3.59 | 3.068 |
| A_52_P628212 | AK044339; | 26380 | | 9.353 | 5.604 | 7.112 | 6.273 | 6.769 | 7.237 |
| A_52_P445799 | AK046777; | 52490 | D18Ertd289e | 0.676 | 0.602 | 0.389 | 0.613 | 0.532 | 0.592 |
| A_51_P268167 | BC037232;XM_621365 | 229363; 218850 | Gmps | 2.665 | 2.898 | 2.35 | 2.187 | 2.366 | 2.198 |
| A_51_P153079 | NM_133923 | 101100 | 4833441J24Rik | 1.312 | 1.174 | 1.271 | 1.222 | 1.343 | 1.299 |
| A_52_P584874 | NM_152895 | 75605 | Jarid1b | 2.228 | 2.064 | 2.215 | 1.844 | 1.912 | 1.769 |
| A_52_P307801 | NM_007847 | 13222 | Defcr-rs2 | 0.119 | 0.091 | 0.0705 | 0.133 | 0.175 | 0.108 |
| A_52_P163148 | AB010370; | 544945 | | 2.883 | 2.86 | 2.64 | 3.58 | 3.156 | 3.085 |
| A_51_P171728 | NM_011926 | 26365 | Ceacam1 | 2.703 | 1.992 | 2.013 | 3.11 | 3.457 | 2.977 |
| A_51_P141660 | AK053665;XM_358382 | 67776; 235380 | Loh11cr2a | 2.996 | 3.339 | 3.08 | 2.433 | 2.601 | 2.365 |
| A_52_P532323 | NM_178410 | 218518 | Marveld2 | 0.399 | 0.326 | 0.264 | 0.434 | 0.363 | 0.431 |
| A_51_P337125 | NM_010566 | 16331 | Inpp5d | 3.162 | 2.936 | 3.033 | 4.399 | 4.495 | 4.161 |
| A_52_P336259 | AK087367;XM_485576 | 319467 | A930002C04Rik | 1.736 | 2.368 | 2.023 | 1.677 | 1.679 | 1.401 |
| A_51_P318577 | AK010729; | 73671 | 2410078J06Rik | 3.283 | 4.227 | 3.815 | 6.599 | 6.454 | 5.366 |
| A_52_P324934 | AK045391; | 16403 | | 1.983 | 2.443 | 1.872 | 2.691 | 2.881 | 2.226 |
| A_52_P826666 | AK007019; | 51799 | Rap2ip | 4.063 | 3.142 | 3.547 | 3.365 | 3.764 | 3.267 |
| A_51_P200223 | XM_619497 | | | 2.332 | 2.259 | 2.383 | 1.575 | 1.797 | 1.553 |
| A_51_P228472 | NM_008343 | 16009 | Igfbp3 | 0.288 | 0.22 | 0.276 | 0.283 | 0.322 | 0.364 |
| A_52_P206573 | NM_027427 | 70439 | Taf15 | 2.14 | 3.003 | 2.282 | 1.904 | 2.123 | 1.956 |
| A_52_P354363 | NM_026574 | 68142 | | 1.74 | 1.737 | 1.937 | 1.525 | 1.61 | 1.378 |
| A_52_P651469 | NM_001012726 | 503692 | | 5.11 | 4.303 | 5.085 | 5.606 | 6.363 | 4.012 |
| A_51_P463789 | BC051947; | 58212 | | 3.253 | 2.961 | 3.321 | 2.093 | 2.306 | 1.934 |
| A_52_P18887 | NM_011468 | 20755 | Sprr2a | 0.21 | 0.171 | 0.214 | 0.282 | 0.277 | 0.25 |
| A_51_P223132 | NM_031183;XM_486227 | 83395; 70549 | Sp6 | 2.329 | 2.088 | 2.196 | 1.457 | 1.639 | 1.36 |
| A_52_P223083 | BC038323; | 72096 | 2010208K18Rik | 4.486 | 6.15 | 3.745 | 3.784 | 3.918 | 3.378 |
| A_52_P523399 | NM_029444 | 75801 | 4930447C04Rik | 3.312 | 7.019 | 4.341 | 3.597 | 4.009 | 2.819 |
| A_52_P282000 | AK020248; | 13800 | Enah | 2.7 | 2.435 | 2.683 | 2.632 | 2.812 | 2.59 |
| A_52_P152983 | AK011739; | 26886 | Cenph | 2.134 | 3.107 | 2.171 | 2.114 | 2.408 | 2.327 |
| A_52_P26416 | AF106279; | 16782 | | 0.254 | 0.252 | 0.241 | 0.358 | 0.391 | 0.378 |
| A_51_P102081 | NM_007847 | 13222 | Defcr-rs2 | 0.061 | 0.0426 | 0.0644 | 0.0791 | 0.158 | 0.0864 |
| A_51_P285725 | NM_009309 | 20997 | T | 0.141 | 0.102 | 0.113 | 0.134 | 0.13 | 0.141 |
| A_52_P827911 | AK081941; | | | 2.387 | 2.657 | 2.47 | 2.075 | 2.44 | 2.116 |
| A_51_P231341 | AK087764; | 108160 | D0HXS9928E | 2.885 | 3.795 | 2.39 | 3.305 | 3.578 | 2.451 |
| A_51_P334093 | NM_026228 | 67547 | Slc39a8 | 0.208 | 0.0771 | 0.105 | 0.19 | 0.198 | 0.231 |
| A_52_P196568 | NM_144513 | 17263 | Gtl2 | 1.892 | 2.538 | 1.516 | 1.938 | 1.959 | 1.652 |
| A_52_P1108139 | AK053079; | | | 1.801 | 2.214 | 1.538 | 1.924 | 2.168 | 1.724 |
| A_52_P636559 | AK053008; | 268469 | 9530033F24Rik | 2.63 | 2.537 | 2.752 | 2.132 | 2.334 | 1.998 |
| A_52_P425734 | NM_145146 | 280662 | Afm | 3.913 | 3.546 | 3.74 | 3.764 | 4.135 | 3.335 |
| A_52_P1011737 | AK039096; | 106258 | Al790442 | 1.846 | 1.977 | 1.978 | 2.087 | 2.67 | 2.065 |
| A_52_P537140 | AK047235; | | | 1.74 | 2.015 | 2.044 | 1.613 | 1.708 | 1.261 |
| A_51_P191990 | AK087548; | 26563 | Ror1 | 1.628 | 1.623 | 1.626 | 1.509 | 1.588 | 1.479 |
| A_51_P478930 | AK080016; | | | 2.436 | 2.701 | 2.337 | 2.779 | 2.919 | 2.466 |
| A_51_P252565 | NM_009660 | 11687 | Alox15 | 0.236 | 0.24 | 0.156 | 0.217 | 0.231 | 0.212 |
| A_52_P81252 | NM_172599 | 218978 | D14Ertd436e | 2.478 | 2.667 | 2.607 | 1.82 | 1.981 | 1.764 |
| A_51_P487004 | NM_027732 | 71241 | Dmrtc2 | 10.3 | 11.69 | 10.98 | 5.024 | 6.192 | 5.685 |
| A_51_P104162 | AK053379; | 328232 | | 2.537 | 3.096 | 3.391 | 3.126 | 2.998 | 3.127 |
| A_52_P555603 | AK032770; | 11787 | Apbb2 | 1.409 | 1.222 | 1.29 | 1.326 | 1.427 | 1.18 |
| A_52_P819356 | AK041684; | | | 3.003 | 2.848 | 2.7 | 3.026 | 2.915 | 3.006 |
| A_52_P1196751 | AK086488; | | | 2.217 | 2.193 | 2.198 | 2.38 | 2.677 | 2.156 |
| A_52_P277415 | AK077313; | 76252 | 0610006O14Rik | 1.282 | 1.291 | 1.224 | 0.99 | 1.044 | 0.981 |
| A_52_P812362 | BC016205; | 408064 | BC064078 | 0.25 | 0.167 | 0.217 | 0.302 | 0.27 | 0.27 |
| A_51_P352572 | NM_019701 | 56365 | Clcnkb | 5.193 | 4.267 | 4.344 | 4.514 | 5.226 | 4.635 |
| A_52_P100042 | AK045143; | 320938 | Tnpo3 | 1.761 | 1.695 | 1.74 | 1.697 | 1.665 | 1.526 |
| A_51_P103397 | AK078521; | 54561 | Nap1l3 | 2.701 | 2.645 | 2.885 | 3.303 | 3.612 | 3.031 |
| A_52_P296244 | BC048734; | | | 0.369 | 0.373 | 0.292 | 0.457 | 0.42 | 0.426 |
| A_51_P305019 | AK020601; | 78423 | 9530049005Rik | 1.297 | 1.416 | 1.484 | 1.43 | 1.551 | 1.387 |
| A_52_P3304 | NM_080446 | 117599 | Helb | 2.823 | 2.774 | 3.121 | 2.206 | 2.73 | 2.462 |
| A_51_P147651 | AK041797;XM_146705 | 245902 | | 2.797 | 2.965 | 2.694 | 2.18 | 2.325 | 1.652 |
| A_51_P203955 | NM_010260 | 14469 | Gbp2 | 0.252 | 0.267 | 0.292 | 0.582 | 0.578 | 0.664 |
| A_51_P176474 | AK010466; | 76484 | 2410012C07Rik | 2.797 | 2.691 | 2.733 | 3.87 | 4.072 | 3.726 |
| A_52_P165497 | NM_011385 | 20481 | Ski | 2.312 | 2.37 | 2.474 | 1.296 | 1.485 | 1.406 |
| A_51_P358714 | AK031433; | | | 2.043 | 2.448 | 1.68 | 2.647 | 2.627 | 1.899 |
| A_51_P106918 | NM_009051 | 19715 | Rex2 | 2.006 | 2.032 | 2.425 | 6.886 | 2.95 | 2.717 |
| A_51_P124315 | NM_177649 | 227712 | BC034076 | 1.796 | 1.742 | 1.797 | 2.396 | 2.68 | 2.14 |
| A_52_P183368 | NM_011535 | 21386 | Tbx3 | 1.964 | 1.81 | 1.931 | 2.023 | 2.272 | 2.026 |
| A_51_P140751 | NM_011397 | 20522 | Slc23a1 | 1.819 | 1.664 | 1.85 | 2.855 | 2.607 | 2.546 |
| A_51_P103406 | NM_011708 | 22371 | Vwf | 3.089 | 2.827 | 2.64 | 4.179 | 4.502 | 3.84 |
| A_51_P381069 | XM_132045 | 231252 | Chrna9 | 1.424 | 2.361 | 3.023 | 3.047 | 2.764 | 2.469 |
| A_52_P75971 | NM_026599 | 68178 | Cgnl1 | 1.56 | 1.535 | 1.702 | 1.265 | 1.357 | 1.222 |
| A_52_P135873 | XM_132015;XM_488584 | 231128 | BC037112 | 1.243 | 1.332 | 1.392 | 1.507 | 1.53 | 1.464 |
| A_51_P165082 | AB041601;XM_489523 | 224019 | D16Bwg1494e | 3.114 | 3.318 | 3.453 | 3.352 | 3.568 | 3.01 |
| A_51_P294566 | AK020687; | 78083 | 9930116N10Rik | 2.623 | 2.571 | 2.706 | 3.706 | 4.077 | 3.155 |
| A_51_P378051 | NM_009675 | 11754 | Aoc3 | 2.988 | 3.162 | 3.341 | 3.251 | 3.583 | 3.381 |
| A_52_P420608 | NM_201366 | 381371 | Gm1631 | 2.287 | 3.006 | 2.818 | 2.552 | 2.723 | 3.242 |
| A_51_P221291 | NM_009229 | 20650 | Sntb2 | 1.876 | 1.793 | 1.783 | 1.622 | 1.693 | 1.524 |
| A_52_P362805 | AK044730; | 269254 | Als4 | 2.262 | 2.248 | 2.26 | 1.818 | 1.967 | 1.718 |
| A_51_P109649 | AK018142; | 17263 | Gtl2 | 1.183 | 1.072 | 1.216 | 1.06 | 1.024 | 0.976 |
| A_52_P413348 | XM_127674 | 75698 | 3110001K24Rik | 2.072 | 2.063 | 2.27 | 1.908 | 2.161 | 1.733 |
| A_52_P400425 | NM_178656 | 193003 | A530088H08Rik | 2.498 | 2.036 | 2.006 | 2.674 | 2.834 | 2.576 |
| A_52_P147870 | NM_029963 | 77721 | Mrps5 | 2.14 | 2.028 | 2.027 | 1.789 | 1.827 | 1.856 |
| A_52_P57078 | AK052455; | | | 2.172 | 1.912 | 1.757 | 1.727 | 1.952 | 1.877 |
| A_52_P389874 | NM_011918 | 24131 | Ldb3 | 2.421 | 2.244 | 2.421 | 2.216 | 2.284 | 2.066 |
| A_52_P224125 | XM_622260 | | | 1.795 | 1.653 | 1.727 | 1.625 | 1.654 | 1.663 |
| A_52_P238468 | XM_133238 | 381853 | Gm160 | 2.307 | 2.173 | 2.136 | 1.929 | 1.906 | 1.683 |
| A_51_P253053 | XM_618867 | | | 2.544 | 3.669 | 3.053 | 2.284 | 2.658 | 3.007 |
| A_52_P500979 | NM_013628 | 18548 | Pcsk1 | 0.75 | 0.686 | 0.598 | 1.08 | 1.031 | 0.991 |
| A_51_P207934 | NM_199016 | 224794 | Enpp4 | 2.477 | 2.57 | 2.229 | 2.606 | 2.707 | 2.427 |
| A_52_P423384 | BC057551;XM_354546 | 104248 | Cabin1 | 1.58 | 1.963 | 1.524 | 1.442 | 1.596 | 1.426 |
| A_52_P560006 | NM_008426 | 16519 | Kcnj3 | 0.763 | 0.536 | 0.497 | 0.479 | 0.449 | 0.493 |
| A_52_P654624 | AK005333; | 236604 | 4933439C20Rik | 2.049 | 1.763 | 2.06 | 1.639 | 1.865 | 1.518 |
| A_52_P32502 | AK020822; | 77944 | A930007B11Rik | 2.593 | 4.558 | 2.534 | 2.973 | 3.231 | 2.237 |
| A_51_P302377 | NM_008009 | 14181 | Fgfbp1 | 0.258 | 0.246 | 0.273 | 0.413 | 0.381 | 0.451 |
| A_52_P255224 | AK032711;XM_486482 | 234371 | BC021367 | 2.31 | 3.108 | 2.526 | 2.817 | 2.716 | 2.5 |
| A_52_P571746 | XM_355533;XM_357399 | 230863 | LOC381571 | 5.455 | 5.043 | 4.955 | 4.478 | 4.627 | 3.701 |
| A_52_P458916 | AK016751; | 218850 | D14Abb1e | 2.421 | 2.013 | 2.081 | 2.688 | 2.359 | 2.992 |
| A_52_P286970 | XM_131162;XM_127565 | 76561 | Snx7 | 0.44 | 0.492 | 0.435 | 0.608 | 0.611 | 0.895 |
| A_51_P266644 | BC055753;XM_125538 | 140742 | | 0.424 | 0.369 | 0.474 | 0.821 | 0.739 | 0.949 |
| A_52_P233673 | NM_020594 | 57432 | Zc3hdc8 | 3.001 | 3.71 | 2.727 | 1.913 | 2.05 | 1.886 |
| A_52_P1084149 | AK051413; | 553138 | 9930021J03Rik | 1.669 | 1.913 | 1.945 | 1.806 | 2.202 | 1.595 |
| A_52_P413661 | BC079861;XM_484162 | 217716 | Mlh3 | 2.28 | 2.065 | 2.239 | 2.466 | 2.362 | 2.456 |
| A_51_P404193 | NM_022435 | 64406 | Sp5 | 0.194 | 0.182 | 0.19 | 0.214 | 0.202 | 0.207 |
| A_52_P565940 | AF419220; | 18193 | Nsd1 | 1.84 | 1.873 | 1.944 | 1.445 | 1.546 | 1.33 |
| A_51_P514177 | NM_028838 | 74249 | Lrrc2 | 3.542 | 3.2 | 3.896 | 3.884 | 4.114 | 3.603 |
| A_52_P237357 | AF095690; | | | 1.813 | 1.675 | 1.846 | 1.603 | 1.706 | 1.432 |
| A_51_P240811 | NM_009290 | 20890 | Wnt8a | 0.709 | 0.589 | 0.527 | 1.532 | 0.979 | 1.578 |
| A_51_P236267 | NM_009183 | 20452 | St8sia4 | 0.265 | 0.182 | 0.148 | 0.233 | 0.291 | 0.32 |
| A_52_P405340 | U76762; | 14158 | Fert2 | 2.538 | 2.399 | 2.414 | 2.011 | 2.124 | 1.824 |
| A_52_P661144 | XM_485365 | | | 2.066 | 2.37 | 2.399 | 2.3 | 2.365 | 2.13 |
| A_52_P1044262 | AK051949; | | | 1.478 | 1.257 | 1.19 | 1.611 | 1.769 | 1.41 |
| A_52_P553100 | AK040413; | | | 2.967 | 3.025 | 3.491 | 3.19 | 3.168 | 2.79 |
| A_51_P358344 | NM_007732 | 12821 | Col17a1 | 3.116 | 2.917 | 3.011 | 3.334 | 3.243 | 2.913 |
| A_51_P321579 | XM_131865;XM_196334 | 433809 | LOC433809 | 2.839 | 2.589 | 2.323 | 1.805 | 1.942 | 1.521 |
| A_52_P485939 | NM_026228 | 67547 | Slc39a8 | 0.407 | 0.418 | 0.26 | 0.31 | 0.338 | 0.278 |
| A_52_P199922 | | | | 3.785 | 1.908 | 2.417 | 2.334 | 2.502 | 2.545 |
| A_51_P463791 | BC051947; | 58212 | | 3.074 | 2.866 | 2.865 | 2.058 | 2.064 | 1.757 |
| A_52_P487362 | NM_028980 | 74521 | 8430415E04Rik | 0.317 | 0.316 | 0.334 | 0.477 | 0.454 | 0.469 |
| A_51_P341137 | AK035176; | 224829 | Trerf1 | 1.935 | 1.833 | 2.021 | 2.024 | 2.334 | 1.984 |
| A_51_P407323 | NM_007976 | 14067 | F5 | 0.17 | 0.072 | NO DATA | 0.352 | 0.305 | 0.242 |
| A_51_P272363 | NM_134160 | 171166 | Mcoln3 | 0.559 | 0.82 | 0.792 | 0.686 | 0.762 | 0.915 |
| A_52_P430927 | AK042385; | 11908 | Atf1 | 1.603 | 1.64 | 1.71 | 1.368 | 1.604 | 1.26 |
| A_52_P626069 | AK040994;XM_284439 | 109151 | Chd9 | 1.61 | 3.967 | 1.955 | 2.209 | 2.26 | 1.715 |
| A_52_P355276 | NM_001002764 | 103677 | Al317223 | 2.549 | 2.993 | 2.548 | 1.703 | 1.935 | 1.511 |
| A_52_P650215 | NM_029278 | 75416 | 2610033H07Rik | 1.439 | 4.127 | 1.948 | 2.359 | 2.484 | 1.577 |
| A_52_P416123 | AK020480; | 77358 | 9430069107Rik | 2.511 | 2.833 | 2.298 | 1.785 | 1.993 | 1.656 |
| A_51_P189631 | NM_172427 | 76455 | 2310067E19Rik | 1.139 | 0.941 | 0.985 | 1.62 | 1.653 | 1.604 |
| A_52_P16973 | AK081934; | 320391 | D930017J03Rik | 2.586 | 2.277 | 2.281 | 2.137 | 2.272 | 1.872 |
| A_52_P947879 | AK048751; | | | 1.687 | 1.729 | 1.647 | 2.149 | 2.232 | 2.009 |
| A_52_P538515 | BC049181; | 226412 | R3hdm | 2.219 | 2.049 | 2.456 | 1.981 | 2.364 | 1.119 |
| A_52_P179250 | AK129127; | 230249 | Al314180 | 5.166 | 6.292 | 4.565 | 8.052 | 10.48 | 5.993 |
| A_52_P795628 | AK086352; | | | 4.861 | 5.344 | 4.508 | 6.792 | 7.236 | 5.913 |
| A_51_P144712 | NM_178930 | 107338 | Gbf1 | 1.805 | 1.74 | 1.841 | 1.291 | 1.456 | 1.202 |
| A_52_P655008 | AK044382; | 320678 | A930037G23Rik | 1.653 | 1.779 | 1.572 | 1.52 | 1.586 | 1.325 |
| A_52_P577072 | NM_011901 | 24074 | Taf7 | 2.198 | 2.692 | 2.144 | 1.999 | 2.035 | 1.851 |
| A_52_P636199 | NM_008017 | 14211 | Smc2l1 | 1.61 | 2.123 | 1.631 | 1.531 | 1.607 | 1.38 |
| A_51_P447189 | AK041326;XM_358090 | 385154 | | 2.336 | 2.35 | 2.641 | 3.32 | 2.976 | 3.108 |
| A_52_P1180315 | AK049088; | 74022 | 3930401K13Rik | 1.789 | 1.737 | 1.505 | 1.313 | 1.364 | 1.207 |
| A_52_P412167 | NM_080467 | 140494 | Atp6v0a4 | 2.324 | 2.449 | 2.406 | 4.276 | 4.719 | 3.334 |
| A_52_P335089 | BE198552; | | | 1.331 | 1.348 | 1.455 | 1.151 | 1.229 | 0.977 |
| A_52_P6621 | | | | 2.585 | 4.954 | 2.769 | 4.277 | 4.565 | 2.653 |
| A_51_P343129 | AK012079; | 70472 | Atad2 | 3.752 | 4.107 | 4.116 | 2.307 | 2.496 | 1.87 |
| A_51_P144417 | NM_181750 | 226412 | R3hdm | 2.79 | 2.726 | 2.677 | 2.283 | 2.582 | 2.164 |
| A_52_P350477 | NM_139295 | 193813 | Mcfd2 | 0.43 | 0.349 | 0.245 | 0.661 | 0.554 | 0.759 |
| A_51_P418884 | NM_001013789 | 432823 | | 1.678 | 2.207 | 2.11 | 2.759 | 2.736 | 2.272 |
| A_52_P581138 | NM_212450 | 329506 | D2Ertd485e | 1.847 | 1.821 | 1.932 | 1.418 | 1.573 | 1.284 |
| A_52_P434549 | NM_023190 | 56215 | Acin1 | 1.868 | 1.876 | 1.953 | 1.241 | 1.454 | 1.123 |
| A_52_P384392 | AK080827; | 328087 | D930001B02 | 2.365 | 2.375 | 2.393 | 1.135 | 1.269 | 1.098 |
| A_51_P270725 | AK089493; | 102093 | Phkb | 2.083 | 3.565 | 2.709 | 2.953 | 2.589 | 2.102 |
| A_51_P193019 | NM_011157 | 19073 | Prg1 | 1.283 | 1.107 | 1.495 | 0.759 | 3.853 | 1.325 |
| A_52_P1172548 | AK084146; | 13204 | Dhx15 | 2.924 | 3.082 | 2.339 | 3.164 | 3.492 | 2.732 |
| A_51_P114606 | NM_016965 | 50884 | Nckap1 | 0.446 | 0.473 | 0.337 | 0.707 | 0.582 | 0.773 |
| A_52_P306387 | NM_178709 | 235315 | D130054N24Rik | 2.399 | 3.176 | 2.415 | 1.899 | 2.109 | 1.481 |
| A_52_P287576 | AK036308; | | | 1.703 | 2.271 | 1.347 | 2.425 | 2.794 | 1.7 |
| A_52_P501767 | AK012272; | 20637 | Snrp70 | 1.314 | 1.333 | 1.34 | 0.962 | 1 | 0.898 |
| A_52_P995717 | AK045222; | | | 3.292 | 3.495 | 3.303 | 2.04 | 2.108 | 1.729 |

**Table 2B - 292 Gene Set (Non Germline Comeptent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 1.152 | 1.407 | 1.101 | 0.859 | 0.96 | 0.924 |
| A_51_P335460 | NM_009132 | 20259 | Scin | 1.658 | 1.913 | 1.519 | 1.101 | 1.225 | 1.107 |
| A_52_P391505 | NM_144513 | 17263 | Gtl2 | 0.0793 | 0.114 | 0.0867 | 0.158 | 0.15 | 0.148 |
| A_51_P497100 | NM_010706 | 16855 | Lgals4 | 0.692 | 0.771 | 0.849 | 1.002 | 0.908 | 0.828 |
| A_51_P378371 | | | | 0.986 | 1.006 | 1.208 | 1.217 | 1.156 | 1.234 |
| A_51_P298266 | NM_025681 | 66643 | Lix1 | 1.123 | 1.145 | 1.109 | 1.143 | 1.15 | 1.141 |
| A_52_P269158 | NM_001003948 | 98496 | AL024069 | 1.775 | 1.419 | 1.506 | 1.494 | 1.391 | 1.254 |
| A_51_P311038 | BC009666;XM_134720 | 57267 | Apba3 | 1.847 | 1.642 | 1.538 | 2.55 | 2.405 | 2.808 |
| A_51_P457187 | NM_028075 | 72049 | Tnfrsf13c | 1.083 | 1.58 | 1.373 | 0.916 | 1.099 | 1.091 |
| A_52_P68702 | NM_145148 | 232288 | Frmd4b | 0.928 | 1.071 | 0.877 | 1.242 | 1.234 | 1.139 |
| A_52_P293443 | AK087208; | 13819 | | 0.179 | 0.222 | 0.219 | 0.543 | 0.565 | 0.552 |
| A_51_P157986 | NM_029930 | 77574 | 3321401G04Rik | 0.909 | 1.196 | 1.088 | 0.563 | 0.621 | 0.598 |
| A_52_P1013441 | | | | 1.255 | 1.032 | 2.041 | 1.173 | 1.203 | 1.341 |
| A_51_P418375 | NM_023844 | 67374 | Jam2 | 1.044 | 0.943 | 0.99 | 0.826 | 0.786 | 0.84 |
| A_52_P557940 | AK004302; | 66704 | 4921506I22Rik | 0.544 | 0.595 | 0.569 | 1.371 | 1.379 | 1.241 |
| A_51_P480311 | NM_010168 | 14061 | F2 | 0.348 | 0.476 | 0.622 | 0.996 | 1.004 | 1.186 |
| A_52_P200458 | NM_010242 | 14345 | Fut4 | 1.003 | 0.997 | 1.017 | 1.108 | 1.203 | 1.09 |
| A_51_P463846 | NM_145545 | 229900 | 9830147J24Rik | 2.28 | 2.199 | 2.49 | 2.068 | 1.958 | 1.72 |
| A_51_P162474 | AF285577; | 107815 | Scml2 | 2.885 | 2.549 | 2.371 | 3.021 | 3.116 | 3.1 |
| A_51_P165244 | NM_018734 | 55932 | Gbp4 | 2.388 | 1.319 | 2.543 | 2.404 | 2.051 | 2.534 |
| A_52_P59318 | NM_133832 | 98711 | Rdh10 | 1.689 | 1.611 | 1.584 | 0.876 | 1.002 | 0.955 |
| A_52_P438036 | NM_175271 | 78134 | Gpr23 | 1.082 | 0.96 | 0.968 | 1.634 | 1.593 | 1.618 |
| A_52_P474242 | BE457768; | | | 1.31 | 1.785 | 1.55 | 1.137 | 1.132 | 0.997 |
| A_51_P499854 | NM_010284 | 14600 | Ghr | 1.55 | 1.19 | 1.486 | 2.502 | 2.537 | 2.196 |
| A_51_P455647 | NM_009801 | 12349 | Car2 | 1.592 | 1.463 | 1.337 | 2.139 | 2.347 | 2.17 |
| A_52_P103929 | NM_009306 | 20979 | Syt1 | 0.898 | 0.938 | 0.967 | 1.626 | 1.464 | 1.422 |
| A_51_P149469 | NM_009608 | 11464 | Actc1 | 1.012 | 1.006 | 0.935 | 0.971 | 0.997 | 1.12 |
| A_52_P557293 | NM_015775 | 50528 | Tmprss2 | 1.142 | 1.091 | 1.111 | 1.208 | 1.127 | 1.225 |
| A_52_P50496 | AY048587;XM_619453 | 114640 | Tifp39 | 1.196 | 1.551 | 1.504 | 1.114 | 1.171 | 1.214 |
| A_51_P180413 | NM_176930 | 319504 | C130076O07Rik | 1.603 | 1.54 | 1.395 | 1.297 | 1.25 | 1.155 |
| A_51_P229613 | NM_008792 | 18549 | Pcsk2 | 1.463 | 1.53 | 1.37 | 1.333 | 1.422 | 1.554 |
| A_51_P121635 | NM_030700 | 80884 | Maged2 | 1.225 | 1.426 | 1.335 | 0.918 | 0.99 | 0.88 |
| A_51_P481777 | NM_018756 | 54382 | Tcstv1 | 0.208 | 0.696 | 0.654 | 0.592 | 0.503 | 0.72 |
| A_51_P412817 | NM_177617 | 216851 | D330014H01Rik | 0.725 | 0.834 | 1.04 | 1.083 | 0.986 | 0.997 |
| A_51_P387528 | AK019664; | 78801 | Ak7 | 0.65 | 0.537 | 0.813 | 0.767 | 0.778 | 1.11 |
| A_52_P78168 | AK031518; | | | 0.277 | 0.385 | 0.299 | 0.704 | 0.652 | 0.544 |
| A_52_P274960 | NM_001005508 | 226652 | 6030405P05Rik | 0.671 | 0.634 | 0.712 | 0.763 | 0.781 | 0.808 |
| A_52_P1188270 | AK042362; | | | 0.341 | 0.594 | 0.564 | 1.758 | 1.462 | 1.658 |
| A_51_P291227 | NM_001005508 | 226652 | 6030405P05Rik | 0.705 | 0.681 | 0.731 | 0.814 | 0.735 | 0.798 |
| A_52_P377299 | AK048360; | 271377 | Zbtb11 | 0.577 | 0.582 | 0.645 | 1.152 | 1.092 | 1.023 |
| A_51_P297131 | NM_009502 | 22330 | Vcl | 1.015 | 1.436 | 0.955 | 0.556 | 0.672 | 0.587 |
| A_52_P804224 | | | | 0.299 | 0.314 | 0.291 | 1.263 | 1.2 | 1.068 |
| A_51_P442894 | AK048310; | 621603 | C130048D07Rik | 1.023 | 0.841 | 1.218 | 0.788 | 0.867 | 0.977 |
| A_52_P309718 | AK173099; | 218850 | D14Abb1e | 1.006 | 0.813 | 1.127 | 1.186 | 1.29 | 1.289 |
| A_52_P359010 | AK129478; | 72193 | Sfrs2ip | 0.54 | 0.706 | 0.574 | 1.484 | 1.398 | 1.403 |
| A_51_P410813 | AK079732; | | | 0.717 | 0.877 | 0.904 | 1.593 | 1.473 | 1.607 |
| A_52_P382565 | XM_483943 | 432493 | LOC432493 | 0.228 | 0.273 | 0.254 | 0.607 | 0.622 | 0.462 |
| A_51_P333253 | NM_178440 | 246177 | Myo1g | 0.535 | 0.491 | 0.538 | 0.474 | 0.481 | 0.37 |
| A_52_P113190 | NM_053214 | 17916 | Myo1f | 0.386 | 0.513 | 0.397 | 0.243 | 0.232 | 0.264 |
| A_51_P473953 | XM_358600;XM_355454 | 381498 | 4930592C13Rik | 2.138 | 1.593 | 1.947 | 2.323 | 2.226 | 2.26 |
| A_52_P519870 | AB010340; | | | 0.758 | 0.675 | 1.128 | 0.989 | 0.863 | 1.393 |
| A_51_P266403 | AK009163; | 76730 | 2310005C01Rik | 0.984 | 0.901 | 1.009 | 1.273 | 1.313 | 1.391 |
| A_52_P3698 | NM_001015099 | 217558 | | 0.658 | 0.653 | 0.676 | 1.487 | 1.564 | 1.537 |
| A_52_P581100 | NM_027285 | 70005 | 1700029I01Rik | 1.164 | 1.1 | 1.141 | 1.525 | 1.383 | 1.305 |
| A_51_P276305 | NM_009292 | 20899 | Stra8 | 0.873 | 0.934 | 0.982 | 0.431 | 0.426 | 0.471 |
| A_51_P455997 | Y13832; | 17263 | Gtl2 | 0.123 | 0.184 | 0.114 | 0.213 | 0.197 | 0.258 |
| A_52_P656336 | NM_023733 | 74114 | Crot | 1.233 | 1.547 | 1.442 | 0.983 | 1.067 | 0.998 |
| A_51_P512172 | AK010953;XM_125673 | 52463 | Cxxc6 | 0.8 | 0.986 | 0.748 | 1.033 | 1.014 | 1.046 |
| A_51_P282227 | NM_020577 | 57344 | As3mt | 0.79 | 0.761 | 0.777 | 1.02 | 0.91 | 0.98 |
| A_52_P386544 | NM_026594 | 68172 | 4930517K11Rik | 0.74 | 0.606 | 0.729 | 1.188 | 1.159 | 1.351 |
| A_51_P125679 | AK010725;XM_134948 | 73673 | 2410076I21Rik | 0.588 | 0.959 | 0.916 | 0.84 | 0.843 | 0.849 |
| A_51_P164504 | NM_007469 | 11812 | Apoc1 | 1.307 | 1.245 | 1.314 | 1.412 | 1.375 | 1.457 |
| A_52_P240706 | NM_007543 | 26367 | Ceacam2 | 0.87 | 0.778 | 0.892 | 0.689 | 0.756 | 0.859 |
| A_52_P420369 | NM_029444 | 75801 | 4930447C04Rik | 1.087 | 1.084 | 1.129 | 1.176 | 0.989 | 1.041 |
| A_51_P242399 | NM_031170 | 16691 | Krt2-8 | 1.013 | 1.199 | 0.939 | 0.962 | 0.977 | 0.946 |
| A_51_P158037 | NM_013505 | 13506 | Dsc2 | 1.238 | 1.562 | 1.304 | 0.743 | 0.819 | 0.875 |
| A_52_P258116 | NM_009521 | 22415 | Wnt3 | 1.304 | 1.249 | 1.083 | 1.811 | 1.943 | 2.006 |
| A_52_P286166 | NM_001002927 | 18619 | Penk1 | 1.649 | 2.509 | 2.439 | 1.542 | 1.453 | 1.374 |
| A_52_P399095 | NM_177793 | 327747 | 9030224M15Rik | 1.11 | 0.976 | 1.056 | 1.583 | 1.692 | 1.596 |
| A_52_P470663 | | | | 2.339 | 2.72 | 2.405 | 1.069 | 1.105 | 1.018 |
| A_52_P376169 | NM_177139 | 320343 | E130115E03Rik | 1.184 | 1.025 | 1.076 | 1.949 | 1.81 | 1.828 |
| A_51_P512085 | NM_130449 | 140792 | Colec12 | 1.574 | 1.25 | 1.471 | 1.463 | 1.651 | 1.693 |
| A_52_P676403 | NM_019494 | 56066 | Cxcl11 | 3.86 | 3.177 | 3.663 | 2.834 | 2.723 | 2.826 |
| A_51_P354307 | NM_172469 | 209195 | Clic6 | 1.31 | 1.578 | 1.57 | 0.719 | 0.748 | 0.659 |
| A_52_P569375 | NM_010203 | 14176 | Fgf5 | 4.338 | 3.206 | 3.958 | 5.889 | 5.927 | 6.75 |
| A_51_P162481 | NM_133194 | 107815 | Scml2 | 2.434 | 2.616 | 2.232 | 2.312 | 2.394 | 2.596 |
| A_51_P202911 | NM_144882 | 67198 | 2810022L02Rik | 1.005 | 0.995 | 0.908 | 0.913 | 0.931 | 0.902 |
| A_51_P418029 | NM_175541 | 245631 | Mum1I1 | 2.026 | 1.803 | 2.159 | 1.492 | 1.654 | 1.714 |
| A_52_P93837 | NM_008604 | 17380 | Mme | 1.516 | 1.805 | 1.761 | 1.442 | 1.369 | 1.329 |
| A_51_P275435 | NM_172411 | 71874 | 2310007B03Rik | 3.355 | 3.105 | 3.53 | 5.636 | 6.155 | 6.288 |
| A_51_P199888 | NM_026082 | 67299 | Dock7 | 1.182 | 1.246 | 1.295 | 0.669 | 0.678 | 1 |
| A_52_P211956 | NM_025658 | 66607 | Ms4a4d | 1.71 | 1.799 | 1.851 | 2.105 | 2.274 | 1.961 |
| A_51_P485985 | NM_134163 | 171170 | Mbnl3 | 1.781 | 1.802 | 1.648 | 0.929 | 1.067 | 1.035 |
| A_52_P630964 | NM_027533 | 70747 | Tspan2 | 1.217 | 1.314 | 1.26 | 0.815 | 0.863 | 0.897 |
| A_51_P294505 | NM_025658 | 66607 | Ms4a4d | 1.546 | 1.551 | 1.883 | 2.127 | 2.066 | 2.183 |
| A_52_P82488 | NM_021476 | 58861 | Cysltr1 | 1.919 | 1.474 | 1.811 | 2.106 | 2.164 | 2.621 |
| A_52_P634829 | NM_023835 | 76681 | Trim12 | 1.52 | 1.676 | 1.775 | 1.472 | 1.69 | 1.577 |
| A_52_P460882 | XM_130319;XM_130308 | 73668; 14725 | Ttc21b | 1.498 | 1.698 | 2.077 | 1.06 | 1.276 | 1.219 |
| A_52_P197826 | NM_010397 | 15039 | H2-T22 | 1.502 | 1.687 | 1.444 | 1.028 | 1.049 | 1.039 |
| A_51_P240253 | NM_019662 | 56437 | Rrad | 1.392 | 1.648 | 1.29 | 0.851 | 0.9 | 0.913 |
| A_51_P101975 | NM_010205 | 14179 | Fgf8 | 2.683 | 2.96 | 2.139 | 3.25 | 3.463 | 3.854 |
| A_52_P21595 | NM_028238 | 72433 | Rab38 | 1.043 | 0.791 | 0.992 | 0.807 | 0.819 | 0.929 |
| A_52_P402786 | NM_008935 | 19126 | Prom1 | 1.498 | 1.71 | 1.749 | 1.028 | 1.023 | 0.92 |
| A_52_P126158 | NM_008326 | 15944 | Ifi1 | 1.157 | 1.034 | 1.006 | 1.254 | 1.3 | 1.232 |
| A_52_P676510 | NM_011579 | 21822 | Tgtp | 1.166 | 1.03 | 1.064 | 1.15 | 1.119 | 1.082 |
| A_52_P175685 | NM_008390 | 16362 | Irf1 | 1.072 | 1.33 | 1.162 | 0.971 | 1.106 | 1.017 |
| A_52_P384718 | BC052056;XM_283264 | 320873 | | 1.488 | 1.451 | 1.435 | 1.051 | 1.032 | 1.026 |
| A_52_P176333 | NM_001005787 | 12695 | Cipp | 1.168 | 1.146 | 1.249 | 0.974 | 1.014 | 1.036 |
| A_52_P358951 | AK081999;XM_134736 | 271457; 22688 | Rab5a | 1.346 | 1.445 | 1.326 | 0.843 | 0.883 | 0.787 |
| A_51_P168646 | AK039580; | | | 1.655 | 1.373 | 1.436 | 2.357 | 2.478 | 2.267 |
| A_52_P620345 | NM_133979 | 102566 | A1604832 | 1.26 | 1.488 | 1.375 | 0.778 | 0.752 | 0.729 |
| A_52_P253179 | AK077477; | 16009 | Igfbp3 | 2.157 | 1.536 | 2.035 | 1.993 | 1.965 | 2.545 |
| A_51_P397003 | XM_133990 | 68904 | 1110065L07Rik | 1.235 | 1.343 | 1.238 | 0.778 | 0.763 | 0.855 |
| A_52_P74441 | NM_011570 | 21753 | Tes | 1.498 | 1.167 | 1.425 | 0.853 | 0.882 | 0.885 |
| A_52_P255250 | NM_009543 | 22644 | Rnf103 | 1.205 | 1.217 | 1.524 | 0.663 | 0.701 | 0.687 |
| A_52_P575505 | NM_008994 | 19302 | Pxmp3 | 1.123 | 1.07 | 1.092 | 0.797 | 0.815 | 0.802 |
| A_52_P469789 | NM_007588 | 12311 | Calcr | 2.087 | 2.206 | 2.278 | 1.646 | 1.638 | 1.533 |
| A_51_P345422 | NM_010747 | 17096 | Lyn | 0.888 | 0.803 | 0.881 | 1.006 | 0.995 | 0.89 |
| A_51_P356003 | NM_001007580 | 333564 | Gm784 | 1.223 | 1.221 | 1.186 | 1.543 | 1.618 | 1.409 |
| A_52_P680941 | NM_010284 | 14600 | Ghr | 1.72 | 1.563 | 1.682 | 2.058 | 1.978 | 2.065 |
| A_52_P380263 | NM_013723 | 27205 | Podxl | 1.179 | 1.159 | 1.66 | 0.899 | 0.909 | 0.942 |
| A_52_P286528 | BC055771; | 102954 | Nudt11 | 1.419 | 1.344 | 1.431 | 1.048 | 1.063 | 1.053 |
| A_51_P105068 | NM_027990 | 71897 | 2310010M24Rik | 1.902 | 1.982 | 1.72 | 2.012 | 2.165 | 2.002 |
| A_52_P132608 | NM_172685 | 229731 | Slc25a24 | 0.809 | 0.906 | 0.807 | 0.708 | 0.722 | 0.693 |
| A_51_P467505 | NM_013689 | 21682 | Tec | 1.077 | 1.062 | 1.153 | 0.842 | 0.861 | 0.881 |
| A_52_P153685 | AK129323; | 70422 | 2810417D08Rik | 1.763 | 1.676 | 1.845 | 2.204 | 2.099 | 2.085 |
| A_51_P174943 | NM_008485 | 16782 | Lamc2 | 1.282 | 1.255 | 1.249 | 1.068 | 1.04 | 1.004 |
| A_52_P301821 | NM_053078 | 27528 | D0H4S11 | 1.076 | 1.275 | 1.138 | 0.88 | 0.828 | 0.741 |
| A_52_P532910 | NM_024427 | 22003 | Tpm1 | 1.081 | 1.106 | 1.238 | 0.891 | 0.87 | 0.851 |
| A_51_P521084 | NM_025626 | 66540 | 3110001A13Rik | 1.423 | 1.425 | 1.474 | 0.832 | 0.865 | 0.776 |
| A_52_P680436 | NM_025793 | 66840 | Wdr45l | 1.338 | 1.213 | 1.346 | 0.755 | 0.702 | 0.805 |
| A_51_P164219 | NM_011909 | 24110 | Usp18 | 1.876 | 1.698 | 1.64 | 1.445 | 1.463 | 1.349 |
| A_52_P421234 | NM_133832 | 98711 | Rdh10 | 1.706 | 1.519 | 1.676 | 0.845 | 0.856 | 0.776 |
| A_52_P586157 | NM_022309 | 12400 | Cbfb | 1.417 | 1.551 | 1.338 | 0.773 | 0.863 | 0.903 |
| A_51_P262171 | NM_008326 | 15944 | Ifi1 | 1.271 | 1.011 | 1.135 | 1.457 | 1.571 | 1.485 |
| A_52_P452689 | NM_007498 | 11910 | Atf3 | 0.672 | 0.61 | 0.659 | 0.89 | 0.955 | 0.943 |
| A_52_P295933 | AK045060; | 14051 | B130023L16Rik | 1.637 | 1.973 | 2.08 | 1.007 | 0.993 | 1.019 |
| A_51_P222773 | NM_010446 | 15376 | Foxa2 | 2.333 | 1.693 | 1.64 | 6.867 | 6.289 | 6.137 |
| A_52_P361534 | NM_009521 | 22415 | Wnt3 | 1.193 | 0.891 | 0.969 | 2.247 | 2.459 | 2.231 |
| A_51_P507880 | NM_028980 | 74521 | 8430415E04Rik | 2.104 | 2.169 | 2.081 | 2.44 | 2.499 | 2.391 |
| A_52_P574668 | NM_011851 | 23959 | Nt5e | 1.611 | 1.127 | 1.284 | 1.764 | 1.707 | 1.826 |
| A_52_P957500 | XM_622354 | | | 0.371 | 0.532 | 0.397 | 1.528 | 1.372 | 1.305 |
| A_52_P345626 | AY149175; | 386626 | Mzf6d | 0.699 | 0.636 | 0.76 | 1.173 | 1.052 | 1.267 |
| A_52_P84015 | AK037762; | 11863 | | 0.695 | 0.924 | 0.842 | 2.965 | 3.344 | 3.023 |
| A_52_P432464 | AK053863; | 192196 | Luc7l2 | 0.7 | 0.863 | 1.032 | 1.457 | 1.269 | 1.582 |
| A_52_P376502 | BC025011; | 227541 | Camk1d | 0.664 | 0.71 | 0.737 | 0.973 | 0.893 | 1.008 |
| A_52_P354785 | AK037929; | 14852 | Gspt1 | 0.487 | 0.598 | 0.585 | 1.587 | 1.447 | 1.483 |
| A_51_P398766 | NM_010259 | 14468 | Gbp1 | 3.558 | 2.476 | 3.155 | 4.099 | 3.953 | 3.971 |
| A_52_P486234 | AK033497; | 22680 | | 0.658 | 0.943 | 0.756 | 2.133 | 2.185 | 1.908 |
| A_52_P628212 | AK044339; | 26380 | | 1.058 | 1.337 | 0.967 | 4.002 | 3.276 | 3.435 |
| A_52_P445799 | AK046777; | 52490 | D18Ertd289e | 2.088 | 2.22 | 2.899 | 2.952 | 2.963 | 3.531 |
| A_51_P268167 | BC037232;XM_621365 | 229363; 218850 | Gmps | 0.465 | 0.448 | 0.523 | 1.267 | 1.053 | 1.205 |
| A_51_P153079 | NM_133923 | 101100 | 4833441J24Rik | 0.42 | 0.232 | 0.278 | 1.153 | 1.158 | 1.047 |
| A_52_P584874 | NM_152895 | 75605 | Jarid1b | 0.737 | 0.83 | 0.752 | 1.242 | 1.251 | 1.242 |
| A_52_P307801 | NM_007847 | 13222 | Defcr-rs2 | 2.149 | 1.602 | 1.28 | 2.942 | 3.1 | 2.825 |
| A_52_P163148 | AB010370; | 544945 | | 0.704 | 0.687 | 0.76 | 1.098 | 1.144 | 1.325 |
| A_51_P171728 | NM_011926 | 26365 | Ceacam1 | 0.474 | 0.703 | 0.686 | 0.748 | 0.952 | 0.943 |
| A_51_P141660 | AK053665;XM_358382 | 67776; 235380 | Loh11cr2a | 0.43 | 0.62 | 0.908 | 1.844 | 1.422 | 1.976 |
| A_52_P532323 | NM_178410 | 218518 | Marveld2 | 1.824 | 2.102 | 1.904 | 0.871 | 0.884 | 1.027 |
| A_51_P337125 | NM_010566 | 16331 | Inpp5d | 0.938 | 0.873 | 1.036 | 1.261 | 1.272 | 1.297 |
| A_52_P336259 | AK087367;XM_485576 | 319467 | A930002C04Rik | 0.552 | 0.507 | 0.623 | 1.053 | 0.913 | 0.8 |
| A_51_P318577 | AK010729; | 73671 | 2410078J06Rik | 0.605 | 0.863 | 0.641 | 0.897 | 1.028 | 1.15 |
| A_52_P324934 | AK045391; | 16403 | | 0.758 | 0.573 | 0.774 | 1.808 | 1.513 | 1.534 |
| A_52_P826666 | AK007019; | 51799 | Rap2ip | 0.894 | 1.131 | 1.14 | 2.913 | 2.479 | 3.106 |
| A_51_P200223 | XM_619497 | | | 0.65 | 0.646 | 0.76 | 1.392 | 1.367 | 1.332 |
| A_51_P228472 | NM_008343 | 16009 | Igfbp3 | 1.472 | 2.26 | 1.637 | 1.02 | 1.11 | 1.239 |
| A_52_P206573 | NM_027427 | 70439 | Taf15 | 0.525 | 0.7 | 0.806 | 1.685 | 1.521 | 1.76 |
| A_52_P354363 | NM_026574 | 68142 | | 0.545 | 0.546 | 0.579 | 1.246 | 1.113 | 1.072 |
| A_52_P651469 | NM_001012726 | 503692 | | 1.087 | 2.041 | 1.585 | 2.114 | 1.954 | 2.198 |
| A_51_P463789 | BC051947; | 58212 | | 0.924 | 0.962 | 0.995 | 1.518 | 1.581 | 1.46 |
| A_52_P18887 | NM_011468 | 20755 | Sprr2a | 7.008 | 6.235 | 5.038 | 6.207 | 6.689 | 6.331 |
| A_51_P223132 | NM_031183;XM_486227 | 83395; 70549 | Sp6 | 0.649 | 0.542 | 0.552 | 1.095 | 0.998 | 1.125 |
| A_52_P223083 | BC038323; | 72096 | 2010208K18Rik | 0.89 | 0.64 | 1.018 | 2.024 | 1.641 | 2.309 |
| A_52_P523399 | NM_029444 | 75801 | 4930447C04Rik | 1.167 | 0.873 | 1.364 | 1.373 | 1.087 | 1.771 |
| A_52_P282000 | AK020248; | 13800 | Enah | 0.368 | 0.482 | 0.376 | 1.791 | 1.63 | 1.536 |
| A_52_P152983 | AK011739; | 26886 | Cenph | 0.423 | 0.805 | 0.28 | 1.393 | 1.582 | 1.833 |
| A_52_P26416 | AF106279; | 16782 | | 1.436 | 1.802 | 1.779 | 0.723 | 0.806 | 0.697 |
| A_51_P102081 | NM_007847 | 13222 | Defcr-rs2 | 2.408 | 2.238 | 1.498 | 3.38 | 3.255 | 2.763 |
| A_51_P285725 | NM_009309 | 20997 | T | 2.672 | 1.6 | 1.512 | 3.129 | 3.214 | 3.267 |
| A_52_P827911 | AK081941; | | | 0.209 | 0.476 | 0.487 | 1.73 | 1.566 | 1.778 |
| A_51_P231341 | AK087764; | 108160 | D0HXS9928E | 0.131 | 0.284 | 0.334 | 1.584 | 1 | 1.654 |
| A_51_P334093 | NM_026228 | 67547 | Slc39a8 | 2.173 | 2.638 | 2.466 | 1.07 | 1.006 | 1.061 |
| A_52_P196568 | NM_144513 | 17263 | Gtl2 | 0.0697 | 0.0369 | 0.0417 | 0.112 | 0.08 | 0.115 |
| A_52_P1108139 | AK053079; | | | 0.169 | 0.348 | 0.199 | 1.068 | 1.372 | 1.161 |
| A_52_P636559 | AK053008; | 268469 | 9530033F24Rik | 0.506 | 0.53 | 0.567 | 1.485 | 1.451 | 1.363 |
| A_52_P425734 | NM_145146 | 280662 | Afm | 1.466 | 1.63 | 2.105 | 1.902 | 1.789 | 2.093 |
| A_52_P1011737 | AK039096; | 106258 | Al790442 | 0.224 | 0.44 | 0.438 | 1.367 | 1.308 | 1.503 |
| A_52_P537140 | AK047235; | | | 0.566 | 0.565 | 0.531 | 1.029 | 1.127 | 1.156 |
| A_51_P191990 | AK087548; | 26563 | Ror1 | 0.378 | 0.368 | 0.389 | 1.197 | 1.149 | 1.176 |
| A_51_P478930 | AK080016; | | | 0.64 | 0.623 | 0.816 | 1.097 | 1.039 | 1.17 |
| A_51_P252565 | NM_009660 | 11687 | Alox15 | 1.618 | 1.425 | 1.194 | 1.366 | 1.436 | 1.457 |
| A_52_P81252 | NM_172599 | 218978 | D14Ertd436e | 0.265 | 0.408 | 0.488 | 1.153 | 1.046 | 0.927 |
| A_51_P487004 | NM_027732 | 71241 | Dmrtc2 | 1.918 | 2.991 | 1.94 | 1.77 | 1.537 | 1.901 |
| A_51_P104162 | AK053379; | 328232 | | 0.351 | 0.468 | 0.481 | 1.289 | 1.159 | 1.082 |
| A_52_P555603 | AK032770; | 11787 | Apbb2 | 0.273 | 0.355 | 0.304 | 1.021 | 1.021 | 1.054 |
| A_52_P819356 | AK041684; | | | 0.651 | 0.704 | 0.763 | 1.654 | 1.657 | 1.821 |
| A_52_P1196751 | AK086488; | | | 0.314 | 0.63 | 0.521 | 1.285 | 1.098 | 1.263 |
| A_52_P277415 | AK077313; | 76252 | 0610006O14Rik | 0.0165 | 0.0277 | 0.0223 | 0.0497 | 0.043 | 0.0552 |
| A_52_P812362 | BC016205; | 408064 | BC064078 | 1.424 | 1.508 | 1.444 | 1.323 | 1.468 | 1.262 |
| A_51_P352572 | NM_019701 | 56365 | Clcnkb | 1.441 | 1.565 | 1.422 | 1.707 | 1.362 | 1.314 |
| A_52_P100042 | AK045143; | 320938 | Tnpo3 | 0.301 | 0.507 | 0.406 | 1.444 | 1.417 | 1.439 |
| A_51_P103397 | AK078521; | 54561 | Nap1l3 | 0.942 | 0.996 | 1.004 | 1.047 | 0.978 | 1.076 |
| A_52_P296244 | BC048734; | | | 2.333 | 2.799 | 1.851 | 2.371 | 2.371 | 2.372 |
| A_51_P305019 | AK020601; | 78423 | 9530049O05Rik | 0.201 | 0.4 | 0.429 | 1.187 | 1.059 | 1.249 |
| A_52_P3304 | NM_080446 | 117599 | Helb | 1.004 | 1.239 | 1.059 | 1.614 | 1.432 | 1.418 |
| A_51_P147651 | AK041797;XM_146705 | 245902 | | 0.511 | 0.729 | 0.963 | 1.47 | 1.214 | 1.502 |
| A_51_P203955 | NM_010260 | 14469 | Gbp2 | 2.194 | 2.061 | 2.214 | 2.45 | 2.681 | 2.345 |
| A_51_P176474 | AK010466; | 76484 | 2410012C07Rik | 0.842 | 0.903 | 1.004 | 0.996 | 1.036 | 1.127 |
| A_52_P165497 | NM_011385 | 20481 | Ski | 0.541 | 0.665 | 0.649 | 1.004 | 0.817 | 0.972 |
| A_51_P358714 | AK031433; | | | 0.23 | 0.294 | 0.418 | 1.005 | 0.775 | 1.214 |
| A_51_P106918 | NM_009051 | 19715 | Rex2 | 1.032 | 1.019 | 0.654 | 1.31 | 1.285 | 1.541 |
| A_51_P124315 | NM_177649 | 227712 | BC034076 | 0.654 | 0.611 | 0.726 | 1.267 | 1.174 | 1.278 |
| A_52_P183368 | NM_011535 | 21386 | Tbx3 | 0.707 | 0.655 | 0.649 | 0.948 | 0.84 | 0.841 |
| A_51_P140751 | NM_011397 | 20522 | Slc23a1 | 0.522 | 0.618 | 0.556 | 1.152 | 1.255 | 1.186 |
| A_51_P103406 | NM_011708 | 22371 | Vwf | 0.798 | 1.058 | 1.093 | 1.007 | 0.932 | 1.019 |
| A_51_P381069 | XM_132045 | 231252 | Chrna9 | 0.803 | 0.789 | 0.615 | 1.017 | 1.052 | 0.983 |
| A_52_P75971 | NM_026599 | 68178 | Cgnl1 | 0.432 | 0.439 | 0.529 | 0.801 | 0.761 | 0.771 |
| A_52_P135873 | XM_132015;XM_488584 | 231128 | BC037112 | 0.389 | 0.408 | 0.392 | 1.158 | 1.118 | 1.166 |
| A_51_P165082 | AB041601;XM_489523 | 224019 | D16Bwg1494e | 1.656 | 1.924 | 1.93 | 1.376 | 1.394 | 1.295 |
| A_51_P294566 | AK020687; | 78083 | 9930116N10Rik | 0.689 | 1.237 | 0.848 | 1.59 | 1.475 | 1.545 |
| A_51_P378051 | NM_009675 | 11754 | Aoc3 | 1.41 | 1.106 | 1.668 | 0.9 | 0.88 | 1.25 |
| A_52_P420608 | NM_201366 | 381371 | Gm1631 | 1.252 | 0.926 | 0.759 | 1.014 | 1.175 | 1.159 |
| A_51_P221291 | NM_009229 | 20650 | Sntb2 | 0.614 | 0.612 | 0.631 | 1.259 | 1.1 | 1.157 |
| A_52_P362805 | AK044730; | 269254 | Als4 | 0.692 | 0.79 | 0.89 | 1.216 | 1.11 | 1.17 |
| A_51_P109649 | AK018142; | 17263 | Gtl2 | 0.11 | 0.17 | 0.136 | 0.167 | 0.167 | 0.195 |
| A_52_P413348 | XM_127674 | 75698 | 3110001 K24Rik | 0.593 | 0.709 | 0.685 | 1.434 | 1.352 | 1.356 |
| A_52_P400425 | NM_178656 | 193003 | A530088H08Rik | 0.241 | 0.469 | 0.547 | 0.79 | 0.835 | 1.024 |
| A_52_P147870 | NM_029963 | 77721 | Mrps5 | 0.574 | 0.622 | 0.64 | 1.321 | 1.377 | 1.297 |
| A_52_P57078 | AK052455; | | | 0.552 | 0.439 | 0.686 | 1.676 | 1.452 | 1.294 |
| A_52_P389874 | NM_011918 | 24131 | Ldb3 | 0.702 | 0.892 | 0.937 | 1.166 | 1.063 | 1.078 |
| A_52_P224125 | XM_622260 | | | 0.593 | 0.637 | 0.657 | 1.232 | 0.927 | 0.967 |
| A_52_P238468 | XM_133238 | 381853 | Gm160 | 0.673 | 0.817 | 0.882 | 1.027 | 1.116 | 1.106 |
| A_51_P253053 | XM_618867 | | | 0.601 | 1.748 | 0.724 | 1.594 | 1.644 | 2.037 |
| A_52_P500979 | NM_013628 | 18548 | Pcsk1 | 4.338 | 3.184 | 4.164 | 4.966 | 5.086 | 4.923 |
| A_51_P207934 | NM_199016 | 224794 | Enpp4 | 0.71 | 0.576 | 0.811 | 1.068 | 0.833 | 1.143 |
| A_52_P423384 | BC057551;XM_354546 | 104248 | Cabin1 | 0.481 | 0.424 | 0.586 | 1.189 | 1.05 | 1.408 |
| A_52_P560006 | NM_008426 | 16519 | Kcnj3 | 2.321 | 2.818 | 2.608 | 2.191 | 1.875 | 2.19 |
| A_52_P654624 | AK005333; | 236604 | 4933439C20Rik | 0.543 | 0.696 | 0.523 | 1.316 | 1.231 | 1.139 |
| A_52_P32502 | AK020822; | 77944 | A930007B11Rik | 1.212 | 1.019 | 1.21 | 2.052 | 1.291 | 1.824 |
| A_51_P302377 | NM_008009 | 14181 | Fgfbp1 | 1.542 | 1.782 | 1.567 | 1.002 | 1.052 | 1.132 |
| A_52_P255224 | AK032711;XM_486482 | 234371 | BC021367 | 0.812 | 0.712 | 0.548 | 2.288 | 1.957 | 2.287 |
| A_52_P571746 | XM_355533;XM_357399 | 230863 | LOC381571 | 1.076 | 1.21 | 1.362 | 2.199 | 2.23 | 2.231 |
| A_52_P458916 | AK016751; | 218850 | D14Abb1e | 0.488 | 0.694 | 0.807 | 1.15 | 1.185 | 1.257 |
| A_52_P286970 | XM_131162;XM_127565 | 76561 | Snx7 | 1.591 | 2.536 | 1.327 | 1.128 | 1.271 | 0.96 |
| A_51_P266644 | BC055753;XM_125538 | 140742 | | 1.51 | 1.548 | 1.621 | 1.064 | 0.96 | 1.069 |
| A_52_P233673 | NM_020594 | 57432 | Zc3hdc8 | 0.731 | 0.498 | 0.877 | 1.204 | 1.041 | 1.395 |
| A_52_P1084149 | AK051413; | 553138 | 9930021 J03Rik | 0.447 | 0.508 | 0.452 | 1.499 | 1.58 | 1.485 |
| A_52_P413661 | BC079861;XM_484162 | 217716 | Mlh3 | 0.513 | 0.589 | 0.64 | 1.73 | 1.608 | 1.611 |
| A_51_P404193 | NM_022435 | 64406 | Sp5 | 1.798 | 1.335 | 1.388 | 3.365 | 3.261 | 3.306 |
| A_52_P565940 | AF419220; | 18193 | Nsd1 | 0.487 | 0.593 | 0.616 | 1.236 | 1.181 | 1.08 |
| A_51_P514177 | NM_028838 | 74249 | Lrrc2 | 0.911 | 0.956 | 0.975 | 1.199 | 1.209 | 1.304 |
| A_52_P237357 | AF095690; | | | 0.454 | 0.422 | 0.442 | 1.34 | 1.372 | 1.159 |
| A_51_P240811 | NM_009290 | 20890 | Wnt8a | 4.437 | 4.432 | 3.479 | 4.373 | 5.655 | 4.468 |
| A_51_P236267 | NM_009183 | 20452 | St8sia4 | 1.305 | 1.382 | 1.289 | 1.168 | 1.234 | 1.322 |
| A_52_P405340 | U76762; | 14158 | Fert2 | 0.434 | 0.372 | 0.598 | 1.654 | 1.494 | 1.493 |
| A_52_P661144 | XM_485365 | | | 0.511 | 0.546 | 0.638 | 1.627 | 1.676 | 2.009 |
| A_52_P1044262 | AK051949; | | | 0.374 | 0.535 | 0.443 | 1.042 | 1.044 | 1.091 |
| A_52_P553100 | AK040413; | | | 0.696 | 0.886 | 0.727 | 2.086 | 2.022 | 1.86 |
| A_51_P358344 | NM_007732 | 12821 | Co117a1 | 0.283 | 0.661 | 0.876 | 1.118 | 0.984 | 1.246 |
| A_51_P321579 | XM_131865;XM_196334 | 433809 | LOC433809 | 0.495 | 0.534 | 0.598 | 1.155 | 1.087 | 1.237 |
| A_52_P485939 | NM_026228 | 67547 | Slc39a8 | 3.203 | 2.606 | 3.687 | 2.136 | 1.903 | 2.146 |
| A_52_P199922 | | | | 0.316 | 0.331 | 0.255 | 1.479 | 1.195 | 1.071 |
| A_51_P463791 | BC051947; | 58212 | | 0.648 | 0.879 | 1.013 | 1.447 | 1.472 | 1.498 |
| A_52_P487362 | NM_028980 | 74521 | 8430415E04Rik | 3.136 | 2.67 | 3.124 | 4.295 | 4.001 | 3.546 |
| A_51_P341137 | AK035176; | 224829 | Trerf1 | 0.563 | 0.731 | 0.649 | 1.51 | 1.436 | 1.382 |
| A_51_P407323 | NM_007976 | 14067 | F5 | 0.837 | 0.684 | 0.686 | 2.154 | 2.023 | 1.925 |
| A_51_P272363 | NM_134160 | 171166 | Mcoln3 | 2.423 | 3.074 | 2.25 | 1.242 | 1.432 | 0.843 |
| A_52_P430927 | AK042385; | 11908 | Atf1 | 0.409 | 0.436 | 0.428 | 1.263 | 1.186 | 1.26 |
| A_52_P626069 | AK040994;XM_284439 | 109151 | Chd9 | 0.449 | 0.512 | 0.687 | 1.302 | 1.102 | 1.65 |
| A_52_P355276 | NM_001002764 | 103677 | Al317223 | 0.704 | 0.605 | 0.784 | 1.531 | 1.432 | 1.606 |
| A_52_P650215 | NM_029278 | 75416 | 2610033H07Rik | 0.869 | 0.561 | 1.153 | 1.294 | 1.106 | 1.919 |
| A_52_P416123 | AK020480; | 77358 | 9430069107Rik | 0.584 | 0.644 | 1.001 | 1.681 | 1.367 | 1.728 |
| A_51_P189631 | NM_172427 | 76455 | 2310067E19Rik | 0.318 | 0.341 | 0.373 | 1.015 | 0.905 | 0.892 |
| A_52_P16973 | AK081934; | 320391 | D930017J03Rik | 0.56 | 0.604 | 0.612 | 1.894 | 1.918 | 1.856 |
| A_52_P947879 | AK048751; | | | 0.354 | 0.496 | 0.449 | 1.501 | 1.634 | 1.919 |
| A_52_P538515 | BC049181; | 226412 | R3hdm | 0.209 | 0.659 | 0.565 | 1.913 | 1.725 | 1 |
| A_52_P179250 | AK129127; | 230249 | AI314180 | 0.641 | 0.606 | 0.56 | 3.467 | 4.289 | 6.052 |
| A_52_P795628 | AK086352; | | | 0.551 | 1.049 | 0.818 | 3.259 | 4.511 | 4.2 |
| A_51_P144712 | NM_178930 | 107338 | Gbf1 | 0.424 | 0.476 | 0.425 | 1.192 | 1.19 | 1.292 |
| A_52_P655008 | AK044382; | 320678 | A930037G23Rik | 0.364 | 0.363 | 0.425 | 1.339 | 1.302 | 1.272 |
| A_52_P577072 | NM_011901 | 24074 | Taf7 | 0.654 | 0.591 | 0.759 | 1.655 | 1.407 | 1.87 |
| A_52_P636199 | NM_008017 | 14211 | Smc2I1 | 0.482 | 0.452 | 0.534 | 1.172 | 1.125 | 1.415 |
| A_51_P447189 | AK041326;XM_358090 | 385154 | | 0.755 | 0.808 | 0.675 | 2.409 | 2.277 | 2.233 |
| A_52_P1180315 | AK049088; | 74022 | 3930401K13Rik | 0.255 | 0.505 | 0.516 | 1.337 | 1.179 | 0.966 |
| A_52_P412167 | NM_080467 | 140494 | Atp6v0a4 | 0.553 | 1.045 | 0.878 | 1.777 | 2.017 | 2.418 |
| A_52_P335089 | BE198552; | | | 0.338 | 0.471 | 0.43 | 0.921 | 0.981 | 0.901 |
| A_52_P6621 | | | | 0.661 | 0.506 | 1.607 | 1.455 | 1.079 | 2.626 |
| A_51_P343129 | AK012079; | 70472 | Atad2 | 0.633 | 0.896 | 0.848 | 1.965 | 1.637 | 1.677 |
| A_51_P144417 | NM_181750 | 226412 | R3hdm | 0.745 | 0.757 | 0.789 | 2.029 | 1.993 | 2.191 |
| A_52_P350477 | NM_139295 | 193813 | Mcfd2 | 1.118 | 1.377 | 1.329 | 0.667 | 0.758 | 0.786 |
| A_51_P418884 | NM_001013789 | 432823 | | 0.496 | 0.536 | 0.695 | 1.716 | 1.402 | 1.593 |
| A_52_P581138 | NM_212450 | 329506 | D2Ertd485e | 0.457 | 0.451 | 0.472 | 1.47 | 1.244 | 1.239 |
| A_52_P434549 | NM_023190 | 56215 | Acin1 | 0.489 | 0.399 | 0.44 | 1.376 | 1.207 | 1.143 |
| A_52_P384392 | AK080827; | 328087 | D930001 B02 | 0.533 | 0.516 | 0.471 | 1.258 | 1.062 | 1.099 |
| A_51_P270725 | AK089493; | 102093 | Phkb | 0.756 | 0.923 | 0.755 | 2.239 | 1.725 | 2.287 |
| A_51_P193019 | NM_011157 | 19073 | Prg1 | 0.263 | 0.419 | 0.589 | 1.602 | 0.893 | 0.295 |
| A_52_P1172548 | AK084146; | 13204 | Dhx15 | 0.655 | 0.839 | 0.889 | 2.469 | 2.249 | 2.786 |
| A_51_P114606 | NM_016965 | 50884 | Nckap1 | 1.512 | 1.408 | 1.677 | 0.736 | 0.694 | 0.805 |
| A_52_P306387 | NM_178709 | 235315 | D130054N24Rik | 0.574 | 0.504 | 0.652 | 1.816 | 1.515 | 1.965 |
| A_52_P287576 | AK036308; | | | 0.284 | 0.537 | 0.429 | 1.515 | 1.516 | 1.834 |
| A_52_P501767 | AK012272; | 20637 | Snrp70 | 0.263 | 0.243 | 0.263 | 1.047 | 1 | 0.926 |
| A_52_P995717 | AK045222; | | | 0.672 | 0.74 | 0.709 | 2.392 | 1.854 | 2.412 |

**Table 3A - 200 Gene Subset (Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 0.266 | 0.299 | 0.281 | 0.396 | 0.368 | 0.407 |
| A_51_P335460 | NM_009132 | 20259 | Scin | 0.718 | 0.631 | 0.719 | 0.678 | 0.674 | 0.666 |
| A_52_P391505 | NM_144513 | 17263 | Gtl2 | 1.36 | 1.291 | 1.327 | 1.053 | 1.04 | 0.852 |
| A_51_P497100 | NM_010706 | 16855 | Lgals4 | 2.115 | 2.049 | 2.368 | 1.716 | 1.861 | 1.527 |
| A_51_P378371 | | | | 0.454 | 0.542 | 0.39 | 0.468 | 0.484 | 0.468 |
| A_51_P298266 | NM_025681 | 66643 | Lix1 | 0.507 | 0.414 | 0.416 | 0.554 | 0.584 | 0.54 |
| A_52_P269158 | NM_001003948 | 98496 | AL024069 | 0.316 | 0.33 | 0.33 | 0.388 | 0.393 | 0.443 |
| A_51_P311038 | BC009666;XM_134720 | 57267 | Apba3 | 0.334 | 0.341 | 0.339 | 0.446 | 0.472 | 0.482 |
| A_51_P457187 | NM_028075 | 72049 | Tnfrsf13c | 2.82 | 3.275 | 3.026 | 2.654 | 3.432 | 2.546 |
| A_52_P68702 | NM_145148 | 232288 | Frmd4b | 0.545 | 0.476 | 0.538 | 0.501 | 0.43 | 0.424 |
| A_52_P293443 | AK087208; | 13819 | | 1.278 | 1.075 | 1.13 | 1.496 | 1.604 | 1.349 |
| A_51_P157986 | NM_029930 | 77574 | 3321401 G04Rik | 0.426 | 0.339 | 0.345 | 0.368 | 0.318 | 0.37 |
| A_52_P1013441 | | | | 3.248 | 3.164 | 3.191 | 4.558 | 5.61 | 4.683 |
| A_51_P418375 | NM_023844 | 67374 | . Jam2 | 1.691 | 1.467 | 1.625 | 2.142 | 2.132 | 2.288 |
| A_52_P557940 | AK004302; | 66704 | 4921506122Rik | 2.114 | 1.863 | 2.221 | 2.17 | 2.013 | 2.051 |
| A_51_P480311 | NM_010168 | 14061 | F2 | 1.991 | 1.809 | 1.969 | 2.465 | 2.4 | 1.874 |
| A_52_P200458 | NM_010242 | 14345 | Fut4 | 0.429 | 0.343 | 0.355 | 0.453 | 0.391 | 0.512 |
| A_51_P463846 | NM_145545 | 229900 | 9830147J24Rik | 0.694 | 0.519 | 0.59 | 0.86 | 0.816 | 0.817 |
| A_51_P162474 | AF285577; | 107815 | Scml2 | 0.539 | 0.413 | 0.405 | 0.643 | 0.645 | 0.71 |
| A_51_P165244 | NM_018734 | 55932 | Gbp4 | 0.585 | 0.512 | 0.474 | 1.008 | 0.981 | 1.006 |
| A_52_P59318 | NM_133832 | 98711 | Rdh10 | 0.306 | 0.273 | 0.275 | 0.373 | 0.395 | 0.445 |
| A_52_P438036 | NM_175271 | 78134 | Gpr23 | 0.376 | 0.334 | 0.36 | 0.455 | 0.441 | 0.489 |
| A_52_P474242 | BE457768; | | | 0.499 | 0.53 | 0.46 | 0.69 | 0.658 | 0.726 |
| A_51_P499854 | NM_010284 | 14600 | Ghr | 0.407 | 0.375 | 0.412 | 0.413 | 0.429 | 0.489 |
| A_51_P455647 | NM_009801 | 12349 | Car2 | 0.335 | 0.288 | 0.301 | 0.568 | 0.516 | 0.638 |
| A_52_P103929 | NM_009306 | 20979 | Syt1 | 0.382 | 0.356 | 0.39 | 0.419 | 0.498 | 0.423 |
| A_51_P149469 | NM_009608 | 11464 | Actc1 | 0.324 | 0.387 | 0.384 | 0.496 | 0.551 | 0.471 |
| A_52_P557293 | NM_015775 | 50528 | Tmprss2 | 0.277 | 0.205 | 0.179 | 0.45 | 0.457 | 0.355 |
| A_52_P50496 | AY048587;XM_619453 | 114640 | Tifp39 | 0.454 | 0.501 | 0.422 | 0.661 | 0.638 | 0.694 |
| A_51_P180413 | NM_176930 | 319504 | C130076007Rik | 0.359 | 0.393 | 0.415 | 0.471 | 0.514 | 0.494 |
| A_51_P229613 | NM_008792 | 18549 | Pcsk2 | 0.512 | 0.483 | 0.515 | 0.58 | 0.58 | 0.504 |
| A_51_P121635 | NM_030700 | 80884 | Maged2 | 0.378 | 0.371 | 0.409 | 0.492 | 0.475 | 0.481 |
| A_51_P481777 | NM_018756 | 54382 | Tcstv1 | 1.532 | 1.822 | 1.792 | 2.619 | 3.155 | 2.956 |
| A_51_P412817 | NM_177617 | 216851 | D330014H01Rik | 3.151 | 3.039 | 3.326 | 2.136 | 2.194 | 1.865 |
| A_51_P387528 | AK019664; | 78801 | Ak7 | 2.693 | 2.814 | 2.57 | 3.786 | 3.834 | 3.71 |
| A_52_P78168 | AK031518; | | | 2.108 | 1.677 | 1.617 | 1.783 | 1.791 | 1.711 |
| A_52_P274960 | NM_001005508 | 226652 | 6030405P05Rik | 1.83 | 1.82 | 1.948 | 2.07 | 2.181 | 2.076 |
| A_52_P1188270 | AK042362; | | | 3.076 | 1.828 | 1.917 | 2.932 | 3.046 | 2.87 |
| A_51_P291227 | NM_001005508 | 226652 | 6030405P05Rik | 1.876 | 1.826 | 1.86 | 2.054 | 2.185 | 2.004 |
| A_52_P377299 | AK048360; | 271377 | Zbtb11 | 1.927 | 1.892 | 1.923 | 1.678 | 1.79 | 1.546 |
| A_51_P297131 | NM_009502 | 22330 | Vcl | 0.402 | 0.372 | 0.378 | 0.411 | 0.377 | 0.401 |
| A_52_P804224 | | | | 1.522 | 1.688 | 1.828 | 2.31 | 2.487 | 2.241 |
| A_51_P442894 | AK048310; | 621603 | C130048D07Rik | 2.632 | 2.364 | 2.281 | 3.657 | 3.714 | 3.102 |
| A_52_P309718 | AK173099; | 218850 | D14Abb1e | 2.324 | 2.579 | 2.18 | 2.614 | 2.72 | 2.711 |
| A_52_P359010 | AK129478; | 72193 | Sfrs2ip | 2.152 | 2.149 | 2.122 | 2.349 | 2.38 | 2.318 |
| A_51_P410813 | AK079732; | | | 2.732 | 2.716 | 2.559 | 2.898 | 2.605 | 2.3 |
| A_52_P382565 | XM_483943 | 432493 | LOC432493 | 0.976 | 1.222 | 1.221 | 1.492 | 1.439 | 1.198 |
| A_51_P333253 | NM_178440 | 246177 | Myo1g | 1.419 | 1.413 | 1.621 | 1.544 | 1.504 | 1.394 |
| A_52_P113190 | NM_053214 | 17916 | Myo1f | 1.428 | 1.513 | 1.396 | 1.988 | 1.959 | 2.083 |
| A_51_P473953 | XM_358600;XM_355454 | 381498 | 4930592C13Rik | 0.42 | 0.306 | 0.298 | 0.365 | 0.365 | 0.38 |
| A_52_P519870 | AB010340; | | | 2.391 | 3.34 | 2.621 | 3.76 | 3.72 | 1.955 |
| A_51_P266403 | AK009163; | 76730 | 2310005C01Rik | 3.079 | 3.053 | 3.092 | 2.733 | 2.967 | 2.734 |
| A_52_P3698 | NM_001015099 | 217558 | | 1.885 | 1.84 | 1.762 | 2.392 | 2.296 | 2.486 |
| A_52_P581100 | NM_027285 | 70005 | 1700029101Rik | 2.295 | 3.266 | 2.708 | 3.168 | 3.146 | 2.879 |
| A_51_P276305 | NM_009292 | 20899 | Stra8 | 3.137 | 3.069 | 3.174 | 2.824 | 2.972 | 2.777 |
| A_51_P455997 | Y13832; | 17263 | Gtl2 | 1.505 | 1.455 | 1.44 | 0.998 | 1.082 | 0.998 |
| A_52_P656336 | NM_023733 | 74114 | Crot | 0.407 | 0.351 | 0.381 | 0.755 | 0.647 | 0.8 |
| A_51_P512172 | AK010953;XM_125673 | 52463 | Cxxc6 | 2.864 | 2.384 | 2.216 | 2.184 | 2.11 | 2.068 |
| A_51_P282227 | NM_020577 | 57344 | As3mt | 2.651 | 2.607 | 2.876 | 3.224 | 3.327 | 3.147 |
| A_52_P386544 | NM_026594 | 68172 | 4930517K11 Rik | 2.843 | 2.618 | 2.802 | 2.673 | 2.913 | 2.894 |
| A 51_P125679 | AK010725;XM_134948 | 73673 | 2410076121Rik | 2.867 | 2.332 | 2.464 | 3.518 | 3.661 | 3.727 |
| A_51_P164504 | NM_007469 | 11812 | Apoc1 | 3.245 | 2.908 | 3.052 | 2.863 | 3.059 | 2.591 |
| A_52_P240706 | NM_007543 | 26367 | Ceacam2 | 2.285 | 1.866 | 1.892 | 2.654 | 2.556 | 2.628 |
| A_52_P420369 | NM_029444 | 75801 | 4930447C04Rik | 3.256 | 2.433 | 2.971 | 2.042 | 2.189 | 2.206 |
| A_51_P242399 | NM_031170 | 16691 | Krt2-8 | 0.193 | 0.233 | 0.208 | 0.403 | 0.4 | 0.39 |
| A_51_P158037 | NM_013505 | 13506 | Dsc2 | 0.271 | 0.249 | 0.258 | 0.437 | 0.415 | 0.453 |
| A_52_P258116 | NM_009521 | 22415 | Wnt3 | 0.156 | 0.164 | 0.16 | 0.312 | 0.309 | 0.296 |
| A_52_P286166 | NM_001002927 | 18619 | Penk1 | 0.497 | 0.324 | 0.339 | 0.671 | 0.658 | 0.647 |
| A_52_P399095 | NM_177793 | 327747 | 9030224M15Rik | 0.366 | 0.325 | 0.163 | 0.433 | 0.401 | 0.466 |
| A_52_P470663 | | | | 0.377 | 0.334 | 0.403 | 0.467 | 0.439 | 0.566 |
| A_52_P376169 | NM_177139 | 320343 | E130115E03Rik | 0.314 | 0.285 | 0.295 | 0.539 | 0.508 | 0.528 |
| A_51_P512085 | NM_130449 | 140792 | Colec12 | 0.335 | 0.364 | 0.296 | 0.382 | 0.368 | 0.385 |
| A_52_P676403 | NM_019494 | 56066 | Cxcl11 | 0.525 | 0.596 | 0.597 | 0.761 | 0.809 | 0.704 |
| A_51_P354307 | NM_172469 | 209195 | Clic6 | 0.43 | 0.319 | 0.329 | 0.398 | 0.398 | 0.387 |
| A_52_P569375 | NM_010203 | 14176 | Fgf5 | 0.776 | 0.678 | 0.554 | 0.45 | 0.467 | 0.574 |
| A_51_P162481 | NM_133194 | 107815 | Scml2 | 0.432 | 0.357 | 0.385 | 0.594 | 0.55 | 0.618 |
| A_51_P202911 | NM_144882 | 67198 | 2810022L02Rik | 0.297 | 0.283 | 0.328 | 0.346 | 0.333 | 0.365 |
| A_51_P418029 | NM_175541 | 245631 | Mum1I1 | 0.319 | 0.265 | 0.278 | 0.419 | 0.397 | 0.534 |
| A_52_P93837 | NM_008604 | 17380 | Mme | 0.559 | 0.439 | 0.513 | 0.653 | 0.573 | 0.747 |
| A_51_P275435 | NM_172411 | 71874 | 2310007B03Rik | 0.368 | 0.492 | 0.206 | 0.875 | 0.83 | 0.857 |
| A_51_P199888 | NM_026082 | 67299 | Dock7 | 0.407 | 0.403 | 0.245 | 0.558 | 0.428 | 0.546 |
| A_52_P211956 | NM_025658 | 66607 | Ms4a4d | 0.61 | 0.455 | 0.457 | 0.534 | 0.519 | 0.685 |
| A_51_P485985 | NM_134163 | 171170 | Mbnl3 | 0.223 | 0.181 | 0.161 | 0.625 | 0.543 | 0.697 |
| A_52_P630964 | NM_027533 | 70747 | Tspan2 | 0.361 | 0.283 | 0.324 | 0.499 | 0.473 | 0.525 |
| A_51_P294505 | NM_025658 | 66607 | Ms4a4d | 0.63 | 0.414 | 0.365 | 0.532 | 0.521 | 0.629 |
| A_52_P82488 | NM_021476 | 58861 | CysItr1 | 0.319 | 0.379 | 0.306 | 0.5 | 0.438 | 0.534 |
| A_52_P634829 | NM_023835 | 76681 | Trim12 | 0.59 | 0.561 | 0.672 | 1.012 | 0.996 | 0.866 |
| A_52_P460882 | XM_130319;XM_130308 | 73668; 14725 | Ttc21 b | 0.412 | 0.637 | 0.533 | 0.396 | 0.633 | 0.502 |
| A_52_P197826 | NM_010397 | 15039 | H2-T22 | 0.541 | 0.505 | 0.41 | 0.653 | 0.634 | 0.622 |
| A_51_P240253 | NM_019662 | 56437 | Rrad | 0.316 | 0.322 | 0.314 | 0.392 | 0.337 | 0.342 |
| A_51_P101975 | NM_010205 | 14179 | Fgf8 | 0.412 | 0.39 | 0.413 | 0.415 | 0.454 | 0.385 |
| A_52_P21595 | NM_028238 | 72433 | Rab38 | 0.234 | 0.188 | 0.168 | 0.439 | 0.467 | 0.472 |
| A_52_P402786 | NM_008935 | 19126 | Prom1 | 0.37 | 0.396 | 0.335 | 0.551 | 0.506 | 0.594 |
| A_52_P126158 | NM_008326 | 15944 | Ifi1 | 0.237 | 0.208 | 0.22 | 0.415 | 0.412 | 0.425 |
| A_52_P676510 | NM_011579 | 21822 | Tgtp | 0.225 | 0.162 | 0.155 | 0.382 | 0.378 | 0.362 |
| A_52_P175685 | NM_008390 | 16362 | Irf1 | 0.401 | 0.37 | 0.3 | 0.663 | 0.637 | 0.749 |
| A_52_P384718 | BC052056;XM_283264 | 320873 | | 0.508 | 0.441 | 0.458 | 0.541 | 0.512 | 0.578 |
| A_52_P176333 | NM_001005787 | 12695 | Cipp | 0.372 | 0.357 | 0.365 | 0.452 | 0.392 | 0.42 |
| A_52_P358951 | AK081999;XM_134736 | 271457; 22688 | Rab5a | 0.417 | 0.378 | 0.448 | 0.464 | 0.449 | 0.46 |
| A_51_P168646 | AK039580; | | | 0.442 | 0.434 | 0.509 | 0.537 | 0.548 | 0.598 |
| A_52_P620345 | NM_133979 | 102566 | AI604832 | 0.368 | 0.402 | 0.382 | 0.441 | 0.438 | 0.493 |
| A_52_P253179 | AK077477; | 16009 | Igfbp3 | 0.397 | 0.511 | 0.257 | 0.517 | 0.468 | 0.482 |
| A_51_P397003 | XM_133990 | 68904 | 1110065L07Rik | 0.45 | 0.438 | 0.438 | 0.542 | 0.479 | 0.613 |
| A_52_P74441 | NM_011570 | 21753 | Tes | 0.423 | 0.418 | 0.328 | 0.54 | 0.558 | 0.659 |
| A_52_P255250 | NM_009543 | 22644 | Rnf103 | 0.532 | 0.371 | 0.413 | 0.471 | 0.455 | 0.491 |
| A_52_P575505 | NM_008994 | 19302 | Pxmp3 | 0.305 | 0.306 | 0.317 | 0.463 | 0.445 | 0.525 |
| A_52_P469789 | NM_007588 | 12311 | Calcr | 0.48 | 0.383 | 0.445 | 0.625 | 0.707 | 0.696 |
| A_51_P345422 | NM_010747 | 17096 | Lyn | 0.244 | 0.247 | 0.256 | 0.373 | 0.392 | 0.424 |
| A_51_P356003 | NM_001007580 | 333564 | Gm784 | 0.494 | 0.445 | 0.513 | 0.509 | 0.483 | 0.534 |
| A_52_P680941 | NM_010284 | 14600 | Ghr | 0.389 | 0.396 | 0.371 | 0.448 | 0.445 | 0.488 |
| A_52_P380263 | NM_013723 | 27205 | Podxl | 0.395 | 0.422 | 0.286 | 0.532 | 0.511 | 0.504 |
| A_52_P286528 | BC055771; | 102954 | Nudt11 | 0.411 | 0.273 | 0.263 | 0.433 | 0.371 | 0.517 |
| A_51_P105068 | NM_027990 | 71897 | 2310010M24Rik | 0.459 | 0.371 | 0.462 | 0.48 | 0.484 | 0.498 |
| A_52_P132608 | NM_172685 | 229731 | Slc25a24 | 0.358 | 0.291 | 0.25 | 0.504 | 0.401 | 0.432 |
| A_51_P467505 | NM_013689 | 21682 | Tec | 0.28 | 0.242 | 0.227 | 0.502 | 0.464 | 0.499 |
| A_52_P153685 | AK129323; | 70422 | 2810417D08Rik | 0.452 | 0.397 | 0.407 | 0.587 | 0.526 | 0.735 |
| A_51_P174943 | NM_008485 | 16782 | Lamc2 | 0.234 | 0.255 | 0.286 | 0.433 | 0.411 | 0.451 |
| A_52_P301821 | NM_053078 | 27528 | D0H4S114 | 0.442 | 0.427 | 0.469 | 0.472 | 0.525 | 0.486 |
| A_52_P532910 | NM_024427 | 22003 | Tpm1 | 0.358 | 0.392 | 0.35 | 0.386 | 0.39 | 0.433 |
| A_51_P521084 | NM_025626 | 66540 | 3110001A13Rik | 0.408 | 0.406 | 0.391 | 0.474 | 0.456 | 0.494 |
| A_52_P680436 | NM_025793 | 66840 | Wdr45l | 0.344 | 0.369 | 0.331 | 0.651 | 0.557 | 0.675 |
| A_51_P164219 | NM_011909 | 24110 | Usp18 | 0.435 | 0.307 | 0.303 | 0.435 | 0.448 | 0.457 |
| A_52_P421234 | NM_133832 | 98711 | Rdh10 | 0.356 | 0.309 | 0.338 | 0.406 | 0.4 | 0.427 |
| A_52_P586157 | NM_022309 | 12400 | Cbfb | 0.481 | 0.354 | 0.384 | 0.606 | 0.523 | 0.75 |
| A_51_P262171 | NM_008326 | 15944 | Ifi1 | 0.29 | 0.264 | 0.261 | 0.474 | 0.475 | 0.507 |
| A_52_P452689 | NM_007498 | 11910 | Atf3 | 0.291 | 0.326 | 0.293 | 0.307 | 0.321 | 0.307 |
| A_52_P295933 | AK045060; | 14051 | B130023L16Rik | 0.336 | 0.293 | 0.594 | 0.466 | 0.5 | 0.466 |
| A_51_P222773 | NM_010446 | 15376 | Foxa2 | 0.423 | 0.314 | 0.4 | 0.447 | 0.809 | 0.489 |
| A_52_P361534 | NM_009521 | 22415 | Wnt3 | 0.0992 | 0.0782 | 0.0774 | 0.211 | 0.198 | 0.243 |
| A_51_P507880 | NM_028980 | 74521 | 8430415E04Rik | 0.223 | 0.221 | 0.235 | 0.35 | 0.326 | 0.332 |
| A_52_P574668 | NM_011851 | 23959 | Nt5e | 0.15 | 0.159 | 0.148 | 0.316 | 0.285 | 0.335 |
| A_52_P957500 | XM_622354 | | | 2.438 | 1.963 | 2.267 | 2.142 | 2.231 | 1.855 |
| A_52_P345626 | AY149175; | 386626 | Mzf6d | 2.677 | 3.059 | 3.002 | 3.203 | 2.748 | 3.757 |
| A_52_P84015 | AK037762; | 11863 | | 3.675 | 3.344 | 3.705 | 4.008 | 3.981 | 3.736 |
| A_52_P432464 | AK053863; | 192196 | Luc7l2 | 2.643 | 3.226 | 2.225 | 1.973 | 2.004 | 1.643 |
| A_52_P376502 | BC025011; | 227541 | Camk1d | 1.575 | 1.641 | 1.563 | 1.989 | 2.543 | 1.959 |
| A_52_P354785 | AK037929; | 14852 | Gspt1 | 2.114 | 1.965 | 1.835 | 1.904 | 1.907 | 2.055 |
| A_51_P398766 | NM_010259 | 14468 | Gbp1 | 0.374 | 0.372 | 0.361 | 0.894 | 0.876 | 0.949 |
| A_52_P486234 | AK033497; | 22680 | | 3.81 | 2.878 | 2.669 | 3.19 | 3.59 | 3.068 |
| A_52_P628212 | AK044339; | 26380 | | 9.353 | 5.604 | 7.112 | 6.273 | 6.769 | 7.237 |
| A_52_P445799 | AK046777; | 52490 | D18Ertd289e | 0.676 | 0.602 | 0.389 | 0.613 | 0.532 | 0.592 |
| A_51_P268167 | BC037232;XM_621365 | 229363; 218850 | Gmps | 2.665 | 2.898 | 2.35 | 2.187 | 2.366 | 2.198 |
| A_51_P153079 | NM_133923 | 101100 | 4833441J24RIk | 1.312 | 1.174 | 1.271 | 1.222 | 1.343 | 1.299 |
| A_52_P584874 | NM_152895 | 75605 | Jarid1b | 2.228 | 2.064 | 2.215 | 1.844 | 1.912 | 1.769 |
| A_52_P307801 | NM_007847 | 13222 | Defcr-rs2 | 0.119 | 0.091 | 0.0705 | 0.133 | 0.175 | 0.108 |
| A_52_P163148 | AB010370; | 544945 | | 2.883 | 2.86 | 2.64 | 3.58 | 3.156 | 3.085 |
| A_51_P171728 | NM_011926 | 26365 | Ceacam1 | 2.703 | 1.992 | 2.013 | 3.11 | 3.457 | 2.977 |
| A_51_P141660 | AK053665;XM_358382 | 67776; 235380 | Loh11cr2a | 2.996 | 3.339 | 3.08 | 2.433 | 2.601 | 2.365 |
| A_52_P532323 | NM_178410 | 218518 | Marveld2 | 0.399 | 0.326 | 0.264 | 0.434 | 0.363 | 0.431 |
| A_51_P337125 | NM_010566 | 16331 | Inpp5d | 3.162 | 2.936 | 3.033 | 4.399 | 4.495 | 4.161 |
| A_52_P336259 | AK087367;XM_485576 | 319467 | A930002C04Rik | 1.736 | 2.368 | 2.023 | 1.677 | 1.679 | 1.401 |
| A_51_P318577 | AK010729; | 73671 | 2410078J06Rik | 3.283 | 4.227 | 3.815 | 6.599 | 6.454 | 5.366 |
| A_52_P324934 | AK045391; | 16403 | | 1.983 | 2.443 | 1.872 | 2.691 | 2.881 | 2.226 |
| A_52_P826666 | AK007019; | 51799 | Rap2ip | 4.063 | 3.142 | 3.547 | 3.365 | 3.764 | 3.267 |
| A_51_P200223 | XM_619497 | | | 2.332 | 2.259 | 2.383 | 1.575 | 1.797 | 1.553 |
| A_51_P228472 | NM_008343 | 16009 | Igfbp3 | 0.288 | 0.22 | 0.276 | 0.283 | 0.322 | 0.364 |
| A_52_P206573 | NM_027427 | 70439 | Taf15 | 2.14 | 3.003 | 2.282 | 1.904 | 2.123 | 1.956 |
| A_52_P354363 | NM_026574 | 68142 | | 1.74 | 1.737 | 1.937 | 1.525 | 1.61 | 1.378 |
| A_52_P651469 | NM_001012726 | 503692 | | 5.11 | 4.303 | 5.085 | 5.606 | 6.363 | 4.012 |
| A_51_P463789 | BC051947; | 58212 | | 3.253 | 2.961 | 3.321 | 2.093 | 2.306 | 1.934 |
| A_52_P18887 | NM_011468 | 20755 | Sprr2a | 0.21 | 0.171 | 0.214 | 0.282 | 0.277 | 0.25 |
| A_51_P223132 | NM_031183;XM_486227 | 83395; 70549 | Sp6 | 2.329 | 2.088 | 2.196 | 1.457 | 1.639 | 1.36 |
| A_52_P223083 | BC038323; | 72096 | 2010208K18Rik | 4.486 | 6.15 | 3.745 | 3.784 | 3.918 | 3.378 |
| A_52_P523399 | NM_029444 | 75801 | 4930447C04Rik | 3.312 | 7.019 | 4.341 | 3.597 | 4.009 | 2.819 |
| A_52_P282000 | AK020248; | 13800 | Enah | 2.7 | 2.435 | 2.683 | 2.632 | 2.812 | 2.59 |
| A_52_P152983 | AK011739; | 26886 | Cenph | 2.134 | 3.107 | 2.171 | 2.114 | 2.408 | 2.327 |
| A_52_P26416 | AF106279; | 16782 | | 0.254 | 0.252 | 0.241 | 0.358 | 0.391 | 0.378 |
| A_51_P102081 | NM_007847 | 13222 | Defcr-rs2 | 0.061 | 0.0426 | 0.0644 | 0.0791 | 0.158 | 0.0864 |
| A_51_P285725 | NM_009309 | 20997 | T | 0.141 | 0.102 | 0.113 | 0.134 | 0.13 | 0.141 |
| A_52_P827911 | AK081941; | | | 2.387 | 2.657 | 2.47 | 2.075 | 2.44 | 2.116 |
| A_51_P231341 | AK087764; | 108160 | D0HXS9928E | 2.885 | 3.795 | 2.39 | 3.305 | 3.578 | 2.451 |
| A_51_P334093 | NM_026228 | 67547 | SIc39a8 | 0.208 | 0.0771 | 0.105 | 0.19 | 0.198 | 0.231 |
| A_52_P196568 | NM_144513 | 17263 | GtI2 | 1.892 | 2.538 | 1.516 | 1.938 | 1.959 | 1.652 |
| A_52_P1108139 | AK053079; | | | 1.801 | 2.214 | 1.538 | 1.924 | 2.168 | 1.724 |
| A_52_P636559 | AK053008; | 268469 | 9530033F24Rik | 2.63 | 2.537 | 2.752 | 2.132 | 2.334 | 1.998 |
| A_52_P425734 | NM_145146 | 280662 | Afm | 3.913 | 3.546 | 3.74 | 3.764 | 4.135 | 3.335 |
| A_52_P1011737 | AK039096; | 106258 | A1790442 | 1.846 | 1.977 | 1.978 | 2.087 | 2.67 | 2.065 |
| A_52_P537140 | AK047235; | | | 1.74 | 2.015 | 2.044 | 1.613 | 1.708 | 1.261 |
| A_51_P191990 | AK087548; | 26563 | Ror1 | 1.628 | 1.623 | 1.626 | 1.509 | 1.588 | 1.479 |
| A_51_P478930 | AK080016; | | | 2.436 | 2.701 | 2.337 | 2.779 | 2.919 | 2.466 |
| A_51_P252565 | NM_009660 | 11687 | Alox15 | 0.236 | 0.24 | 0.156 | 0.217 | 0.231 | 0.212 |
| A_52_P81252 | NM_172599 | 218978 | D14Ertd436e | 2.478 | 2.667 | 2.607 | 1.82 | 1.981 | 1.764 |
| A_51_P487004 | NM_027732 | 71241 | Dmrtc2 | 10.3 | 11.69 | 10.98 | 5.024 | 6.192 | 5.685 |
| A_51_P104162 | AK053379; | 328232 | | 2.537 | 3.096 | 3.391 | 3.126 | 2.998 | 3.127 |
| A_52_P555603 | AK032770; | 11787 | Apbb2 | 1.409 | 1.222 | 1.29 | 1.326 | 1.427 | 1.18 |
| A_52_P819356 | AK041684; | | | 3.003 | 2.848 | 2.7 | 3.026 | 2.915 | 3.006 |
| A_52_P1196751 | AK086488; | | | 2.217 | 2.193 | 2.198 | 2.38 | 2.677 | 2.156 |
| A_52_P277415 | AK077313; | 76252 | 0610006014Rik | 1.282 | 1.291 | 1.224 | 0.99 | 1.044 | 0.981 |
| A_52_P812362 | BC016205; | 408064 | BC064078 | 0.25 | 0.167 | 0.217 | 0.302 | 0.27 | 0.27 |
| A_51_P352572 | NM_019701 | 56365 | Clcnkb | 5.193 | 4.267 | 4.344 | 4.514 | 5.226 | 4.635 |
| A_52_P100042 | AK045143; | 320938 | Tnpo3 | 1.761 | 1.695 | 1.74 | 1.697 | 1.665 | 1.526 |
| A_51_P103397 | AK078521; | 54561 | Nap1I3 | 2.701 | 2.645 | 2.885 | 3.303 | 3.612 | 3.031 |
| A_52_P296244 | BC048734; | | | 0.369 | 0.373 | 0.292 | 0.457 | 0.42 | 0.426 |
| A_51_P305019 | AK020601; | 78423 | 9530049005Rik | 1.297 | 1.416 | 1.484 | 1.43 | 1.551 | 1.387 |
| A_52_P3304 | NM_080446 | 117599 | Helb | 2.823 | 2.774 | 3.121 | 2.206 | 2.73 | 2.462 |
| A_51_P147651 | AK041797;XM_146705 | 245902 | | 2.797 | 2.965 | 2.694 | 2.18 | 2.325 | 1.652 |
| A_51_P203955 | NM_010260 | 14469 | Gbp2 | 0.252 | 0.267 | 0.292 | 0.582 | 0.578 | 0.664 |
| A_51_P176474 | AK010466; | 76484 | 2410012C07Rik | 2.797 | 2.691 | 2.733 | 3.87 | 4.072 | 3.726 |
| A_52_P165497 | NM_011385 | 20481 | Ski | 2.312 | 2.37 | 2.474 | 1.296 | 1.485 | 1.406 |
| A_51_P358714 | AK031433; | | | 2.043 | 2.448 | 1.68 | 2.647 | 2.627 | 1.899 |

**Table 3B - 200 Gene Subset (Non Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 1.152 | 1.407 | 1.101 | 0.859 | 0.96 | 0.924 |
| A_51_P335460 | NM_009132 | 20259 | Scin | 1.658 | 1.913 | 1.519 | 1.101 | 1.225 | 1.107 |
| A_52_P391505 | NM_144513 | 17263 | Gtl2 | 0.0793 | 0.114 | 0.0867 | 0.158 | 0.15 | 0.148 |
| A_51_P497100 | NM_010706 | 16855 | Lgals4 | 0.692 | 0.771 | 0.849 | 1.002 | 0.908 | 0.828 |
| A_51_P378371 | | | | 0.986 | 1.006 | 1.208 | 1.217 | 1.156 | 1.234 |
| A_51_P298266 | NM_025681 | 66643 | Lix1 | 1.123 | 1.145 | 1.109 | 1.143 | 1.15 | 1.141 |
| A_52_P269158 | NM_001003948 | 98496 | AL024069 | 1.775 | 1.419 | 1.506 | 1.494 | 1.391 | 1.254 |
| A_51_P311038 | BC009666;XM_134720 | 57267 | Apba3 | 1.847 | 1.642 | 1.538 | 2.55 | 2.405 | 2.808 |
| A_51_P457187 | NM_028075 | 72049 | Tnfrsf13c | 1.083 | 1.58 | 1.373 | 0.916 | 1.099 | 1.091 |
| A_52_P68702 | NM_145148 | 232288 | Frmd4b | 0.928 | 1.071 | 0.877 | 1.242 | 1.234 | 1.139 |
| A_52_P293443 | AK087208; | 13819 | | 0.179 | 0.222 | 0.219 | 0.543 | 0.565 | 0.552 |
| A_51_P157986 | NM_029930 | 77574 | 3321401G04Rik | 0.909 | 1.196 | 1.088 | 0.563 | 0.621 | 0.598 |
| A_52_P1013441 | | | | 1.255 | 1.032 | 2.041 | 1.173 | 1.203 | 1.341 |
| A_51_P418375 | NM_023844 | 67374 | Jam2 | 1.044 | 0.943 | 0.99 | 0.826 | 0.786 | 0.84 |
| A_52_P557940 | AK004302; | 66704 | 4921506I22Rik | 0.544 | 0.595 | 0.569 | 1.371 | 1.379 | 1.241 |
| A_51_P480311 | NM_010168 | 14061 | F2 | 0.348 | 0.476 | 0.622 | 0.996 | 1.004 | 1.186 |
| A_52_P200458 | NM_010242 | 14345 | Fut4 | 1.003 | 0.997 | 1.017 | 1.108 | 1.203 | 1.09 |
| A_51_P463846 | NM_145545 | 229900 | 9830147J24Rik | 2.28 | 2.199 | 2.49 | 2.068 | 1.958 | 1.72 |
| A_51_P162474 | AF285577; | 107815 | ScmI2 | 2.885 | 2.549 | 2.371 | 3.021 | 3.116 | 3.1 |
| A_51_P165244 | NM_018734 | 55932 | Gbp4 | 2.388 | 1.319 | 2.543 | 2.404 | 2.051 | 2.534 |
| A_52_P59318 | NM_133832 | 98711 | Rdh10 | 1.689 | 1.611 | 1.584 | 0.876 | 1.002 | 0.955 |
| A_52_P438036 | NM_175271 | 78134 | Gpr23 | 1.082 | 0.96 | 0.968 | 1.634 | 1.593 | 1.618 |
| A_52_P474242 | BE457768; | | | 1.31 | 1.785 | 1.55 | 1.137 | 1.132 | 0.997 |
| A_51_P499854 | NM_010284 | 14600 | Ghr | 1.55 | 1.19 | 1.486 | 2.502 | 2.537 | 2.196 |
| A_51_P455647 | NM_009801 | 12349 | Car2 | 1.592 | 1.463 | 1.337 | 2.139 | 2.347 | 2.17 |
| A_52_P103929 | NM_009306 | 20979 | Syt1 | 0.898 | 0.938 | 0.967 | 1.626 | 1.464 | 1.422 |
| A_51_P149469 | NM_009608 | 11464 | Actc1 | 1.012 | 1.006 | 0.935 | 0.971 | 0.997 | 1.12 |
| A_52_P557293 | NM_015775 | 50528 | Tmprss2 | 1.142 | 1.091 | 1.111 I | 1.208 | 1.127 | 1.225 |
| A_52_P50496 | AY048587;XM_619453 | 114640 | Tifp39 | 1.196 | 1.551 | 1.504 | 1.114 | 1.171 | 1.214 |
| A_51_P180413 | NM_176930 | 319504 | C130076O07Rik | 1.603 | 1.54 | 1.395 | 1.297 | 1.25 | 1.155 |
| A_51_P229613 | NM_008792 | 18549 | Pcsk2 | 1.463 | 1.53 | 1.37 | 1.333 | 1.422 | 1.554 |
| A_51_P121635 | NM_030700 | 80884 | Maged2 | 1.225 | 1.426 | 1.335 | 0.918 | 0.99 | 0.88 |
| A_51_P481777 | NM_018756 | 54382 | Tcstv1 | 0.208 | 0.696 | 0.654 | 0.592 | 0.503 | 0.72 |
| A_51_P412817 | NM_177617 | 216851 | D330014H01Rik | 0.725 | 0.834 | 1.04 | 1.083 | 0.986 | 0.997 |
| A_51_P387528 | AK019664; | 78801 | Ak7 | 0.65 | 0.537 | 0.813 | 0.767 | 0.778 | 1.11 I |
| A_52_P78168 | AK031518; | | | 0.277 | 0.385 | 0.299 | 0.704 | 0.652 | 0.544 |
| A_52_P274960 | NM_001005508 | 226652 | 6030405P05Rik | 0.671 | 0.634 | 0.712 | 0.763 | 0.781 | 0.808 |
| A_52_P 1188270 | AK042362; | | | 0.341 | 0.594 | 0.564 | 1.758 | 1.462 | 1.658 |
| A_51_P291227 | NM_001005508 | 226652 | 6030405P05Rik | 0.705 | 0.681 | 0.731 | 0.814 | 0.735 | 0.798 |
| A_52_P377299 | AK048360; | 271377 | Zbtb11 | 0.577 | 0.582 | 0.645 | 1.152 | 1.092 | 1.023 |
| A_51_P297131 | NM_009502 | 22330 | Vcl | 1.015 | 1.436 | 0.955 | 0.556 | 0.672 | 0.587 |
| A_52_P804224 | | | | 0.299 | 0.314 | 0.291 | 1.263 | 1.2 | 1.068 |
| A_51_P442894 | AK048310; | 621603 | C130048D07Rik | 1.023 | 0.841 | 1.218 | 0.788 | 0.867 | 0.977 |
| A_52_P309718 | AK173099; | 218850 | D14Abb1e | 1.006 | 0.813 | 1.127 | 1.186 | 1.29 | 1.289 |
| A_52_P359010 | AK129478; | 72193 | Sfrs2ip | 0.54 | 0.706 | 0.574 | 1.484 | 1.398 | 1.403 |
| A_51_P410813 | AK079732; | | | 0.717 | 0.877 | 0.904 | 1.593 | 1.473 | 1.607 |
| A_52_P382565 | XM_483943 | 432493 | LOC432493 | 0.228 | 0.273 | 0.254 | 0.607 | 0.622 | 0.462 |
| A_51_P333253 | NM_178440 | 246177 | Myo1g | 0.535 | 0.491 | 0.538 | 0.474 | 0.481 | 0.37 |
| A_52_P113190 | NM_053214 | 17916 | Myo1f | 0.386 | 0.513 | 0.397 | 0.243 | 0.232 | 0.264 |
| A 51_P473953 | XM_358600;XM_355454 | 381498 | 4930592C13Rik | 2.138 | 1.593 | 1.947 | 2.323 | 2.226 | 2.26 |
| A_52_P519870 | AB010340; | | | 0.758 | 0.675 | 1.128 | 0.989 | 0.863 | 1.393 |
| A_51_P266403 | AK009163; | 76730 | 2310005C01Rik | 0.984 | 0.901 | 1.009 | 1.273 | 1.313 | 1.391 |
| A_52_P3698 | NM_001015099 | 217558 | | 0.658 | 0.653 | 0.676 | 1.487 | 1.564 | 1.537 |
| A_52_P581100 | NM_027285 | 70005 | 1700029101 Rik | 1.164 | 1.1 | 1.141 | 1.525 | 1.383 | 1.305 |
| A_51_P276305 | NM_009292 | 20899 | Stra8 | 0.873 | 0.934 | 0.982 | 0.431 | 0.426 | 0.471 |
| A_51_P455997 | Y13832; | 17263 | Gtl2 | 0.123 | 0.184 | 0.114 | 0.213 | 0.197 | 0.258 |
| A_52_P656336 | NM_023733 | 74114 | Crot | 1.233 | 1.547 | 1.442 | 0.983 | 1.067 | 0.998 |
| A_51_P512172 | AK010953;XM_125673 | 52463 | Cxxc6 | 0.8 | 0.986 | 0.748 | 1.033 | 1.014 | 1.046 |
| A_51_P282227 | NM_020577 | 57344 | As3mt | 0.79 | 0.761 | 0.777 | 1.02 | 0.91 | 0.98 |
| A_52_P386544 | NM_026594 | 68172 | 4930517K11 Rik | 0.74 | 0.606 | 0.729 | 1.188 | 1.159 | 1.351 |
| A_51_P125679 | AK010725;XM_134948 | 73673 | 2410076121Rik | 0.588 | 0.959 | 0.916 | 0.84 | 0.843 | 0.849 |
| A_51_P164504 | NM_007469 | 11812 | Apocl | 1.307 | 1.245 | 1.314 | 1.412 | 1.375 | 1.457 |
| A_52_P240706 | NM_007543 | 26367 | Ceacam2 | 0.87 | 0.778 | 0.892 | 0.689 | 0.756 | 0.859 |
| A_52_P420369 | NM_029444 | 75801 | 4930447C04Rik | 1.087 | 1.084 | 1.129 | 1.176 | 0.989 | 1.041 |
| A_51_P242399 | NM_031170 | 16691 | Krt2-8 | 1.013 | 1.199 | 0.939 | 0.962 | 0.977 | 0.946 |
| A_51_P158037 | NM_013505 | 13506 | Dsc2 | 1.238 | 1.562 | 1.304 | 0.743 | 0.819 | 0.875 |
| A_52_P258116 | NM_009521 | 22415 | Wnt3 | 1.304 | 1.249 | 1.083 | 1.811 | 1.943 | 2.006 |
| A_52_P286166 | NM_001002927 | 18619 | Penk1 | 1.649 | 2.509 | 2.439 | 1.542 | 1.453 | 1.374 |
| A_52_P399095 | NM_177793 | 327747 | 9030224M15Rik | 1.11 | 0.976 | 1.056 | 1.583 | 1.692 | 1.596 |
| A_52_P470663 | | | | 2.339 | 2.72 | 2.405 | 1.069 | 1.105 | 1.018 |
| A_52_P376169 | NM_177139 | 320343 | E130115E03Rik | 1.184 | 1.025 | 1.076 | 1.949 | 1.81 | 1.828 |
| A_51_P512085 | NM_130449 | 140792 | Colec12 | 1.574 | 1.25 | 1.471 | 1.463 | 1.651 | 1.693 |
| A_52_P676403 | NM_019494 | 56066 | Cxcl11 | 3.86 | 3.177 | 3.663 | 2.834 | 2.723 | 2.826 |
| A_51_P354307 | NM_172469 | 209195 | Clic6 | 1.31 | 1.578 | 1.57 | 0.719 | 0.748 | 0.659 |
| A_52_P569375 | NM_010203 | 14176 | Fgf5 | 4.338 | 3.206 | 3.958 | 5.889 | 5.927 | 6.75 |
| A_51_P162481 | NM_133194 | 107815 | Scml2 | 2.434 | 2.616 | 2.232 | 2.312 | 2.394 | 2.596 |
| A_51_P202911 | NM_144882 | 67198 | 2810022L02Rik | 1.005 | 0.995 | 0.908 | 0.913 | 0.931 | 0.902 |
| A_51_P418029 | NM_175541 | 245631 | Mum1I1 | 2.026 | 1.803 | 2.159 | 1.492 | 1.654 | 1.714 |
| A_52_P93837 | NM_008604 | 17380 | Mme | 1.516 | 1.805 | 1.761 | 1.442 | 1.369 | 1.329 |
| A_51_P275435 | NM_172411 | 71874 | 2310007B03Rik | 3.355 | 3.105 | 3.53 | 5.636 | 6.155 | 6.288 |
| A_51_P199888 | NM_026082 | 67299 | Dock7 | 1.182 | 1.246 | 1.295 | 0.669 | 0.678 | 1 |
| A_52_P211956 | NM_025658 | 66607 | Ms4a4d | 1.71 | 1.799 | 1.851 | 2.105 | 2.274 | 1.961 |
| A_51_P485985 | NM_134163 | 171170 | Mbnl3 | 1.781 | 1.802 | 1.648 | 0.929 | 1.067 | 1.035 |
| A_52_P630964 | NM_027533 | 70747 | Tspan2 | 1.217 | 1.314 | 1.26 | 0.815 | 0.863 | 0.897 |
| A_51_P294505 | NM_025658 | 66607 | Ms4a4d | 1.546 | 1.551 | 1.883 | 2.127 | 2.066 | 2.183 |
| A_52_P82488 | NM_021476 | 58861 | Cysltr1 | 1.919 | 1.474 | 1.811 | 2.106 | 2.164 | 2.621 |
| A_52_P634829 | NM_023835 | 76681 | Trim12 | 1.52 | 1.676 | 1.775 | 1.472 | 1.69 | 1.577 |
| A_52_P460882 | XM_130319;XM_130308 | 73668; 14725 | Ttc21b | 1.498 | 1.698 | 2.077 | 1.06 | 1.276 | 1.219 |
| A_52_P197826 | NM_010397 | 15039 | H2-T22 | 1.502 | 1.687 | 1.444 | 1.028 | 1.049 | 1.039 |
| A_51_P240253 | NM_019662 | 56437 | Rrad | 1.392 | 1.648 | 1.29 | 0.851 | 0.9 | 0.913 |
| A_51_P101975 | NM_010205 | 14179 | Fgf8 | 2.683 | 2.96 - | 2.139 | 3.25 | 3.463 | 3.854 |
| A_52_P21595 | NM_028238 | 72433 | Rab38 | 1.043 | 0.791 | 0.992 | 0.807 | 0.819 | 0.929 |
| A_52_P402786 | NM_008935 | 19126 | Prom1 | 1.498 | 1.71 | 1.749 | 1.028 | 1.023 | 0.92 |
| A_52_P126158 | NM_008326 | 15944 | Ifi1 | 1.157 | 1.034 | 1.006 | 1.254 | 1.3 | 1.232 |
| A_52_P676510 | NM_011579 | 21822 | Tgtp | 1.166 | 1.03 | 1.064 | 1.15 | 1.119 | 1.082 |
| A_52_P175685 | N_M_008390 | 16362 | Irf1 | 1.072 | 1.33 | 1.162 | 0.971 | 1.106 | 1.017 |
| A_52_P384718 | BC052056;XM_283264 | 320873 | | 1.488 | 1.451 | 1.435 | 1.051 | 1.032 | 1.026 |
| A_52_P176333 | NM_001005787 | 12695 | Cipp | 1.168 | 1.146 | 1.249 | 0.974 | 1.014 | 1.036 |
| A_52_P358951 | AK081999;XM_134736 | 271457; 22688 | Rab5a | 1.346 | 1.445 | 1.326 | 0.843 | 0.883 | 0.787 |
| A_51_P168646 | AK039580; | | | 1.655 | 1.373 | 1.436 | 2.357 | 2.478 | 2.267 |
| A_52_P620345 | NM_133979 | 102566 | AI604832 | 1.26 | 1.488 | 1.375 | 0.778 | 0.752 | 0.729 |
| A_52_P253179 | AK077477; | 16009 | lgfbp3 | 2.157 | 1.536 | 2.035 | 1.993 | 1.965 | 2.545 |
| A_51_P397003 | XM_133990 | 68904 | 1110065L07Rik | 1.235 | 1.343 | 1.238 | 0.778 | 0.763 | 0.855 |
| A_52_P74441 | NM_011570 | 21753 | Tes | 1.498 | 1.167 | 1.425 | 0.853 | 0.882 | 0.885 |
| A_52_P255250 | NM_009543 | 22644 | Rnf103 | 1.205 | 1.217 | 1.524 | 0.663 | 0.701 | 0.687 |
| A_52_P575505 | NM_008994 | 19302 | Pxmp3 | 1.123 | 1.07 | 1.092 | 0.797 | 0.815 | 0.802 |
| A_52_P469789 | NM_007588 | 12311 | Calcr | 2.087 | 2.206 | 2.278 | 1.646 | 1.638 | 1.533 |
| A_51_P345422 | NM_010747 | 17096 | Lyn | 0.888 | 0.803 | 0.881 | 1.006 | 0.995 | 0.89 |
| A_51_P356003 | NM_001007580 | 333564 | Gm784 | 1.223 | 1.221 | 1.186 | 1.543 | 1.618 | 1.409 |
| A_52_P680941 | NM_010284 | 14600 | Ghr | 1.72 | 1.563 | 1.682 | 2.058 | 1.978 | 2.065 |
| A_52_P380263 | NM_013723 | 27205 | Podxl | 1.179 | 1.159 | 1.66 | 0.899 | 0.909 | 0.942 |
| A_52_P286528 | BC055771; | 102954 | Nudt11 | 1.419 | 1.344 | 1.431 | 1.048 | 1.063 | 1.053 |
| A_51_P105068 | NM_027990 | 71897 | 2310010M24Rik | 1.902 | 1.982 | 1.72 | 2.012 | 2.165 | 2.002 |
| A_52_P132608 | NM_172685 | 229731 | Slc25a24 | 0.809 | 0.906 | 0.807 | 0.708 | 0.722 | 0.693 |
| A_51_P467505 | NM_013689 | 21682 | Tec | 1.077 | 1.062 | 1.153 | 0.842 | 0.861 | 0.881 |
| A_52_P153685 | AK129323; | 70422 | 2810417D08Rik | 1.763 | 1.676 | 1.845 | 2.204 | 2.099 | 2.085 |
| A_51_P174943 | NM_008485 | 16782 | Lamc2 | 1.282 | 1.255 | 1.249 | 1.068 | 1.04 | 1.004 |
| A_52_P301821 | NM_053078 | 27528 | D0H4S114 | 1.076 | 1.275 | 1.138 | 0.88 | 0.828 | 0.741 |
| A_52_P532910 | NM_024427 | 22003 | Tpm1 | 1.081 | 1.106 | 1.238 | 0.891 | 0.87 | 0.851 |
| A_51_P521084 | NM_025626 | 66540 | 3110001A13Rik | 1.423 | 1.425 | 1.474 | 0.832 | 0.865 | 0.776 |
| A_52_P680436 | NM_025793 | 66840 | Wdr45I | 1.338 | 1.213 | 1.346 | 0.755 | 0.702 | 0.805 |
| A_51_P164219 | NM_011909 | 24110 | Usp18 | 1.876 | 1.698 | 1.64 | 1.445 | 1.463 | 1.349 |
| A_52_P421234 | NM_133832 | 98711 | Rdh10 | 1.706 | 1.519 | 1.676 | 0.845 | 0.856 | 0.776 |
| A_52_P586157 | NM_022309 | 12400 | Cbfb | 1.417 | 1.551 | 1.338 | 0.773 | 0.863 | 0.903 |
| A_51_P262171 | NM_008326 | 15944 | Ifi1 | 1.271 | 1.011 | 1.135 | 1.457 | 1.571 | 1.485 |
| A_52_P452689 | NM_007498 | 11910 | Atf3 | 0.672 | 0.61 | 0.659 | 0.89 | 0.955 | 0.943 |
| A_52_P295933 | AK045060; | 14051 | B130023L16Rik | 1.637 | 1.973 | 2.08 | 1.007 | 0.993 | 1.019 |
| A_51_P222773 | NM_010446 | 15376 | Foxa2 | 2.333 | 1.693 | 1.64 | 6.867 | 6.289 | 6.137 |
| A_52_P361534 | NM_009521 | 22415 | Wnt3 | 1.193 | 0.891 | 0.969 | 2.247 | 2.459 | 2.231 |
| A_51_P507880 | NM_028980 | 74521 | 8430415E04Rik | 2.104 | 2.169 | 2.081 | 2.44 | 2.499 | 2.391 |
| A_52_P574668 | NM_011851 | 23959 | Nt5e | 1.611 | 1.127 | 1.284 | 1.764 | 1.707 | 1.826 |
| A_52_P957500 | XM_622354 | | | 0.371 | 0.532 | 0.397 | 1.528 | 1.372 | 1.305 |
| A_52_P345626 | AY149175; | 386626 | Mzf6d | 0.699 | 0.636 | 0.76 | 1.173 | 1.052 | 1.267 |
| A_52_P84015 | AK037762; | 11863 | | 0.695 | 0.924 | 0.842 | 2.965 | 3.344 | 3.023 |
| A_52_P432464 | AK053863; | 192196 | Luc7l2 | 0.7 | 0.863 | 1.032 | 1.457 | 1.269 | 1.582 |
| A_52_P376502 | BC025011; | 227541 | Camk1d | 0.664 | 0.71 | 0.737 | 0.973 | 0.893 | 1.008 |
| A_52_P354785 | AK037929; | 14852 | Gspt1 | 0.487 | 0.598 | 0.585 | 1.587 | 1.447 | 1.483 |
| A_51_P398766 | NM_010259 | 14468 | Gbp1 | 3.558 | 2.476 | 3.155 | 4.099 | 3.953 | 3.971 |
| A_52_P486234 | AK033497; | 22680 | | 0.658 | 0.943 | 0.756 | 2.133 | 2.185 | 1.908 |
| A_52_P628212 | AK044339; | 26380 | | 1.058 | 1.337 | 0.967 | 4.002 | 3.276 | 3.435 |
| A_52_P445799 | AK046777; | 52490 | D18Ertd289e | 2.088 | 2.22 | 2.899 | 2.952 | 2.963 | 3.531 |
| A_51_P268167 | BC037232;XM_621365 | 229363; 218850 | Gmps | 0.465 | 0.448 | 0.523 | 1.267 | 1.053 | 1.205 |
| A_51_P153079 | NM_133923 | 101100 | 4833441J24Rik | 0.42 | 0.232 | 0.278 | 1.153 | 1.158 | 1.047 |
| A_52_P584874 | NM_152895 | 75605 | Jarid1b | 0.737 | 0.83 | 0.752 | 1.242 | 1.251 | 1.242 |
| A_52_P307801 | NM_007847 | 13222 | Defcr-rs2 | 2.149 | 1.602 | 1.28 | 2.942 | 3.1 | 2.825 |
| A_52_P163148 | AB010370; | 544945 | | 0.704 | 0.687 | 0.76 | 1.098 | 1.144 | 1.325 |
| A_51_P171728 | NM_011926 | 26365 | Ceacam1 | 0.474 | 0.703 | 0.686 | 0.748 | 0.952 | 0.943 |
| A_51_P 41660 | AK053665;XM_358382 | 67776; 235380 | Loh 11 cr2a | 0.43 | 0.62 | 0.908 | 1.844 | 1.422 | 1.976 |
| A_52_P532323 | NM_178410 | 218518 | Marveld2 | 1.824 | 2.102 | 1.904 | 0.871 | 0.884 | 1.027 |
| A_51_P337125 | NM_010566 | 16331 | Inpp5d | 0.938 | 0.873 | 1.036 | 1.261 | 1.272 | 1.297 |
| A_52_P336259 | AK087367;XM_485576 | 319467 | A930002C04Rik | 0.552 | 0.507 | 0.623 | 1.053 | 0.913 | 0.8 |
| A_51_P318577 | AK010729; | 73671 | 2410078J06Rik | 0.605 | 0.863 | 0.641 | 0.897 | 1.028 | 1.15 |
| A_52_P324934 | AK045391; | 16403 | | 0.758 | 0.573 | 0.774 | 1.808 | 1.513 | 1.534 |
| A_52_P826666 | AK007019; | 51799 | Rap2ip | 0.894 | 1.131 | 1.14 | 2.913 | 2.479 | 3.106 |
| A_51_P200223 | XM_619497 | | | 0.65 | 0.646 | 0.76 | 1.392 | 1.367 | 1.332 |
| A_51_P228472 | NM_008343 | 16009 | lgfbp3 | 1.472 | 2.26 | 1.637 | 1.02 | 1.11 | 1.239 |
| A_52_P206573 | NM_027427 | 70439 | Taf15 | 0.525 | 0.7 | 0.806 | 1.685 | 1.521 | 1.76 |
| A_52_P354363 | NM_026574 | 68142 | | 0.545 | 0.546 | 0.579 | 1.246 | 1.113 | 1.072 |
| A_52_P651469 | NM_001012726 | 503692 | | 1.087 | 2.041 | 1.585 | 2.114 | 1.954 | 2.198 |
| A_51_P463789 | BC051947; | 58212 | | 0.924 | 0.962 | 0.995 | 1.518 | 1.581 | 1.46 |
| A_52_P18887 | NM_011468 | 20755 | Sprr2a | 7.008 | 6.235 | 5.038 | 6.207 | 6.689 | 6.331 |
| A_51_P223132 | NM_031183;XM_486227 | 83395; 70549 | Sp6 | 0.649 | 0.542 | 0.552 | 1.095 | 0.998 | 1.125 |
| A_52_P223083 | BC038323; | 72096 | 2010208K18Rik | 0.89 | 0.64 | 1.018 | 2.024 | 1.641 | 2.309 |
| A_52_P523399 | NM_029444 | 75801 | 4930447C04Rik | 1.167 | 0.873 | 1.364 | 1.373 | 1.087 | 1.771 |
| A_52_P282000 | AK020248; | 13800 | Enah | 0.368 | 0.482 | 0.376 | 1.791 | 1.63 | 1.536 |
| A_52_P152983 | AK011739; | 26886 | Cenph | 0.423 | 0.805 | 0.28 | 1.393 | 1.582 | 1.833 |
| A_52_P26416 | AF106279; | 16782 | | 1.436 | 1.802 | 1.779 | 0.723 | 0.806 | 0.697 |
| A_51_P102081 | NM_007847 | 13222 | Defcr-rs2 | 2.408 | 2.238 | 1.498 | 3.38 | 3.255 | 2.763 |
| A_51_P285725 | NM_009309 | 20997 | T | 2.672 | 1.6 | 1.512 | 3.129 | 3.214 | 3.267 |
| A_52_P827911 | AK081941; | | | 0.209 | 0.476 | 0.487 | 1.73 | 1.566 | 1.778 |
| A_51_P231341 | AK087764; | 108160 | D0HXS9928E | 0.131 | 0.284 | 0.334 | 1.584 | 1 | 1.654 |
| A_51_P334093 | NM_026228 | 67547 | SIc39a8 | 2.173 | 2.638 | 2.466 | 1.07 | 1.006 | 1.061 |
| A_52_P196568 | NM_144513 | 17263 | Gtl2 | 0.0697 | 0.0369 | 0.0417 | 0.112 | 0.0801 | 0.115 |
| A_52_P1108139 | AK053079; | | | 0.169 | 0.348 | 0.199 | 1.068 | 1.372 | 1.161 |
| A_52_P636559 | AK053008; | 268469 | 9530033F24Rik | 0.506 | 0.53 | 0.567 | 1.485 | 1.451 | 1.363 |
| A_52_P425734 | NM_145146 | 280662 | Afm | 1.466 | 1.63 | 2.105 | 1.902 | 1.789 | 2.093 |
| A_52_P1011737 | AK039096; | 106258 | Al790442 | 0.224 | 0.44 | 0.438 | 1.367 | 1.308 | 1.503 |
| A_52_P537140 | AK047235; | | | 0.566 | 0.565 | 0.531 | 1.029 | 1.127 | 1.156 |
| A_51_P191990 | AK087548; | 26563 | Ror1 | 0.378 | 0.368 | 0.389 | 1.197 | 1.149 | 1.176 |
| A_51_P478930 | AK080016; | | | 0.64 | 0.623 | 0.816 | 1.097 | 1.039 | 1.17 |
| A_51_P252565 | NM_009660 | 11687 | Alox15 | 1.618 | 1.425 | 1.194 | 1.366 | 1.436 | 1.457 |
| A_52_P81252 | NM_172599 | 218978 | D14Ertd436e | 0.265 | 0.408 | 0.488 | 1.153 | 1.046 | 0.927 |
| A_51_P487004 | NM_027732 | 71241 | Dmrtc2 | 1.918 | 2.991 | 1.94 | 1.77 | 1.537 | 1.901 |
| A_51_P104162 | AK053379; | 328232 | | 0.351 | 0.468 | 0.481 | 1.289 | 1.159 | 1.082 |
| A_52_P555603 | AK032770; | 11787 | Apbb2 | 0.273 | 0.355 | 0.304 | 1.021 | 1.021 | 1.054 |
| A_52_P819356 | AK041684; | | | 0.651 | 0.704 | 0.763 | 1.654 | 1.657 | 1.821 |
| A_52_P1196751 | AK086488; | | | 0.314 | 0.63 | 0.521 | 1.285 | 1.098 | 1.263 |
| A_52_P277415 | AK077313; | 76252 | 0610006O14Rik | 0.0165 | 0.0277 | 0.0223 | 0.0497 | 0.0428 | 0.0552 |
| A_52_P812362 | BC016205; | 408064 | BC064078 | 1.424 | 1.508 | 1.444 | 1.323 | 1.468 | 1.262 |
| A_51_P352572 | NM_019701 | 56365 | Clcnkb | 1.441 | 1.565 | 1.422 | 1.707 | 1.362 | 1.314 |
| A_52_P100042 | AK045143; | 320938 | Tnpo3 | 0.301 | 0.507 | 0.406 | 1.444 | 1.417 | 1.439 |
| A_51_P103397 | AK078521; | 54561 | Nap1I3 | 0.942 | 0.996 | 1.004 | 1.047 | 0.978 | 1.076 |
| A_52_P296244 | BC048734; | | | 2.333 | 2.799 | 1.851 | 2.371 | 2.371 | 2.372 |
| A_51_P305019 | AK020601; | 78423 | 9530049O05Rik | 0.201 | 0.4 | 0.429 | 1.187 | 1.059 | 1.249 |
| A_52_P3304 | NM_080446 | 117599 | Helb | 1.004 | 1.239 | 1.059 | 1.614 | 1.432 | 1.418 |
| A_51_P147651 | AK041797;XM_146705 | 245902 | | 0.511 | 0.729 | 0.963 | 1.47 | 1.214 | 1.502 |
| A_51_P203955 | NM_010260 | 14469 | Gbp2 | 2.194 | 2.061 | 2.214 | 2.45 | 2.681 | 2.345 |
| A_51_P176474 | AK010466; | 76484 | 2410012C07Rik | 0.842 | 0.903 | 1.004 | 0.996 | 1.036 | 1.127 |
| A_52_P165497 | NM_011385 | 20481 | Ski | 0.541 | 0.665 | 0.649 | 1.004 | 0.817 | 0.972 |
| A_51_P358714 | AK031433; | | | 0.23 | 0.294 | 0.418 | 1.005 | 0.775 | 1.214 |

**Table 4A - 100 Gene Subset (Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 0.266 | 0.299 | 0.281 | 0.396 | 0.368 | 0.407 |
| A_51_P335460 | NM_009132 | 20259 | Scin | 0.718 | 0.631 | 0.719 | 0.678 | 0.674 | 0.666 |
| A_52_P391505 | NM_144513 | 17263 | Gtl2 | 1.36 | 1.291 | 1.327 | 1.053 | 1.04 | 0.852 |
| A_51_P497100 | NM_010706 | 16855 | Lgals4 | 2.115 | 2.049 | 2.368 | 1.716 | 1.861 | 1.527 |
| A_51_P378371 | | | | 0.454 | 0.542 | 0.39 | 0.468 | 0.484 | 0.468 |
| A_51_P298266 | NM_025681 | 66643 | Lix1 | 0.507 | 0.414 | 0.416 | 0.554 | 0.584 | 0.54 |
| A_52_P269158 | NM_001003948 | 98496 | AL024069 | 0.316 | 0.33 | 0.33 | 0.388 | 0.393 | 0.443 |
| A_51_P311038 | BC009666;XM_134720 | 57267 | Apba3 | 0.334 | 0.341 | 0.339 | 0.446 | 0.472 | 0.482 |
| A_51_P457187 | NM_028075 | 72049 | Tnfrsf13c | 2.82 | 3.275 | 3.026 | 2.654 | 3.432 | 2.546 |
| A_52_P68702 | NM_145148 | 232288 | Frmd4b | 0.545 | 0.476 | 0.538 | 0.501 | 0.43 | 0.424 |
| A_52_P293443 | AK087208; | 13819 | | 1.278 | 1.075 | 1.13 | 1.496 | 1.604 | 1.349 |
| A_51_P157986 | NM_029930 | 77574 | 3321401 G04Rik | 0.426 | 0.339 | 0.345 | 0.368 | 0.318 | 0.37 |
| A_52_P1013441 | | | | 3.248 | 3.164 | 3.191 | 4.558 | 5.61 | 4.683 |
| A_51_P418375 | NM_023844 | 67374 | Jam2 | 1.691 | 1.467 | 1.625 | 2.142 | 2.132 | 2.288 |
| A_52_P557940 | AK004302; | 66704 | 4921506I22Rik | 2.114 | 1.863 | 2.221 | 2.17 | 2.013 | 2.051 |
| A_51_P480311 | NM_010168 | 14061 | F2 | 1.991 | 1.809 | 1.969 | 2.465 | 2.4 | 1.874 |
| A_52_P200458 | NM_010242 | 14345 | Fut4 | 0.429 | 0.343 | 0.355 | 0.453 | 0.391 | 0.512 |
| A_51_P463846 | NM_145545 | 229900 | 9830147J24Rik | 0.694 | 0.519 | 0.59 | 0.86 | 0.816 | 0.817 |
| A_51_P162474 | AF285577; | 107815 | Scml2 | 0.539 | 0.413 | 0.405 | 0.643 | 0.645 | 0.71 |
| A_51_P165244 | NM_018734 | 55932 | Gbp4 | 0.585 | 0.512 | 0.474 | 1.008 | 0.981 | 1.006 |
| A_52_P59318 | NM_133832 | 98711 | Rdh10 | 0.306 | 0.273 | 0.275 | 0.373 | 0.395 | 0.445 |
| A_52_P438036 | NM_175271 | 78134 | Gpr23 | 0.376 | 0.334 | 0.36 | 0.455 | 0.441 | 0.489 |
| A_52_P474242 | BE457768; | | | 0.499 | 0.53 | 0.46 | 0.69 | 0.658 | 0.726 |
| A_51_P499854 | NM_010284 | 14600 | Ghr | 0.407 | 0.375 | 0.412 | 0.413 | 0.429 | 0.489 |
| A_51_P455647 | NM_009801 | 12349 | Car2 | 0.335 | 0.288 | 0.301 | 0.568 | 0.516 | 0.638 |
| A_52_P103929 | NM_009306 | 20979 | Syt1 | 0.382 | 0.356 | 0.39 | 0.419 | 0.498 | 0.423 |
| A_51_P149469 | NM_009608 | 11464 | Actc1 | 0.324 | 0.387 | 0.384 | 0.496 | 0.551 | 0.471 |
| A_52_P557293 | NM_015775 | 50528 | Tmprss2 | 0.277 | 0.205 | 0.179 | 0.45 | 0.457 | 0.355 |
| A_52_P50496 | AY048587;XM_619453 | 114640 | Tifp39 | 0.454 | 0.501 | 0.422 | 0.661 | 0.638 | 0.694 |
| A_51_P180413 | NM_176930 | 319504 | C130076O07Rik | 0.359 | 0.393 | 0.415 | 0.471 | 0.514 | 0.494 |
| A_51_P229613 | NM_008792 | 18549 | Pcsk2 | 0.512 | 0.483 | 0.515 | 0.58 | 0.58 | 0.504 |
| A_51_P121635 | NM_030700 | 80884 | Maged2 | 0.378 | 0.371 | 0.409 | 0.492 | 0.475 | 0.481 |
| A_51_P481777 | NM_018756 | 54382 | Tcstv1 | 1.532 | 1.822 | 1.792 | 2.619 | 3.155 | 2.956 |
| A_51_P412817 | NM_177617 | 216851 | D330014H01Rik | 3.151 | 3.039 | 3.326 | 2.136 | 2.194 | 1.865 |
| A_51_P387528 | AK019664; | 78801 | Ak7 | 2.693 | 2.814 | 2.57 | 3.786 | 3.834 | 3.71 |
| A_52_P78168 | AK031518; | | | 2.108 | 1.677 | 1.617 | 1.783 | 1.791 | 1.711 |
| A_52_P274960 | NM_001005508 | 226652 | 6030405P05Rik | 1.83 | 1.82 | 1.948 | 2.07 | 2.181 | 2.076 |
| A_52_P1188270 | AK042362; | | | 3.076 | 1.828 | 1.917 | 2.932 | 3.046 | 2.87 |
| A_51_P291227 | NM_001005508 | 226652 | 6030405P05Rik | 1.876 | 1.826 | 1.86 | 2.054 | 2.185 | 2.004 |
| A_52_P377299 | AK048360; | 271377 | Zbtb11 | 1.927 | 1.892 | 1.923 | 1.678 | 1.79 | 1.546 |
| A_51_P297131 | NM_009502 | 22330 | Vcl | 0.402 | 0.372 | 0.378 | 0.411 | 0.377 | 0.401 |
| A_52_P804224 | | | | 1.522 | 1.688 | 1.828 | 2.31 | 2.487 | 2.241 |
| A_51_P442894 | AK048310; | 621603 | C130048D07Rik | 2.632 | 2.364 | 2.281 | 3.657 | 3.714 | 3.102 |
| A_52_P309718 | AK173099; | 218850 | D14Abb1e | 2.324 | 2.579 | 2.18 | 2.614 | 2.72 | 2.711 |
| A_52_P359010 | AK129478; | 72193 | Sfrs2ip | 2.152 | 2.149 | 2.122 | 2.349 | 2.38 | 2.318 |
| A_51_P410813 | AK079732; | | | 2.732 | 2.716 | 2.559 | 2.898 | 2.605 | 2.3 |
| A_52_P382565 | XM_483943 | 432493 | LOC432493 | 0.976 | 1.222 | 1.221 | 1.492 | 1.439 | 1.198 |
| A_51_P333253 | NM_178440 | 246177 | Myo1g | 1.419 | 1.413 | 1.621 | 1.544 | 1.504 | 1.394 |
| A_52_P113190 | NM_053214 | 17916 | Myo1f | 1.428 | 1.513 | 1.396 | 1.988 | 1.959 | 2.083 |
| A_51_P473953 | XM_358600;XM_355454 | 381498 | 4930592C13Rik | 0.42 | 0.306 | 0.298 | 0.365 | 0.365 | 0.38 |
| A_52_P519870 | AB010340; | | | 2.391 | 3.34 | 2.621 | 3.76 | 3.72 | 1.955 |
| A_51_P266403 | AK009163; | 76730 | 2310005C01Rik | 3.079 | 3.053 | 3.092 | 2.733 | 2.967 | 2.734 |
| A_52_P3698 | NM_001015099 | 217558 | | 1.885 | 1.84 | 1.762 | 2.392 | 2.296 | 2.486 |
| A_52_P581100 | NM_027285 | 70005 | 1700029I01Rik | 2.295 | 3.266 | 2.708 | 3.168 | 3.146 | 2.879 |
| A_51_P276305 | NM_009292 | 20899 | Stra8 | 3.137 | 3.069 | 3.174 | 2.824 | 2.972 | 2.777 |
| A_51_P455997 | Y13832; | 17263 | Gtl2 | 1.505 | 1.455 | 1.44 | 0.998 | 1.082 | 0.998 |
| A_52_P656336 | NM_023733 | 74114 | Crot | 0.407 | 0.351 | 0.381 | 0.755 | 0.647 | 0.8 |
| A_51_P512172 | AK010953;XM_125673 | 52463 | Cxxc6 | 2.864 | 2.384 | 2.216 | 2.184 | 2.11 | 2.068 |
| A_51_P282227 | NM_020577 | 57344 | As3mt | 2.651 | 2.607 | 2.876 | 3.224 | 3.327 | 3.147 |
| A_52_P386544 | NM_026594 | 68172 | 4930517K11Rik | 2.843 | 2.618 | 2.802 | 2.673 | 2.913 | 2.894 |
| A_51_P125679 | AK010725;XM_134948 | 73673 | 2410076I21Rik | 2.867 | 2.332 | 2.464 | 3.518 | 3.661 | 3.727 |
| A_51_P164504 | NM_007469 | 11812 | Apoc1 | 3.245 | 2.908 | 3.052 | 2.863 | 3.059 | 2.591 |
| A_52_P240706 | NM_007543 | 26367 | Ceacam2 | 2.285 | 1.866 | 1.892 | 2.654 | 2.556 | 2.628 |
| A_52_P420369 | NM_029444 | 75801 | 4930447C04Rik | 3.256 | 2.433 | 2.971 | 2.042 | 2.189 | 2.206 |
| A_51_P242399 | NM_031170 | 16691 | Krt2-8 | 0.193 | 0.233 | 0.208 | 0.403 | 0.4 | 0.39 |
| A_51_P158037 | NM_013505 | 13506 | Dsc2 | 0.271 | 0.249 | 0.258 | 0.437 | 0.415 | 0.453 |
| A_52_P258116 | NM_009521 | 22415 | Wnt3 | 0.156 | 0.164 | 0.16 | 0.312 | 0.309 | 0.296 |
| A_52_P286166 | NM_001002927 | 18619 | Penk1 | 0.497 | 0.324 | 0.339 | 0.671 | 0.658 | 0.647 |
| A_52_P399095 | NM_177793 | 327747 | 9030224M15Rik | 0.366 | 0.325 | 0.163 | 0.433 | 0.401 | 0.466 |
| A_52_P470663 | | | | 0.377 | 0.334 | 0.403 | 0.467 | 0.439 | 0.566 |
| A_52_P376169 | NM_177139 | 320343 | E130115E03Rik | 0.314 | 0.285 | 0.295 | 0.539 | 0.508 | 0.528 |
| A_51_P512085 | NM_130449 | 140792 | Colec12 | 0.335 | 0.364 | 0.296 | 0.382 | 0.368 | 0.385 |
| A_52_P676403 | NM_019494 | 56066 | Cxcl11 | 0.525 | 0.596 | 0.597 | 0.761 | 0.809 | 0.704 |
| A_51_P354307 | NM_172469 | 209195 | Clic6 | 0.43 | 0.319 | 0.329 | 0.398 | 0.398 | 0.387 |
| A_52_P569375 | NM_010203 | 14176 | Fgf5 | 0.776 | 0.678 | 0.554 | 0.45 | 0.467 | 0.574 |
| A_51_P162481 | NM_133194 | 107815 | Scml2 | 0.432 | 0.357 | 0.385 | 0.594 | 0.55 | 0.618 |
| A_51_P202911 | NM_144882 | 67198 | 2810022L02Rik | 0.297 | 0.283 | 0.328 | 0.346 | 0.333 | 0.365 |
| A_51_P418029 | NM_175541 | 245631 | Mum1I1 | 0.319 | 0.265 | 0.278 | 0.419 | 0.397 | 0.534 |
| A_52_P93837 | NM_008604 | 17380 | Mme | 0.559 | 0.439 | 0.513 | 0.653 | 0.573 | 0.747 |
| A_51_P275435 | NM_172411 | 71874 | 2310007B03Rik | 0.368 | 0.492 | 0.206 | 0.875 | 0.83 | 0.857 |
| A_51_P199888 | NM_026082 | 67299 | Dock7 | 0.407 | 0.403 | 0.245 | 0.558 | 0.428 | 0.546 |
| A_52_P211956 | NM_025658 | 66607 | Ms4a4d | 0.61 | 0.455 | 0.457 | 0.534 | 0.519 | 0.685 |
| A_51_P485985 | NM_134163 | 171170 | Mbnl3 | 0.223 | 0.181 | 0.161 | 0.625 | 0.543 | 0.697 |
| A_52_P630964 | NM_027533 | 70747 | Tspan2 | 0.361 | 0.283 | 0.324 | 0.499 | 0.473 | 0.525 |
| A_51_P294505 | NM_025658 | 66607 | Ms4a4d | 0.63 | 0.414 | 0.365 | 0.532 | 0.521 | 0.629 |
| A_52_P82488 | NM_021476 | 58861 | Cysltr1 | 0.319 | 0.379 | 0.306 | 0.5 | 0.438 | 0.534 |
| A_52_P634829 | NM_023835 | 76681 | Trim12 | 0.59 | 0.561 | 0.672 | 1.012 | 0.996 | 0.866 |
| A_52_P460882 | XM_130319;XM_130308 | 73668; 14725 | Ttc21 b | 0.412 | 0.637 | 0.533 | 0.396 | 0.633 | 0.502 |
| A_52_P197826 | NM_010397 | 15039 | H2-T22 | 0.541 | 0.505 | 0.41 | 0.653 | 0.634 | 0.622 |
| A_51_P240253 | NM_019662 | 56437 | Rrad | 0.316 | 0.322 | 0.314 | 0.392 | 0.337 | 0.342 |
| A_51_P101975 | NM_010205 | 14179 | Fgf8 | 0.412 | 0.39 | 0.413 | 0.415 | 0.454 | 0.385 |
| A_52_P21595 | NM_028238 | 72433 | Rab38 | 0.234 | 0.188 | 0.168 | 0.439 | 0.467 | 0.472 |
| A_52_P402786 | NM_008935 | 19126 | Prom1 | 0.37 | 0.396 | 0.335 | 0.551 | 0.506 | 0.594 |
| A_52_P126158 | | 15944 | Ifi1 | 0.237 | 0.208 | 0.22 | 0.415 | 0.412 | 0.425 |
| A_52_P676510 | NM_008326 NM_011579 | 21822 | Tgtp | 0.225 | 0.162 | 0.155 | 0.382 | 0.378 | 0.362 |
| A_52_P175685 | NM_008390 | 16362 | Irf1 | 0.401 | 0.37 | 0.3 | 0.663 | 0.637 | 0.749 |
| A_52_P384718 | BC052056;XM_283264 | 320873 | | 0.508 | 0.441 | 0.458 | 0.541 | 0.512 | 0.578 |
| A_52_P176333 | NM_001005787 | 12695 | Cipp | 0.372 | 0.357 | 0.365 | 0.452 | 0.392 | 0.42 |
| A_52_P358951 | AK081999;XM_134736 | 271457; 22688 | Rab5a | 0.417 | 0.378 | 0.448 | 0.464 | 0.449 | 0.46 |
| A_51_P168646 | AK039580; | | | 0.442 | 0.434 | 0.509 | 0.537 | 0.548 | 0.598 |

**Table 4B-100 Gene Subset (Non Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 1.152 | 1.407 | 1.101 | 0.859 | 0.96 | 0.924 |
| A_51_P335460 | NM_009132 | 20259 | Scin | 1.658 | 1.913 | 1.519 | 1.101 | 1.225 | 1.107 |
| A_52_P391505 | NM_144513 | 17263 | Gtl2 | 0.0793 | 0.114 | 0.0867 | 0.158 | 0.15 | 0.148 |
| A_51_P497100 | NM_010706 | 16855 | Lgals4 | 0.692 | 0.771 | 0.849 | 1.002 | 0.908 | 0.828 |
| A_51_P378371 | | | | 0.986 | 1.006 | 1.208 | 1.217 | 1.156 | 1.234 |
| A_51_P298266 | NM_025681 | 66643 | Lix1 | 1.123 | 1.145 | 1.109 | 1.143 | 1.15 | 1.141 |
| A_52_P269158 | NM_001003948 | 98496 | AL024069 | 1.775 | 1.419 | 1.506 | 1.494 | 1.391 | 1.254 |
| A_51_P311038 | BC009666;XM_134720 | 57267 | Apba3 | 1.847 | 1.642 | 1.538 | 2.55 | 2.405 | 2.808 |
| A_51_P457187 | NM_028075 | 72049 | Tnfrsf13c | 1.083 | 1.58 | 1.373 | 0.916 | 1.099 | 1.091 |
| A_52_P68702 | NM_145148 | 232288 | Frmd4b | 0.928 | 1.071 | 0.877 | 1.242 | 1.234 | 1.139 |
| A_52_P293443 | AK087208; | 13819 | | 0.179 | 0.222 | 0.219 | 0.543 | 0.565 | 0.552 |
| A_51_P157986 | NM_029930 | 77574 | 3321401G04Rik | 0.909 | 1.196 | 1.088 | 0.563 | 0.621 | 0.598 |
| A_52_P1013441 | | | | 1.255 | 1.032 | 2.041 | 1.173 | 1.203 | 1.341 |
| A_51_P418375 | NM_023844 | 67374 | Jam2 | 1.044 | 0.943 | 0.99 | 0.826 | 0.786 | 0.84 |
| A_52_P557940 | AK004302; | 66704 | 4921506I22Rik | 0.544 | 0.595 | 0.569 | 1.371 | 1.379 | 1.241 |
| A_51_P480311 | NM_010168 | 14061 | F2 | 0.348 | 0.476 | 0.622 | 0.996 | 1.004 | 1.186 |
| A_52_P200458 | NM_010242 | 14345 | Fut4 | 1.003 | 0.997 | 1.017 | 1.108 | 1.203 | 1.09 |
| A_51_P463846 | NM_145545 | 229900 | 9830147J24Rik | 2.28 | 2.199 | 2.49 | 2.068 | 1.958 | 1.72 |
| A_51_P162474 | AF285577; | 107815 | Scml2 | 2.885 | 2.549 | 2.371 | 3.021 | 3.116 | 3.1 |
| A_51_P165244 | NM_018734 | 55932 | Gbp4 | 2.388 | 1.319 | 2.543 | 2.404 | 2.051 | 2.534 |
| A_52_P59318 | NM_133832 | 98711 | Rdh10 | 1.689 | 1.611 | 1.584 | 0.876 | 1.002 | 0.955 |
| A_52_P438036 | NM_175271 | 78134 | Gpr23 | 1.082 | 0.96 | 0.968 | 1.634 | 1.593 | 1.618 |
| A_52_P474242 | BE457768; | | | 1.31 | 1.785 | 1.55 | 1.137 | 1.132 | 0.997 |
| A_51_P499854 | NM_010284 | 14600 | Ghr | 1.55 | 1.19 | 1.486 | 2.502 | 2.537 | 2.196 |
| A_51_P455647 | NM_009801 | 12349 | Car2 | 1.592 | 1.463 | 1.337 | 2.139 | 2.347 | 2.17 |
| A_52_P103929 | NM_009306 | 20979 | Syt1 | 0.898 | 0.938 | 0.967 | 1.626 | 1.464 | 1.422 |
| A_51_P149469 | NM_009608 | 11464 | Actc1 | 1.012 | 1.006 | 0.935 | 0.971 | 0.997 | 1.12 |
| A_52_P557293 | NM_015775 | 50528 | Tmprss2 | 1.142 | 1.091 | 1.111 | 1.208 | 1.127 | 1.225 |
| A_52_P50496 | AY048587;XM_619453 | 114640 | Tifp39 | 1.196 | 1.551 | 1.504 | 1.114 | 1.171 | 1.214 |
| A_51_P180413 | NM_176930 | 319504 | C130076O07Rik | 1.603 | 1.54 | 1.395 | 1.297 | 1.25 | 1.155 |
| A_51_P229613 | NM_008792 | 18549 | Pcsk2 | 1.463 | 1.53 | 1.37 | 1.333 | 1.422 | 1.554 |
| A_51_P121635 | NM_030700 | 80884 | Maged2 | 1.225 | 1.426 | 1.335 | 0.918 | 0.99 | 0.88 |
| A_51_P481777 | NM_018756 | 54382 | Tcstv1 | 0.208 | 0.696 | 0.654 | 0.592 | 0.503 | 0.72 |
| A_51_P412817 | NM_177617 | 216851 | D330014H01 Rik | 0.725 | 0.834 | 1.04 | 1.083 | 0.986 | 0.997 |
| A_51_P387528 | AK019664; | 78801 | Ak7 | 0.65 | 0.537 | 0.813 | 0.767 | 0.778 | 1.11 |
| A_52_P78168 | AK031518; | | | 0.277 | 0.385 | 0.299 | 0.704 | 0.652 | 0.544 |
| A_52_P274960 | NM_001005508 | 226652 | 6030405P05Rik | 0.671 | 0.634 | 0.712 | 0.763 | 0.781 | 0.808 |
| A_52_P1188270 | AK042362; | | | 0.341 | 0.594 | 0.564 | 1.758 | 1.462 | 1.658 |
| A_51_P291227 | NM_001005508 | 226652 | 6030405P05Rik | 0.705 | 0.681 | 0.731 | 0.814 | 0.735 | 0.798 |
| A_52_P377299 | AK048360; | 271377 | Zbtb11 | 0.577 | 0.582 | 0.645 | 1.152 | 1.092 | 1.023 |
| A_51_P297131 | NM_009502 | 22330 | Vcl | 1.015 | 1.436 | 0.955 | 0.556 | 0.672 | 0.587 |
| A_52_P804224 | | | | 0.299 | 0.314 | 0.291 | 1.263 | 1.2 | 1.068 |
| A_51_P442894 | AK048310; | 621603 | C130048D07Rik | 1.023 | 0.841 | 1.218 | 0.788 | 0.867 | 0.977 |
| A_52_P309718 | AK173099; | 218850 | D14Abb1e | 1.006 | 0.813 | 1.127 | 1.186 | 1.29 | 1.289 |
| A_52_P359010 | AK129478; | 72193 | Sfrs2ip | 0.54 | 0.706 | 0.574 | 1.484 | 1.398 | 1.403 |
| A_51_P410813 | AK079732; | | | 0.717 | 0.877 | 0.904 | 1.593 | 1.473 | 1.607 |
| A_52_P382565 | XM_483943 | 432493 | LOC432493 | 0.228 | 0.273 | 0.254 | 0.607 | 0.622 | 0.462 |
| A_51_P333253 | NM_178440 | 246177 | Myo1g | 0.535 | 0.491 | 0.538 | 0.474 | 0.481 | 0.37 |
| A_52_P113190 | NM_053214 | 17916 | Myo1f | 0.386 | 0.513 | 0.397 | 0.243 | 0.232 | 0.264 |
| A_51_P473953 | XM_358600;XM_355454 | 381498 | 4930592C13Rik | 2.138 | 1.593 | 1.947 | 2.323 | 2.226 | 2.26 |
| A_52_P519870 | AB010340; | | | 0.758 | 0.675 | 1.128 | 0.989 | 0.863 | 1.393 |
| A_51_P266403 | AK009163; | 76730 | 2310005C01 Rik | 0.984 | 0.901 | 1.009 | 1.273 | 1.313 | 1.391 |
| A_52_P3698 | NM_001015099 | 217558 | | 0.658 | 0.653 | 0.676 | 1.487 | 1.564 | 1.537 |
| A_52_P581100 | NM_027285 | 70005 | 1700029101Rik | 1.164 | 1.1 | 1.141 | 1.525 | 1.383 | 1.305 |
| A_51_P276305 | NM_009292 | 20899 | Stra8 | 0.873 | 0.934 | 0.982 | 0.431 | 0.426 | 0.471 |
| A_51_P455997 | Y13832; | 17263 | Gtl2 | 0.123 | 0.184 | 0.114 | 0.213 | 0.197 | 0.258 |
| A_52_P656336 | NM_023733 | 74114 | Crot | 1.233 | 1.547 | 1.442 | 0.983 | 1.067 | 0.998 |
| A_51_P512172 | AK010953;XM_125673 | 52463 | Cxxc6 | 0.8 | 0.986 | 0.748 | 1.033 | 1.014 | 1.046 |
| A_51_P282227 | NM_020577 | 57344 | As3mt | 0.79 | 0.761 | 0.777 | 1.02 | 0.91 | 0.98 |
| A_52_P386544 | NM_026594 | 68172 | 4930517K11Rik | 0.74 | 0.606 | 0.729 | 1.188 | 1.159 | 1.351 |
| A_51_P125679 | AK010725;XM_134948 | 73673 | 2410076I21Rik | 0.588 | 0.959 | 0.916 | 0.84 | 0.843 | 0.849 |
| A_51_P164504 | NM_007469 | 11812 | Apoc1 | 1.307 | 1.245 | 1.314 | 1.412 | 1.375 | 1.457 |
| A_52_P240706 | NM_007543 | 26367 | Ceacam2 | 0.87 | 0.778 | 0.892 | 0.689 | 0.756 | 0.859 |
| A_52_P420369 | NM_029444 | 75801 | 4930447C04Rik | 1.087 | 1.084 | 1.129 | 1.176 | 0.989 | 1.041 |
| A_51_P242399 | NM_031170 | 16691 | Krt2-8 | 1.013 | 1.199 | 0.939 | 0.962 | 0.977 | 0.946 |
| A_51_P158037 | NM_013505 | 13506 | Dsc2 | 1.238 | 1.562 | 1.304 | 0.743 | 0.819 | 0.875 |
| A_52_P258116 | NM_009521 | 22415 | Wnt3 | 1.304 | 1.249 | 1.083 | 1.811 | 1.943 | 2.006 |
| A_52_P286166 | NM_001002927 | 18619 | Penk1 | 1.649 | 2.509 | 2.439 | 1.542 | 1.453 | 1.374 |
| A_52_P399095 | NM_177793 | 327747 | 9030224M15Rik | 1.11 | 0.976 | 1.056 | 1.583 | 1.692 | 1.596 |
| A_52_P470663 | | | | 2.339 | 2.72 | 2.405 | 1.069 | 1.105 | 1.018 |
| A_52_P376169 | NM_177139 | 320343 | E130115E03Rik | 1.184 | 1.025 | 1.076 | 1.949 | 1.81 | 1.828 |
| A_51_P512085 | NM_130449 | 140792 | Colec12 | 1.574 | 1.25 | 1.471 | 1.463 | 1.651 | 1.693 |
| A_52_P676403 | NM_019494 | 56066 | Cxcl11 | 3.86 | 3.177 | 3.663 | 2.834 | 2.723 | 2.826 |
| A_51_P354307 | NM_172469 | 209195 | Clic6 | 1.31 | 1.578 | 1.57 | 0.719 | 0.748 | 0.659 |
| A_52_P569375 | NM_010203 | 14176 | Fgf5 | 4.338 | 3.206 | 3.958 | 5.889 | 5.927 | 6.75 |
| A_51_P162481 | NM_133194 | 107815 | Scml2 | 2.434 | 2.616 | 2.232 | 2.312 | 2.394 | 2.596 |
| A_51_P202911 | NM_144882 | 67198 | 2810022L02Rik | 1.005 | 0.995 | 0.908 | 0.913 | 0.931 | 0.902 |
| A_51_P418029 | NM_175541 | 245631 | Mum1I1 | 2.026 | 1.803 | 2.159 | 1.492 | 1.654 | 1.714 |
| A_52_P93837 | NM_008604 | 17380 | Mme | 1.516 | 1.805 | 1.761 | 1.442 | 1.369 | 1.329 |
| A_51_P275435 | NM_172411 | 71874 | 2310007B03Rik | 3.355 | 3.105 | 3.53 | 5.636 | 6.155 | 6.288 |
| A_51_P199888 | NM_026082 | 67299 | Dock7 | 1.182 | 1.246 | 1.295 | 0.669 | 0.678 | 1 |
| A_52_P211956 | NM_025658 | 66607 | Ms4a4d | 1.71 | 1.799 | 1.851 | 2.105 | 2.274 | 1.961 |
| A_51_P485985 | NM_134163 | 171170 | Mbnl3 | 1.781 | 1.802 | 1.648 | 0.929 | 1.067 | 1.035 |
| A_52_P630964 | NM_027533 | 70747 | Tspan2 | 1.217 | 1.314 | 1.26 | 0.815 | 0.863 | 0.897 |
| A_51_P294505 | NM_025658 | 66607 | Ms4a4d | 1.546 | 1.551 | 1.883 | 2.127 | 2.066 | 2.183 |
| A_52_P82488 | NM_021476 | 58861 | Cysltr1 | 1.919 | 1.474 | 1.811 | 2.106 | 2.164 | 2.621 |
| A_52_P634829 | NM_023835 | 76681 | Trim12 | 1.52 | 1.676 | 1.775 | 1.472 | 1.69 | 1.577 |
| A_52_P460882 | XM_130319;XM_130308 | 73668; 14725 | Ttc21 b | 1.498 | 1.698 | 2.077 | 1.06 | 1.276 | 1.219 |
| A_52_P197826 | NM_010397 | 15039 | H2-T22 | 1.502 | 1.687 | 1.444 | 1.028 | 1.049 | 1.039 |
| A_51_P240253 | NM_019662 | 56437 | Rrad | 1.392 | 1.648 | 1.29 | 0.851 | 0.9 | 0.913 |
| A_51_P101975 | NM_010205 | 14179 | Fgf8 | 2.683 | 2.96 | 2.139 | 3.25 | 3.463 | 3.854 |
| A_52_P21595 | NM_028238 | 72433 | Rab38 | 1.043 | 0.791 | 0.992 | 0.807 | 0.819 | 0.929 |
| A_52_P402786 | NM_008935 | 19126 | Proml | 1.498 | 1.71 | 1.749 | 1.028 | 1.023 | 0.92 |
| A_52_P126158 | NM_008326 | 15944 | Ifi1 | 1.157 | 1.034 | 1.006 | 1.254 | 1.3 | 1.232 |
| A_52_P676510 | NM_011579 | 21822 | Tgtp | 1.166 | 1.03 | 1.064 | 1.15 | 1.119 | 1.082 |
| A_52_P175685 | NM_008390 | 16362 | Irf1 | 1.072 | 1.33 | 1.162 | 0.971 | 1.106 | 1.017 |
| A_52_P384718 | BC052056;XM_283264 | 320873 | | 1.488 | 1.451 | 1.435 | 1.051 | 1.032 | 1.026 |
| A_52_P176333 | NM_001005787 | 12695 | Cipp | 1.168 | 1.146 | 1.249 | 0.974 | 1.014 | 1.036 |
| A_52_P358951 | AK081999;XM_134736 | 271457; 22688 | Rab5a | 1.346 | 1.445 | 1.326 | 0.843 | 0.883 | 0.787 |
| A_51_P168646 | AK039580; | | | 1.655 | 1.373 | 1.436 | 2.357 | 2.478 | 2.267 |

**Table 5A - 50 Gene Subset (Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 0.266 | 0.299 | 0.281 | 0.396 | 0.368 | 0.407 |
| A_51_P335460 | NM_009132 | 20259 | Scin | 0.718 | 0.631 | 0.719 | 0.678 | 0.674 | 0.666 |
| A_52_P391505 | NM_144513 | 17263 | Gtl2 | 1.36 | 1.291 | 1.327 | 1.053 | 1.04 | 0.852 |
| A_51_P497100 | NM_010706 | 16855 | Lgals4 | 2.115 | 2.049 | 2.368 | 1.716 | 1.861 | 1.527 |
| A_51_P378371 | | | | 0.454 | 0.542 | 0.39 | 0.468 | 0.484 | 0.468 |
| A_51_P298266 | NM_025681 | 66643 | Lix1 | 0.507 | 0.414 | 0.416 | 0.554 | 0.584 | 0.54 |
| A_52_P269158 | NM_001003948 | 98496 | AL024069 | 0.316 | 0.33 | 0.33 | 0.388 | 0.393 | 0.443 |
| A_51_P311038 | BC009666;XM_134720 | 57267 | Apba3 | 0.334 | 0.341 | 0.339 | 0.446 | 0.472 | 0.482 |
| A_51_P457187 | NM_028075 | 72049 | Tnfrsf13c | 2.82 | 3.275 | 3.026 | 2.654 | 3.432 | 2.546 |
| A_52_P68702 | NM_145148 | 232288 | Frmd4b | 0.545 | 0.476 | 0.538 | 0.501 | 0.43 | 0.424 |
| A_52_P293443 | AK087208; | 13819 | | 1.278 | 1.075 | 1.13 | 1.496 | 1.604 | 1.349 |
| A_51_P157986 | NM_029930 | 77574 | 3321401 G04Rik | 0.426 | 0.339 | 0.345 | 0.368 | 0.318 | 0.37 |
| A_52_P1013441 | | | | 3.248 | 3.164 | 3.191 | 4.558 | 5.61 | 4.683 |
| A_51_P418375 | NM_023844 | 67374 | Jam2 | 1.691 | 1.467 | 1.625 | 2.142 | 2.132 | 2.288 |
| A_52_P557940 | AK004302; | 66704 | 4921506I22Rik | 2.114 | 1.863 | 2.221 | 2.17 | 2.013 | 2.051 |
| A_51_P480311 | NM_010168 | 14061 | F2 | 1.991 | 1.809 | 1.969 | 2.465 | 2.4 | 1.874 |
| A_52_P200458 | NM_010242 | 14345 | Fut4 | 0.429 | 0.343 | 0.355 | 0.453 | 0.391 | 0.512 |
| A_51_P463846 | NM_145545 | 229900 | 9830147J24Rik | 0.694 | 0.519 | 0.59 | 0.86 | 0.816 | 0.817 |
| A_51_P162474 | AF285577; | 107815 | Scm12 | 0.539 | 0.413 | 0.405 | 0.643 | 0.645 | 0.71 |
| A_51_P165244 | NM_018734 | 55932 | Gbp4 | 0.585 | 0.512 | 0.474 | 1.008 | 0.981 | 1.006 |
| A_52_P59318 | NM_133832 | 98711 | Rdh10 | 0.306 | 0.273 | 0.275 | 0.373 | 0.395 | 0.445 |
| A_52_P438036 | NM_175271 | 78134 | Gpr23 | 0.376 | 0.334 | 0.36 | 0.455 | 0.441 | 0.489 |
| A_52_P474242 | BE457768; | | | 0.499 | 0.53 | 0.46 | 0.69 | 0.658 | 0.726 |
| A_51_P499854 | NM_010284 | 14600 | Ghr | 0.407 | 0.375 | 0.412 | 0.413 | 0.429 | 0.489 |
| A_51_P455647 | NM_009801 | 12349 | Car2 | 0.335 | 0.288 | 0.301 | 0.568 | 0.516 | 0.638 |
| A_52_P103929 | NM_009306 | 20979 | Syt1 | 0.382 | 0.356 | 0.39 | 0.419 | 0.498 | 0.423 |
| A_51_P149469 | NM_009608 | 11464 | Actc1 | 0.324 | 0.387 | 0.384 | 0.496 | 0.551 | 0.471 |
| A_52_P557293 | NM_015775 | 50528 | Tmprss2 | 0.277 | 0.205 | 0.179 | 0.45 | 0.457 | 0.355 |
| A_52_P50496 | AY048587;XM_619453 | 114640 | Tifp39 | 0.454 | 0.501 | 0.422 | 0.661 | 0.638 | 0.694 |
| A_51_P180413 | NM_176930 | 319504 | C130076O07Rik | 0.359 | 0.393 | 0.415 | 0.471 | 0.514 | 0.494 |
| A_51_P229613 | NM_008792 | 18549 | Pcsk2 | 0.512 | 0.483 | 0.515 | 0.58 | 0.58 | 0.504 |
| A_51_P121635 | NM_030700 | 80884 | Maged2 | 0.378 | 0.371 | 0.409 | 0.492 | 0.475 | 0.481 |
| A_51_P481777 | NM_018756 | 54382 | Tcstv1 | 1.532 | 1.822 | 1.792 | 2.619 | 3.155 | 2.956 |
| A_51_P412817 | NM_177617 | 216851 | D330014H01Rik | 3.151 | 3.039 | 3.326 | 2.136 | 2.194 | 1.865 |
| A_51_P387528 | AK019664; | 78801 | Ak7 | 2.693 | 2.814 | 2.57 | 3.786 | 3.834 | 3.71 |
| A_52_P78168 | AK031518; | | | 2.108 | 1.677 | 1.617 | 1.783 | 1.791 | 1.711 |
| A_52_P274960 | NM_001005508 | 226652 | 6030405P05Rik | 1.83 | 1.82 | 1.948 | 2.07 | 2.181 | 2.076 |
| A_52_P1188270 | AK042362; | | | 3.076 | 1.828 | 1.917 | 2.932 | 3.046 | 2.87 |
| A_51_P291227 | NM_001005508 | 226652 | 6030405P05Rik | 1.876 | 1.826 | 1.86 | 2.054 | 2.185 | 2.004 |
| A_52_P377299 | AK048360; | 271377 | Zbtb11 | 1.927 | 1.892 | 1.923 | 1.678 | 1.79 | 1.546 |
| A_51_P297131 | NM_009502 | 22330 | Vcl | 0.402 | 0.372 | 0.378 | 0.411 | 0.377 | 0.401 |
| A_52_P804224 | | | | 1.522 | 1.688 | 1.828 | 2.31 | 2.487 | 2.241 |
| A_51_P442894 | AK048310; | 621603 | C130048D07Rik | 2.632 | 2.364 | 2.281 | 3.657 | 3.714 | 3.102 |
| A_52_P309718 | AK173099; | 218850 | D14Abb1e | 2.324 | 2.579 | 2.18 | 2.614 | 2.72 | 2.711 |
| A_52_P359010 | AK129478; | 72193 | Sfrs2ip | 2.152 | 2.149 | 2.122 | 2.349 | 2.38 | 2.318 |
| A_51_P410813 | AK079732; | | | 2.732 | 2.716 | 2.559 | 2.898 | 2.605 | 2.3 |
| A_52_P382565 | XM_483943 | 432493 | LOC432493 | 0.976 | 1.222 | 1.221 | 1.492 | 1.439 | 1.198 |
| A_51_P333253 | NM_178440 | 246177 | Myo1g | 1.419 | 1.413 | 1.621 | 1.544 | 1.504 | 1.394 |
| A_52_P113190 | NM_053214 | 17916 | Myo1f | 1.428 | 1.513 | 1.396 | 1.988 | 1.959 | 2.083 |
| A_51_P473953 | XM_358600;XM_355454 | 381498 | 4930592C13Rik | 0.42 | 0.306 | 0.298 | 0.365 | 0.365 | 0.38 |

**Table 5B - 50 Gene Subset (Non Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51 P237040 | NM_008711 | 18121 | Nog | 1.152 | 1.407 | 1.101 | 0.859 | 0.96 | 0.924 |
| A_51_P335460 | NM_009132 | 20259 | Scin | 1.658 | 1.913 | 1.519 | 1.101 | 1.225 | 1.107 |
| A_52_P391505 | NM_144513 | 17263 | Gtl2 | 0.0793 | 0.114 | 0.0867 | 0.158 | 0.15 | 0.148 |
| A_51_P497100 | NM_010706 | 16855 | Lgals4 | 0.692 | 0.771 | 0.849 | 1.002 | 0.908 | 0.828 |
| A_51_P378371 | | | | 0.986 | 1.006 | 1.208 | 1.217 | 1.156 | 1.234 |
| A_51_P298266 | NM_025681 | 66643 | Lix1 | 1.123 | 1.145 | 1.109 | 1.143 | 1.15 | 1.141 |
| A_52_P269158 | NM_001003948 | 98496 | AL024069 | 1.775 | 1.419 | 1.506 | 1.494 | 1.391 | 1.254 |
| A_51_P311038 | BC009666;XM_134720 | 57267 | Apba3 | 1.847 | 1.642 | 1.538 | 2.55 | 2.405 | 2.808 |
| A_51_P457187 | NM_028075 | 72049 | Tnfrsf13c | 1.083 | 1.58 | 1.373 | 0.916 | 1.099 | 1.091 |
| A_52_P68702 | NM_145148 | 232288 | Frmd4b | 0.928 | 1.071 | 0.877 | 1.242 | 1.234 | 1.139 |
| A_52_P293443 | AK087208; | 13819 | | 0.179 | 0.222 | 0.219 | 0.543 | 0.565 | 0.552 |
| A_51_P157986 | NM_029930 | 77574 | 3321401 G04Rik | 0.909 | 1.196 | 1.088 | 0.563 | 0.621 | 0.598 |
| A_52_P1013441 | | | | 1.255 | 1.032 | 2.041 | 1.173 | 1.203 | 1.341 |
| A_51_P418375 | NM_023844 | 67374 | Jam2 | 1.044 | 0.943 | 0.99 | 0.826 | 0.786 | 0.84 |
| A_52_P557940 | AK004302; | 66704 | 4921506122Rik | 0.544 | 0.595 | 0.569 | 1.371 | 1.379 | 1.241 |
| A_51_P480311 | NM_010168 | 14061 | F2 | 0.348 | 0.476 | 0.622 | 0.996 | 1.004 | 1.186 |
| A_52_P200458 | NM_010242 | 14345 | Fut4 | 1.003 | 0.997 | 1.017 | 1.108 | 1.203 | 1.09 |
| A_51_P463846 | NM_145545 | 229900 | 9830147J24Rik | 2.28 | 2.199 | 2.49 | 2.068 | 1.958 | 1.72 |
| A_51_P162474 | AF285577; | 107815 | Scml2 | 2.885 | 2.549 | 2.371 | 3.021 | 3.116 | 3.1 |
| A_51_P165244 | NM_018734 | 55932 | Gbp4 | 2.388 | 1.319 | 2.543 | 2.404 | 2.051 | 2.534 |
| A_52_P59318 | NM_133832 | 98711 | Rdh10 | 1.689 | 1.611 | 1.584 | 0.876 | 1.002 | 0.955 |
| A_52_P438036 | NM_175271 | 78134 | Gpr23 | 1.082 | 0.96 | 0.968 | 1.634 | 1.593 | 1.618 |
| A_52_P474242 | BE457768; | | | 1.31 | 1.785 | 1.55 | 1.137 | 1.132 | 0.997 |
| A_51_P499854 | NM_010284 | 14600 | Ghr | 1.55 | 1.19 | 1.486 | 2.502 | 2.537 | 2.196 |
| A_51_P455647 | NM_00980 | 12349 | Car2 | 1.592 | 1.463 | 1.337 | 2.139 | 2.347 | 2.17 |
| A_52_P103929 | NM_009306 | 20979 | Syt1 | 0.898 | 0.938 | 0.967 | 1.626 | 1.464 | 1.422 |
| A_51_P149469 | NM_009608 | 11464 | Actc1 | 1.012 | 1.006 | 0.935 | 0.971 | 0.997 | 1.12 |
| A_52_P557293 | NM_015775 | 50528 | Tmprss2 | 1.142 | 1.091 | 1.111 | 1.208 | 1.127 | 1.225 |
| A_52_P50496 | AY048587;XM_619453 | 114640 | Tifp39 | 1.196 | 1.551 | 1.504 | 1.114 | 1.171 | 1.214 |
| A_51_P180413 | NM_176930 | 319504 | C130076O07Rik | 1.603 | 1.54 | 1.395 | 1.297 | 1.25 | 1.155 |
| A_51_P229613 | NM_008792 | 18549 | Pcsk2 | 1.463 | 1.53 | 1.37 | 1.333 | 1.422 | 1.554 |
| A_51_P121635 | NM_030700 | 80884 | Maged2 | 1.225 | 1.426 | 1.335 | 0.918 | 0.99 | 0.88 |
| A_51_P481777 | NM_018756 | 54382 | Tcstv1 | 0.208 | 0.696 | 0.654 | 0.592 | 0.503 | 0.72 |
| A_51_P412817 | NM_177617 | 216851 | D330014H01Rik | 0.725 | 0.834 | 1.04 | 1.083 | 0.986 | 0.997 |
| A_51_P387528 | AK019664; | 78801 | Ak7 | 0.65 | 0.537 | 0.813 | 0.767 | 0.778 | 1.11 |
| A_52_P78168 | AK031518; | | | 0.277 | 0.385 | 0.299 | 0.704 | 0.652 | 0.544 |
| A_52_P274960 | NM_001005508 | 226652 | 6030405P05Rik | 0.671 | 0.634 | 0.712 | 0.763 | 0.781 | 0.808 |
| A_52_P1188270 | AK042362; | | | 0.341 | 0.594 | 0.564 | 1.758 | 1.462 | 1.658 |
| A_51_P291227 | NM_001005508 | 226652 | 6030405P05Rik | 0.705 | 0.681 | 0.731 | 0.814 | 0.735 | 0.798 |
| A_52_P377299 | AK048360; | 271377 | Zbtb11 | 0.577 | 0.582 | 0.645 | 1.152 | 1.092 | 1.023 |
| A_51_P297131 | NM_009502 | 22330 | Vcl | 1.015 | 1.436 | 0.955 | 0.556 | 0.672 | 0.587 |
| A_52_P804224 | | | | 0.299 | 0.314 | 0.291 | 1.263 | 1.2 | 1.068 |
| A_51_P442894 | AK048310; | 621603 | C130048D07Rik | 1.023 | 0.841 | 1.218 | 0.788 | 0.867 | 0.977 |
| A_52_P309718 | AK173099; | 218850 | D14Abb1e | 1.006 | 0.813 | 1.127 | 1.186 | 1.29 | 1.289 |
| A_52_P359010 | AK129478; | 72193 | Sfrs2ip | 0.54 | 0.706 | 0.574 | 1.484 | 1.398 | 1.403 |
| A_51_P410813 | AK079732; | | | 0.717 | 0.877 | 0.904 | 1.593 | 1.473 | 1.607 |
| A_52_P382565 | XM_483943 | 432493 | LOC432493 | 0.228 | 0.273 | 0.254 | 0.607 | 0.622 | 0.462 |
| A_51_P333253 | NM_178440 | 246177 | Myo1g | 0.535 | 0.491 | 0.538 | 0.474 | 0.481 | 0.37 |
| A_52_P113190 | NM_053214 | 17916 | Myo1f | 0.386 | 0.513 | 0.397 | 0.243 | 0.232 | 0.264 |
| A_51_P473953 | XM_358600;XM_355454 | 381498 | 4930592C13Rik | 2.138 | 1.593 | 1.947 | 2.323 | 2.226 | 2.26 |

**Table 6A - 25 Gene Subset (Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 0.266 | 0.299 | 0.281 | 0.396 | 0.368 | 0.407 |
| A_51_P335460 | NM_009132 | 20259 | Scin | 0.718 | 0.631 | 0.719 | 0.678 | 0.674 | 0.666 |
| A_52_P391505 | NM_144513 | 17263 | Gtl2 | 1.36 | 1.291 | 1.327 | 1.053 | 1.04 | 0.852 |
| A_51_P497100 | NM_010706 | 16855 | Lgals4 | 2.115 | 2.049 | 2.368 | 1.716 | 1.861 | 1.527 |
| A_51_P378371 | | | | 0.454 | 0.542 | 0.39 | 0.468 | 0.484 | 0.468 |
| A_51_P298266 | NM_025681 | 66643 | Lix1 | 0.507 | 0.414 | 0.416 | 0.554 | 0.584 | 0.54 |
| A_52_P269158 | NM_001003948 | 98496 | AL024069 | 0.316 | 0.33 | 0.33 | 0.388 | 0.393 | 0.443 |
| A_51_P311038 | BC009666;XM_134720 | 57267 | Apba3 | 0.334 | 0.341 | 0.339 | 0.446 | 0.472 | 0.482 |
| A_51_P457187 | NM_028075 | 72049 | Tnfrsf13c | 2.82 | 3.275 | 3.026 | 2.654 | 3.432 | 2.546 |
| A_52_P68702 | NM_145148 | 232288 | Frmd4b | 0.545 | 0.476 | 0.538 | 0.501 | 0.43 | 0.424 |
| A_52_P293443 | AK087208; | 13819 | | 1.278 | 1.075 | 1.13 | 1.496 | 1.604 | 1.349 |
| A_51_P157986 | NM_029930 | 77574 | 3321401 G04Rik | 0.426 | 0.339 | 0.345 | 0.368 | 0.318 | 0.37 |
| A_52_P1013441 | | | | 3.248 | 3.164 | 3.191 | 4.558 | 5.61 | 4.683 |
| A_51_P418375 | NM_023844 | 67374 | Jam2 | 1.691 | 1.467 | 1.625 | 2.142 | 2.132 | 2.288 |
| A_52_P557940 | AK004302; | 66704 | 4921506122Rik | 2.114 | 1.863 | 2.221 | 2.17 | 2.013 | 2.051 |
| A_51_P480311 | NM_010168 | 14061 | F2 | 1.991 | 1.809 | 1.969 | 2.465 | 2.4 | 1.874 |
| A_52_P200458 | NM_010242 | 14345 | Fut4 | 0.429 | 0.343 | 0.355 | 0.453 | 0.391 | 0.512 |
| A_51_P463846 | NM_145545 | 229900 | 9830147J24Rik | 0.694 | 0.519 | 0.59 | 0.86 | 0.816 | 0.817 |
| A_51_P162474 | AF285577; | 107815 | Scml2 | 0.539 | 0.413 | 0.405 | 0.643 | 0.645 | 0.71 |
| A_51_P165244 | NM_018734 | 55932 | Gbp4 | 0.585 | 0.512 | 0.474 | 1.008 | 0.981 | 1.006 |
| A_52_P59318 | NM_133832 | 98711 | Rdh10 | 0.306 | 0.273 | 0.275 | 0.373 | 0.395 | 0.445 |
| A_52_P438036 | NM_175271 | 78134 | Gpr23 | 0.376 | 0.334 | 0.36 | 0.455 | 0.441 | 0.489 |
| A_52_P474242 | BE457768; | | | 0.499 | 0.53 | 0.46 | 0.69 | 0.658 | 0.726 |
| A_51_P499854 | NM_010284 | 14600 | Ghr | 0.407 | 0.375 | 0.412 | 0.413 | 0.429 | 0.489 |
| A_51_P455647 | NM_009801 | 12349 | Car2 | 0.335 | 0.288 | 0.301 | 0.568 | 0.516 | 0.638 |

**Table 6B - 25 Gene Subset (Non Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 1.152 | 1.407 | 1.101 | 0.859 | 0.96 | 0.924 |
| A_51_P335460 | NM_009132 | 20259 | Scin | 1.658 | 1.913 | 1.519 | 1.101 | 1.225 | 1.107 |
| A_52_P391505 | NM_144513 | 17263 | Gtl2 | 0.0793 | 0.114 | 0.0867 | 0.158 | 0.15 | 0.148 |
| A_51_P497100 | NM_010706 | 16855 | Lgals4 | 0.692 | 0.771 | 0.849 | 1.002 | 0.908 | 0.828 |
| A_51_P378371 | | | | 0.986 | 1.006 | 1.208 | 1.217 | 1.156 | 1.234 |
| A_51_P298266 | NM_025681 | 66643 | Lix1 | 1.123 | 1.145 | 1.109 | 1.143 | 1.15 | 1.141 |
| A_52_P269158 | NM_001003948 | 98496 | AL024069 | 1.775 | 1.419 | 1.506 | 1.494 | 1.391 | 1.254 |
| A_51_P311038 | BC009666;XM_134720 | 57267 | Apba3 | 1.847 | 1.642 | 1.538 | 2.55 | 2.405 | 2.808 |
| A_51_P457187 | NM_028075 | 72049 | Tnfrsf13c | 1.083 | 1.58 | 1.373 | 0.916 | 1.099 | 1.091 |
| A_52_P68702 | NM_145148 | 232288 | Frmd4b | 0.928 | 1.071 | 0.877 | 1.242 | 1.234 | 1.139 |
| A_52_P293443 | AK087208; | 13819 | | 0.179 | 0.222 | 0.219 | 0.543 | 0.565 | 0.552 |
| A_51_P157986 | NM_029930 | 77574 | 3321401 G04Rik | 0.909 | 1.196 | 1.088 | 0.563 | 0.621 | 0.598 |
| A_52_P 1013441 | | | | 1.255 | 1.032 | 2.041 | 1.173 | 1.203 | 1.341 |
| A_51_P418375 | NM_023844 | 67374 | Jam2 | 1.044 | 0.943 | 0.99 | 0.826 | 0.786 | 0.84 |
| A_52_P557940 | AK004302; | 66704 | 4921506I22Rik | 0.544 | 0.595 | 0.569 | 1.371 | 1.379 | 1.241 |
| A_51_P480311 | NM_010168 | 14061 | F2 | 0.348 | 0.476 | 0.622 | 0.996 | 1.004 | 1.186 |
| A_52_P200458 | NM_010242 | 14345 | Fut4 | 1.003 | 0.997 | 1.017 | 1.108 | 1.203 | 1.09 |
| A_51_P463846 | NM_145545 | 229900 | 9830147J24Rik | 2.28 | 2.199 | 2.49 | 2.068 | 1.958 | 1.72 |
| A_51_P162474 | AF285577; | 107815 | Scml2 | 2.885 | 2.549 | 2.371 | 3.021 | 3.116 | 3.1 |
| A_51_P165244 | NM_018734 | 55932 | Gbp4 | 2.388 | 1.319 | 2.543 | 2.404 | 2.051 | 2.534 |
| A_52_P59318 | NM_133832 | 98711 | Rdh10 | 1.689 | 1.611 | 1.584 | 0.876 | 1.002 | 0.955 |
| A_52_P438036 | NM_175271 | 78134 | Gpr23 | 1.082 | 0.96 | 0.968 | 1.634 | 1.593 | 1.618 |
| A_52_P474242 | BE457768; | | | 1.31 | 1.785 | 1.55 | 1.137 | 1.132 | 0.997 |
| A_51_P499854 | NM_010284 | 14600 | Ghr | 1.55 | 1.19 | 1.486 | 2.502 | 2.537 | 2.196 |
| A_51_P455647 | NM_009801 | 12349 | Car2 | 1.592 | 1.463 | 1.337 | 2.139 | 2.347 | 2.17 |

**Table 7A - 10 Gene Subset (Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 0.266 | 0.299 | 0.281 | 0.396 | 0.368 | 0.407 |
| A_51_P335460 | NM_009132 | 20259 | Scin | 0.718 | 0.631 | 0.719 | 0.678 | 0.674 | 0.666 |
| A_52_P391505 | NM_144513 | 17263 | Gtl2 | 1.36 | 1.291 | 1.327 | 1.053 | 1.04 | 0.852 |
| A_51_P497100 | NM_010706 | 16855 | Lgals4 | 2.115 | 2.049 | 2.368 | 1.716 | 1.861 | 1.527 |
| A_51_P378371 | | | | 0.454 | 0.542 | 0.39 | 0.468 | 0.484 | 0.468 |
| A_51_P298266 | NM_025681 | 66643 | Lix1 | 0.507 | 0.414 | 0.416 | 0.554 | 0.584 | 0.54 |
| A_52_P269158 | NM_001003948 | 98496 | AL024069 | 0.316 | 0.33 | 0.33 | 0.388 | 0.393 | 0.443 |
| A_51_P311038 | BC009666;XM_134720 | 57267 | Apba3 | 0.334 | 0.341 | 0.339 | 0.446 | 0.472 | 0.482 |
| A_51_P457187 | NM_028075 | 72049 | Tnfrsf13c | 2.82 | 3.275 | 3.026 | 2.654 | 3.432 | 2.546 |
| A_52_P68702 | NM_145148 | 232288 | Frmd4b | 0.545 | 0.476 | 0.538 | 0.501 | 0.43 | 0.424 |

**Table 7B - 10 Gene Subset (Non Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 1.152 | 1.407 | 1.101 | 0.859 | 0.96 | 0.924 |
| A_51_P335460 | NM_009132 | 20259 | Scin | 1.658 | 1.913 | 1.519 | 1.101 | 1.225 | 1.107 |
| A_52_P391505 | NM_144513 | 17263 | Gtl2 | 0.0793 | 0.114 | 0.0867 | 0.158 | 0.15 | 0.148 |
| A_51_P497100 | NM_010706 | 16855 | Lgals4 | 0.692 | 0.771 | 0.849 | 1.002 | 0.908 | 0.828 |
| A 51 P378371 | | | | 0.986 | 1.006 | 1.208 | 1.217 | 1.156 | 1.234 |
| A_51_P298266 | NM_025681 | 66643 | Lix1 | 1.123 | 1.145 | 1.109 | 1.143 | 1.15 | 1.141 |
| A_52_P269158 | NM_001003948 | 98496 | AL024069 | 1.775 | 1.419 | 1.506 | 1.494 | 1.391 | 1.254 |
| A_51_P311038 | BC009666;XM_134720 | 57267 | Apba3 | 1.847 | 1.642 | 1.538 | 2.55 | 2.405 | 2.808 |
| A_51_P457187 | NM_028075 | 72049 | Tnfrsf13c | 1.083 | 1.58 | 1.373 | 0.916 | 1.099 | 1.091 |
| A_52_P68702 | NM_145148 | 232288 | Frmd4b | 0.928 | 1.071 | 0.877 | 1.242 | 1.234 | 1.139 |

**Table 8A - 5 Gene Subset (Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 0.266 | 0.299 | 0.281 | 0.396 | 0.368 | 0.407 |
| A_51_P335460 | NM_009132 | 20259 | Scin | 0.718 | 0.631 | 0.719 | 0.678 | 0.674 | 0.666 |
| A_52_P391505 | NM_144513 | 17263 | Gtl2 | 1.36 | 1.291 | 1.327 | 1.053 | 1.04 | 0.852 |
| A_51_P497100 | NM_010706 | 16855 | Lgals4 | 2.115 | 2.049 | 2.368 | 1.716 | 1.861 | 1.527 |
| A_51_P378371 | | | | 0.454 | 0.542 | 0.39 | 0.468 | 0.484 | 0.468 |

**Table 8B - 5 Gene Subset (Non Germline Competent)**

| **Agilent Probe ID** | **Genbank** | **Entrez ID** | **Gene Symbol** | **Clone 1** | | | **Clone 2** | | |
|---|---|---|---|---|---|---|---|---|---|
| | | | | **rep.1** | **rep. 2** | **rep.3** | **rep. 1** | **rep. 2** | **rep.3** |
| A_51_P237040 | NM_008711 | 18121 | Nog | 1.152 | 1.407 | 1.101 | 0.859 | 0.96 | 0.924 |
| A_51_P335460 | NM_009132 | 20259 | Scin | 1.658 | 1.913 | 1.519 | 1.101 | 1.225 | 1.107 |
| A_52_P391505 | NM_144513 | 17263 | Gtl2 | 0.0793 | 0.114 | 0.0867 | 0.158 | 0.15 | 0.148 |
| A_51_P497100 | NM_010706 | 16855 | Lgals4 | 0.692 | 0.771 | 0.849 | 1.002 | 0.908 | 0.828 |
| A_51_P378371 | | | | 0.986 | 1.006 | 1.208 | 1.217 | 1.156 | 1.234 |

Although the foregoing invention has been described in some detail by way of illustration and examples, it will be readily apparent to those of ordinary skill in the art that certain changes and modifications may be made to the teachings of the invention.

## Claims

1. A method for selecting an embryonic stem cell (ES cell) that is germline competent, comprising providing an expression profile of a subset of ES cell genes from an ES cell and comparing the expression profile of the subset of ES cell genes from the ES cell with an expression profile of a subset of ES cell genes from a germline competent ES cell, wherein the subset of ES cell genes is one or more genes listed in Table 8 in conjunction with one or more different genes selected from the genes listed in Table 5.

2. The method of claim 1, wherein the expression profiles are generated on an expression array.

3. The method of claim 1, wherein the expression profiles are generated by an ELISA method or a protein expression assay.

4. The method of claim 1, wherein the expression profiles are profiles of expressed and secreted genes.

5. The method of claim 1, wherein the expression profiles are generated using an ELISA assay.

6. The method of claim 1, wherein the expression profiles are generated using an affinity assay using microspheres in the liquid phase.

7. The method of claim 1, wherein the subset of genes is selected from the group consisting of 50 genes, 25 genes, 10 genes, 5 genes, and 2 genes.

## Patentansprüche

1. Verfahren zur Auswahl einer embryonalen Stammzelle (ES-Zelle), die keimbahnfähig ist, umfassend das Bereitstellen eines Expressionsprofils eines Subsets von ES-Zell-Genen aus einer ES-Zelle und Vergleichen des Expressionsprofils des Subsets der ES-Zell-Gene aus der ES-Zelle mit einem Expressionsprofil eines Subsets von ES-Zell-Genen aus einer keimbahnfähigen ES-Zelle, wobei das Subset der ES-Zell-Gene aus ein oder mehreren der in Tabelle 8 aufgelisteten Genen in Verbindung mit ein oder mehreren unterschiedlichen Genen, ausgewählt aus den in Tabelle 5 aufgelisteten Genen, besteht.

2. Verfahren nach Anspruch 1, wobei die Expressionsprofile auf einem Expressionsarray erstellt sind.

3. Verfahren nach Anspruch 1, wobei die Expressionsprofile mittels einer ELISA-Methode oder eines Proteinexpressionsassays erstellt sind.

4. Verfahren nach Anspruch 1, wobei die Expressionsprofile Profile von exprimierten und sekretierten Genen sind.

5. Verfahren nach Anspruch 1, wobei die Expressionsprofile unter Anwendung eines ELISA-Assays erstellt sind.

6. Verfahren nach Anspruch 1, wobei die Expressionsprofile unter Anwendung eines Affinitätsassays unter Verwendung von Mikrokügelchen in der flüssigen Phase erstellt sind.

7. Verfahren nach Anspruch 1, wobei das Subset der Gene ausgewählt ist aus der Gruppe, bestehend aus 50 Genen, 25 Genen, 10 Genen, 5 Genen und 2 Genen.

## Revendications

1. Méthode de sélection d'une cellule souche embryonnaire (cellule ES) qui présente une compétence germinale, comprenant l'utilisation d'un profil d'expression d'une sous-catégorie de gènes de cellule ES provenant d'une cellule ES et la comparaison du profil d'expression de la sous-catégorie de gènes de cellule ES provenant de la cellule ES avec un profil d'expression d'une sous-catégorie de gènes de cellule ES provenant d'une cellule ES à compétence germinale, la sous-catégorie de gènes de cellule ES étant un ou plusieurs gènes listées dans le tableau 8 conjointement avec un ou plusieurs gènes différents choisis parmi les gènes listés dans le tableau 5.

2. Méthode selon la revendication 1, dans laquelle les profils d'expression sont générés sur une matrice d'expression.

3. Méthode selon la revendication 1, dans laquelle les profils d'expression sont générés par une méthode ELISA ou un dosage d'expression des protéines.

4. Méthode selon la revendication 1, dans laquelle les profils d'expression sont des profils de gènes exprimés et sécrétés.

5. Méthode selon la revendication 1, dans laquelle les profils d'expression sont générés en utilisant un dosage ELISA.

6. Méthode selon la revendication 1, dans laquelle les profils d'expression sont générés en utilisant un dosage d'affinité en utilisant des microsphères dans la phase liquide.

7. Méthode selon la revendication 1, dans laquelle la sous-catégorie de gènes est choisie dans le groupe constitué par 50 gènes, 25 gènes, 10 gènes, 5 gènes et 2 gènes.
